# EUROPEAN PATENT SPECIFICATION

(11) **EP 3 163 644 B1**
(45) Date of publication and mention of the grant of the patent: **24.03.2021**
(21) Application number: 15811569.1
(22) Date of filing: 17.06.2015
(51) Int. Cl.: C07D 401/10

(54) **ORGANIC ELECTROLUMINESCENT ELEMENT**
ORGANISCHES ELEKTROLUMINESZENTES ELEMENT
ÉLÉMENT ÉLECTROLUMINESCENT ORGANIQUE

(30) Priority: 26.06.2014 JP 2014130969
(43) Date of publication of application: 03.05.2017
(73) Proprietor: Hodogaya Chemical Co., Ltd., Chuo-ku Tokyo 104-0028 (JP)
(72) Inventor: KABASAWA, Naoaki, Tokyo 104-0028 (JP); KANDA, Daizou, Tokyo 104-0028 (JP); YOKOYAMA, Norimasa, Tokyo 104-0028 (JP); HAYASHI, Shuichi, Tokyo 104-0028 (JP); MOCHIDUKI, Shunji, Tokyo 104-0028 (JP)
(74) Representative: Müller-Boré & Partner Patentanwälte PartG mbB
(86) International application number: PCT/JP2015/003035
(87) International publication number: WO 2015/198563

(56) References cited:
- WO-A1-2007/080704
- WO-A1-2009/139475
- WO-A1-2010/098458
- WO-A1-2013/038627
- WO-A1-2014/129201
- WO-A1-2015/004875
- JP-A- 2011 222 831
- JP-A- 2014 511 352
- KR-A- 20150 024 803
- US-A1- 2012 181 520

## Description

The present invention relates to an organic electroluminescent device which is a preferred self-luminous device for various display devices. Specifically, this invention relates to organic electroluminescent devices (hereinafter referred to as organic EL devices) using specific arylamine compounds and specific compounds having an anthracene ring structure (and specific luminous dopants (luminous dopants having a specific structure)).

The organic EL device is a self-luminous device and has been actively studied for their brighter, superior visibility and the ability to display clearer images in comparison with liquid crystal devices.

In 1987, C. W. Tang and colleagues at Eastman Kodak developed a laminated structure device using materials assigned with different roles, realizing practical applications of an organic EL device with organic materials. These researchers laminated an electron-transporting phosphor and a hole-transporting organic substance, and injected both charges into a phosphor layer to cause emission in order to obtain a high luminance of 1,000 cd/m² or more at a voltage of 10 V or less (refer to Patent Documents 1 and 2, for example).

To date, various improvements have been made for practical applications of the organic EL device. Various roles of the laminated structure are further subdivided to provide an electroluminescence device that includes an anode, a hole injection layer, a hole transport layer, a light emitting layer, an electron transport layer, an electron injection layer, and a cathode successively formed on a substrate, and high efficiency and durability have been achieved by the electroluminescence device (refer to Non-Patent Document 1, for example).

Further, there have been attempts to use triplet excitons for further improvements of luminous efficiency, and the use of a phosphorescence-emitting compound has been examined (refer to Non-Patent Document 2, for example).

Devices that use light emission caused by thermally activated delayed fluorescence (TADF) have also been developed. In 2011, Adachi et al. at Kyushu University, National University Corporation realized 5.3% external quantum efficiency with a device using a thermally activated delayed fluorescent material (refer to Non-Patent Document 3, for example).

The light emitting layer can be also fabricated by doping a charge-transporting compound generally called a host material, with a fluorescent compound, a phosphorescence-emitting compound, or a delayed fluorescent-emitting material. As described in the Non-Patent Document, the selection of organic materials in an organic EL device greatly influences various device characteristics such as efficiency and durability (refer to Non-Patent Document 2, for example).

In an organic EL device, charges injected from both electrodes recombine in a light emitting layer to cause emission. What is important here is how efficiently the hole and electron charges are transferred to the light emitting layer in order to form a device having excellent carrier balance. The probability of hole-electron recombination can be improved by improving hole injectability and electron blocking performance of blocking injected electrons from the cathode, and high luminous efficiency can be obtained by confining excitons generated in the light emitting layer. The role of a hole transport material is therefore important, and there is a need for a hole transport material that has high hole injectability, high hole mobility, high electron blocking performance, and high durability to electrons.

Heat resistance and amorphousness of the materials are also important with respect to the lifetime of the device. The materials with low heat resistance cause thermal decomposition even at a low temperature by heat generated during the drive of the device, which leads to the deterioration of the materials. The materials with low amorphousness cause crystallization of a thin film even in a short time and lead to the deterioration of the device. The materials in use are therefore required to have characteristics of high heat resistance and satisfactory amorphousness.

N,N'-diphenyl-N,N'-di(α-naphthyl)benzidine (NPD) and various aromatic amine derivatives are known as the hole transport materials used for the organic EL device (refer to Patent Documents 1 and 2, for example). Although NPD has desirable hole transportability, its glass transition point (Tg), which is an index of heat resistance, is as low as 96°C, which causes the degradation of device characteristics by crystallization under a high-temperature condition (refer to Non-Patent Document 4, for example). The aromatic amine derivatives described in the Patent Documents include a compound known to have an excellent hole mobility of 10⁻³ cm²/Vs or higher (refer to Patent Documents 1 and 2, for example). However, since the compound is insufficient in terms of electron blocking performance, some of the electrons pass through the light emitting layer, and improvements in luminous efficiency cannot be expected. For such a reason, a material with higher electron blocking performance, a more stable thin-film state and higher heat resistance is needed for higher efficiency. Although an aromatic amine derivative having high durability is reported (refer to Patent Document 3, for example), the derivative is used as a charge transporting material used in an electrophotographic photoconductor, and there is no example of using the derivative in the organic EL device.

Arylamine compounds having a substituted carbazole structure are proposed as compounds improved in the characteristics such as heat resistance and hole injectability (refer to Patent Documents 4 and 5, for example). However, while the devices using these compounds for the hole injection layer or the hole transport layer have been improved in heat resistance, luminous efficiency and the like, the improvements are still insufficient. Further lower driving voltage and higher luminous efficiency are therefore needed.

In order to improve characteristics of the organic EL device and to improve the yield of the device production, it has been desired to develop a device having high luminous efficiency, low driving voltage and a long lifetime by using in combination the materials that excel in hole and electron injection/transport performances, stability as a thin film and durability, permitting holes and electrons to be highly efficiently recombined together.

Further, in order to improve characteristics of the organic EL device, it has been desired to develop a device that maintains carrier balance and has high efficiency, low driving voltage and a long lifetime by using in combination the materials that excel in hole and electron injection/transport performances, stability as a thin film and durability.

Patent Document 1: JP-A-8-048656
Patent Document 2: Japanese Patent No. 3194657
Patent Document 3: Japanese Patent No. 4943840
Patent Document 4: JP-A-2006-151979
Patent Document 5: WO2008/62636
Patent Document 6: WO2005/115970
Patent Document 7: WO2011/059000
Patent Document 8: WO2003/060956
Patent Document 9: KR-A-2013-060157

Non-Patent Document 1: The Japan Society of Applied Physics, 9th Lecture Preprints, pp. 55 to 61 (2001)
Non-Patent Document 2: The Japan Society of Applied Physics, 9th Lecture Preprints, pp. 23 to 31 (2001)
Non-Patent Document 3: Appl. Phys. Let., 98, 083302 (2011)
Non-Patent Document 4: Organic EL Symposium, the 3rd Regular presentation Preprints, pp. 13 to 14 (2006)

JP 2014 511352 A discloses a compound and an organic light-emitting device comprising the same.

WO 2009/139475 A1 discloses an organic electroluminescent device.

WO 2010/098458 A1 discloses an organic electroluminescent device.

An object of the present invention is to provide an organic EL device having high efficiency, low driving voltage and a long lifetime, by combining various materials for an organic EL device, which are excellent, as materials for an organic EL device having high efficiency and high durability, in hole and electron injection/transport performances, electron blocking ability, stability in a thin-film state and durability, so as to allow the respective materials to effectively reveal their characteristics.

Physical properties of the organic compound to be provided by the present invention include (1) good hole injection characteristics, (2) large hole mobility, (3) excellent electron blocking ability, (4) stability in a thin-film state, and (5) excellent heat resistance. Physical properties of the organic EL device to be provided by the present invention include (1) high luminous efficiency and high power efficiency, (2) low turn on voltage, (3) low actual driving voltage, and (4) a long lifetime.

To achieve the above object, the present inventors have noted that an arylamine material is excellent in hole injection and transport abilities, stability as a thin film and durability, and compounds having an anthracene ring structure are excellent in electron injection and transport abilities, stability as a thin film and durability. They have selected specific arylamine compounds and specific compounds having an anthracene ring structure such that holes and electrons can be efficiently injected and transported into a light emitting layer, and have produced various organic EL devices by combining a hole transport material and an electron transport material in good carrier balance. Then, they have intensively conducted characteristic evaluations of the devices. Also, they have selected specific arylamine compounds and specific compounds having an anthracene ring structure, and have selected specific luminous dopants (luminous dopants having a specific structure), and have produced various organic EL devices that maintain carrier balance by refining combinations of those. Then, they have intensively conducted characteristic evaluations of the devices. As a result, they have completed the present invention.

Specifically, according to the present invention, the following organic EL devices are provided.
1) An organic EL device comprising at least an anode, a hole transport layer, a light emitting layer, an electron transport layer and a cathode in this order, wherein the hole transport layer is a single layer, an arylamine compound of the following general formula (1) is used as the hole transport layer, and the electron transport layer comprises a compound of the following general formula (2c) having an anthracene ring structure.
   [Chemical Formula 1] 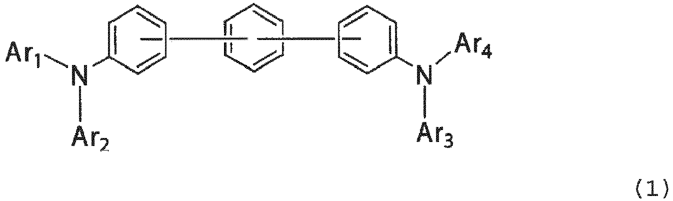
   In the general formula (1), Ar₁ to Ar₄ may be the same or different, and represent a substituted or unsubstituted aromatic hydrocarbon group, a substituted or unsubstituted aromatic heterocyclic group, or a substituted or unsubstituted condensed polycyclic aromatic group.
   The compound having an anthracene ring structure is a compound of the following general formula (2c) having an anthracene ring structure.
   [Chemical Formula 5] 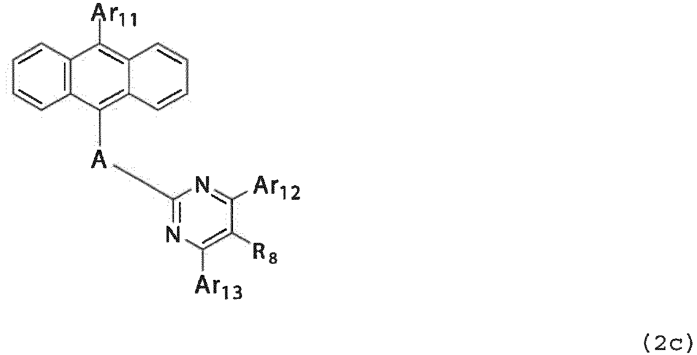
   In the general formula (2c), A represents a divalent group of a substituted or unsubstituted aromatic hydrocarbon, a divalent group of a substituted or unsubstituted aromatic heterocyclic ring, a divalent group of substituted or unsubstituted condensed polycyclic aromatics, or a single bond; Ar₁₁, Ar₁₂ and Ar₁₃ may be the same or different, and represent a substituted or unsubstituted aromatic hydrocarbon group, a substituted or unsubstituted aromatic heterocyclic group, or a substituted or unsubstituted condensed polycyclic aromatic group; and R₈ represents a hydrogen atom, a deuterium atom, a fluorine atom, a chlorine atom, cyano, nitro, linear or branched alkyl of 1 to 6 carbon atoms that may have a substituent, cycloalkyl of 5 to 10 carbon atoms that may have a substituent, linear or branched alkenyl of 2 to 6 carbon atoms that may have a substituent, linear or branched alkyloxy of 1 to 6 carbon atoms that may have a substituent, cycloalkyloxy of 5 to 10 carbon atoms that may have a substituent, a substituted or unsubstituted aromatic hydrocarbon group, a substituted or unsubstituted aromatic heterocyclic group, or a substituted or unsubstituted condensed polycyclic aromatic group, or substituted or unsubstituted aryloxy.
2) The organic EL device of 1), wherein the light emitting layer includes a blue luminous dopant.
3) The organic EL device of 2), wherein the light emitting layer includes the blue luminous dopant that is a pyrene derivative.
4) The organic EL device of 2), wherein the light emitting layer includes the blue luminous dopant that is an amine derivative having a fluorene ring as a part of a condensed ring.
5) The organic EL device of one of 1) to 4), wherein the light emitting layer includes an anthracene derivative.
6) The organic EL device of 5), wherein the light emitting layer includes a host material that is an anthracene derivative.

Specific examples of the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by Ar₁ to Ar₄ in the general formula (1) include phenyl, biphenylyl, terphenylyl, naphthyl, anthracenyl, phenanthrenyl, fluorenyl, indenyl, pyrenyl, perylenyl, fluoranthenyl, triphenylenyl, pyridyl, pyrimidinyl, triazinyl, furyl, pyrrolyl, thienyl, quinolyl, isoquinolyl, benzofuranyl, benzothienyl, indolyl, carbazolyl, benzoxazolyl, benzothiazolyl, quinoxalinyl, benzoimidazolyl, pyrazolyl, dibenzofuranyl, dibenzothienyl, naphthyridinyl, phenanthrolinyl, acridinyl, and carbolinyl.

Specific examples of the "substituent" in the "substituted aromatic hydrocarbon group", the "substituted aromatic heterocyclic group", or the "substituted condensed polycyclic aromatic group" represented by Ar₁ to Ar₄ in the general formula (1) include a deuterium atom; cyano; nitro; halogen atoms such as a fluorine atom, a chlorine atom, a bromine atom, and an iodine atom; linear or branched alkyloxys of 1 to 6 carbon atoms such as methyloxy, ethyloxy, and propyloxy; alkenyls such as vinyl and allyl; aryloxys such as phenyloxy and tolyloxy; arylalkyloxys such as benzyloxy and phenethyloxy; aromatic hydrocarbon groups or condensed polycyclic aromatic groups such as phenyl, biphenylyl, terphenylyl, naphthyl, anthracenyl, phenanthrenyl, fluorenyl, indenyl, pyrenyl, perylenyl, fluoranthenyl, and triphenylenyl; and aromatic heterocyclic groups such as pyridyl, thienyl, furyl, pyrrolyl, quinolyl, isoquinolyl, benzofuranyl, benzothienyl, indolyl, carbazolyl, benzoxazolyl, benzothiazolyl, quinoxalinyl, benzoimidazolyl, pyrazolyl, dibenzofuranyl, dibenzothienyl, and carbolinyl. These substituents may be further substituted with the exemplified substituents above. These substituents may bind to each other via a single bond, substituted or unsubstituted methylene, an oxygen atom, or a sulfur atom to form a ring.

Specific examples of the "aromatic hydrocarbon", the "aromatic heterocyclic ring", or the "condensed polycyclic aromatics" of the "substituted or unsubstituted aromatic hydrocarbon", the "substituted or unsubstituted aromatic heterocyclic ring", or the "substituted or unsubstituted condensed polycyclic aromatics" in the "divalent group of a substituted or unsubstituted aromatic hydrocarbon", the "divalent group of a substituted or unsubstituted aromatic heterocyclic ring", or the "divalent group of substituted or unsubstituted condensed polycyclic aromatics" represented by A in the general formula (2c) include benzene, biphenyl, terphenyl, tetrakisphenyl, styrene, naphthalene, anthracene, acenaphthylene, fluorene, phenanthrene, indane, pyrene, pyridine, pyrimidine, triazine, pyrrole, furan, thiophene, quinoline, isoquinoline, benzofuran, benzothiophene, indoline, carbazole, carboline, benzoxazole, benzothiazole, quinoxaline, benzimidazole, pyrazole, dibenzofuran, dibenzothiophene, naphthyridine, phenanthroline, and acridine.

The "divalent group of a substituted or unsubstituted aromatic hydrocarbon", the "divalent group of a substituted or unsubstituted aromatic heterocyclic ring", or the "divalent group of substituted or unsubstituted condensed polycyclic aromatics" represented by A in the general formula (2c) is a divalent group that results from the removal of two hydrogen atoms from the above "aromatic hydrocarbon", "aromatic heterocyclic ring", or "condensed polycyclic aromatics".

Examples of the "substituent" of the "substituted aromatic hydrocarbon", the "substituted aromatic heterocyclic ring", or the "substituted condensed polycyclic aromatics" in the "divalent group of a substituted or unsubstituted aromatic hydrocarbon", the "divalent group of a substituted or unsubstituted aromatic heterocyclic ring", or the "divalent group of substituted or unsubstituted condensed polycyclic aromatics" represented by A in the general formula (2c) include the same substituents exemplified as the "substituent" in the "substituted aromatic hydrocarbon group", the "substituted aromatic heterocyclic group", or the "substituted condensed polycyclic aromatic group" represented by Ar₁ to Ar4 in the general formula (1), and possible embodiments may also be the same embodiments as the exemplified embodiments.

Specific examples of the "linear or branched alkyl of 1 to 6 carbon atoms", the "cycloalkyl of 5 to 10 carbon atoms", or the "linear or branched alkenyl of 2 to 6 carbon atoms" in the "linear or branched alkyl of 1 to 6 carbon atoms that may have a substituent", the "cycloalkyl of 5 to 10 carbon atoms that may have a substituent", or the "linear or branched alkenyl of 2 to 6 carbon atoms that may have a substituent" represented by R₈ in the general formula (2c) include methyl, ethyl, n-propyl, isopropyl, n-butyl, isobutyl, tert-butyl, n-pentyl, isopentyl, neopentyl, n-hexyl, cyclopentyl, cyclohexyl, 1-adamantyl, 2-adamantyl, vinyl, allyl, isopropenyl, and 2-butenyl.

Specific examples of the "substituent" in the "linear or branched alkyl of 1 to 6 carbon atoms that has a substituent", the "cycloalkyl of 5 to 10 carbon atoms that has a substituent", or the "linear or branched alkenyl of 2 to 6 carbon atoms that has a substituent" represented by R₈ in the general formula (2c) include a deuterium atom; cyano; nitro; halogen atoms such as a fluorine atom, a chlorine atom, a bromine atom, and an iodine atom; linear or branched alkyloxys of 1 to 6 carbon atoms such as methyloxy, ethyloxy, and propyloxy; alkenyls such as vinyl and allyl; aryloxys such as phenyloxy and tolyloxy; arylalkyloxys such as benzyloxy and phenethyloxy; aromatic hydrocarbon groups or condensed polycyclic aromatic groups such as phenyl, biphenylyl, terphenylyl, naphthyl, anthracenyl, phenanthrenyl, fluorenyl, indenyl, pyrenyl, perylenyl, fluoranthenyl, and triphenylenyl; aromatic heterocyclic groups such as pyridyl, thienyl, furyl, pyrrolyl, quinolyl, isoquinolyl, benzofuranyl, benzothienyl, indolyl, carbazolyl, benzoxazolyl, benzothiazolyl, quinoxalinyl, benzoimidazolyl, pyrazolyl, dibenzofuranyl, dibenzothienyl, and carbolinyl. These substituents may be further substituted with the exemplified substituents above. These substituents may bind to each other via a single bond, substituted or unsubstituted methylene, an oxygen atom, or a sulfur atom to form a ring.

Specific examples of the "linear or branched alkyloxy of 1 to 6 carbon atoms" or the "cycloalkyloxy of 5 to 10 carbon atoms" in the "linear or branched alkyloxy of 1 to 6 carbon atoms that may have a substituent" or the "cycloalkyloxy of 5 to 10 carbon atoms that may have a substituent" represented by R₈ in the general formula (2c) include methyloxy, ethyloxy, n-propyloxy, isopropyloxy, n-butyloxy, tert-butyloxy, n-pentyloxy, n-hexyloxy, cyclopentyloxy, cyclohexyloxy, cycloheptyloxy, cyclooctyloxy, 1-adamantyloxy, and 2-adamantyloxy.

Examples of the "substituent" in the "linear or branched alkyloxy of 1 to 6 carbon atoms that has a substituent" or the "cycloalkyloxy of 5 to 10 carbon atoms that has a substituent" represented by R₈ in the general formula (2c) include the same substituents exemplified as the "substituent" in the "linear or branched alkyl of 1 to 6 carbon atoms that has a substituent", the "cycloalkyl of 5 to 10 carbon atoms that has a substituent", or the "linear or branched alkenyl of 2 to 6 carbon atoms that has a substituent" represented by R₈ in the general formula (2c), and possible embodiments may also be the same embodiments as the exemplified embodiments.

Examples of the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by R₈ in the general formula (2c) include the same groups exemplified as the groups for the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by Ar₁ to Ar₄ in the general formula (1).

These groups may have a substituent, and examples of the substituent include the same substituents exemplified as the "substituent" in the "substituted aromatic hydrocarbon group", the "substituted aromatic heterocyclic group", or the "substituted condensed polycyclic aromatic group" represented by Ar₁ to Ar₄ in the general formula (1), and possible embodiments may also be the same embodiments as the exemplified embodiments.

Specific examples of the "aryloxy" in the "substituted or unsubstituted aryloxy" represented by R₈ in the general formula (2c) include phenyloxy, biphenylyloxy, terphenylyloxy, naphthyloxy, anthracenyloxy, phenanthrenyloxy, fluorenyloxy, indenyloxy, pyrenyloxy, and perylenyloxy.

These groups may have a substituent, and examples of the substituent include the same substituents exemplified as the "substituent" in the "substituted aromatic hydrocarbon group", the "substituted aromatic heterocyclic group", or the "substituted condensed polycyclic aromatic group" represented by Ar₁ to Ar₄ in the general formula (1), and possible embodiments may also be the same embodiments as the exemplified embodiments.

Examples of the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by Arn, Ar₁₂, and Ar₁₃ in the general formula (2c) include the same groups exemplified as the groups for the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by Ar₁ to Ar₄ in the general formula (1).

These groups may have a substituent, and examples of the substituent include the same substituents exemplified as the "substituent" in the "substituted aromatic hydrocarbon group", the "substituted aromatic heterocyclic group", or the "substituted condensed polycyclic aromatic group" represented by Ar₁ to Ar₄ in the general formula (1), and possible embodiments may also be the same embodiments as the exemplified embodiments.

The "substituent" in the "substituted aromatic hydrocarbon group", the "substituted aromatic heterocyclic group", or the "substituted condensed polycyclic aromatic group" represented by Ar₁ to Ar₄ in the general formula (1) is preferably a deuterium atom, linear or branched alkyl of 1 to 6 carbon atoms that may have a substituent, linear or branched alkenyl of 2 to 6 carbon atoms that may have a substituent, the "substituted or unsubstituted aromatic hydrocarbon group", or the "substituted or unsubstituted condensed polycyclic aromatic group", far preferably, a deuterium atom, phenyl, biphenylyl, naphthyl, or vinyl. It is also preferable that these groups bind to each other via a single bond to form a condensed aromatic ring.

A in the general formula (2c) is preferably the "divalent group of a substituted or unsubstituted aromatic hydrocarbon" or the "divalent group of substituted or unsubstituted condensed polycyclic aromatics", far preferably, a divalent group that results from the removal of two hydrogen atoms from benzene, biphenyl, naphthalene, or phenanthrene, particularly preferably, a divalent group that results from the removal of two hydrogen atoms from benzene.

The arylamine compounds of the general formula (1), for use in the organic EL device of the present invention, are used as a constitutive material of a hole transport layer and optionally used as a constitutive material of a hole injection layer of an organic EL device. The arylamine compounds of the general formula (1) have high hole mobility and are therefore the compounds as material of a hole transport layer and optionally as a material of a hole injection layer.

The compounds of the general formula (2c) having an anthracene ring structure, for use in the organic EL device of the present invention, are used as a constitutive material of an electron transport layer of an organic EL device. The compounds of the general formula (2c) having an anthracene ring structure excel in electron injection and transport abilities and are therefore the compounds as material of an electron transport layer.

The organic EL device of the present invention combines materials for an organic EL device exceling in hole and electron injection/transport performances, stability as a thin film and durability, taking carrier balance into consideration. Therefore, compared with the conventional organic EL devices, hole transport efficiency to the light emitting layer from the hole transport layer is improved. And electron transport efficiency to the light emitting layer from the electron transport layer is also improved. (Further, a combination of materials matching characteristics of selected luminous dopants is selected in an embodiment using specific luminous dopants (luminous dopants having a specific structure).) As a result, luminous efficiency is improved and driving voltage is decreased, and durability of the organic EL device can thereby be improved.

Thus, an organic EL device having high efficiency, low driving voltage and a long lifetime can be attained in the present invention.

The organic EL device of the present invention can achieve an organic EL device having high efficiency, low driving voltage and a long lifetime as a result of attaining efficient hole injection/transport to the light emitting layer from the hole transport layer and improving electron injection/transport efficiency to the light emitting layer from the hole transport layer by selecting specific arylamine compounds which can effectively exhibit hole injection/transport roles and selecting specific compounds having an anthracene ring structure which can effectively exhibit electron injection/transport roles.

An organic EL device having high efficiency, low driving voltage and a long lifetime can be achieved by selecting specific arylamine compounds and specific compounds having an anthracene ring structure, further selecting specific luminous dopants (luminous dopants having a specific structure) and combining those compounds so as to achieve good carrier balance.

The organic EL device of the present invention can improve luminous efficiency, driving voltage and durability of the conventional organic EL devices.

### Brief Description of the Drawings

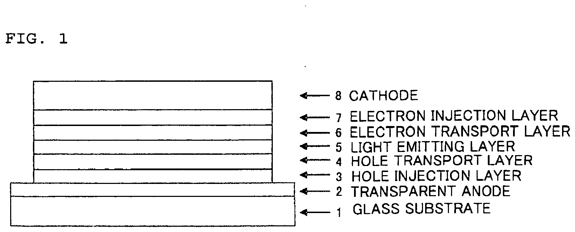

FIG. 1 is a diagram illustrating the configuration of the organic EL devices of Examples 45 and 46 and Comparative Examples 1 and 2.

The following presents specific examples of preferred compounds among the arylamine compounds of the general formula (1) used in the organic EL device of the present invention. The present invention, however, is not restricted to these compounds.

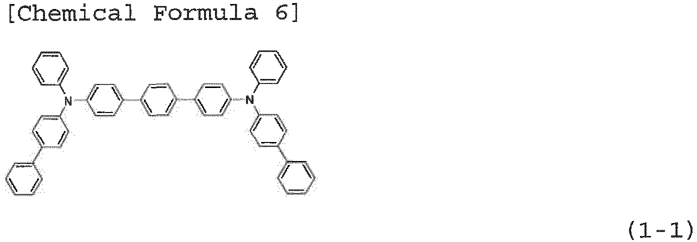

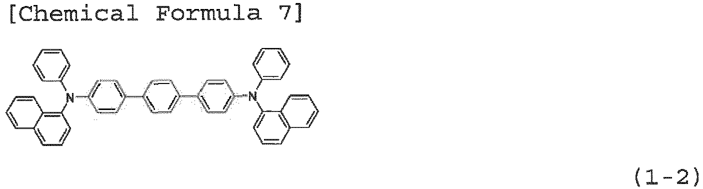

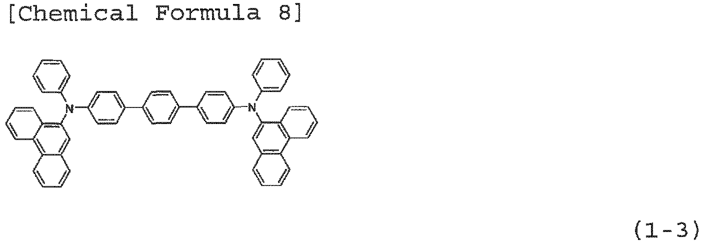

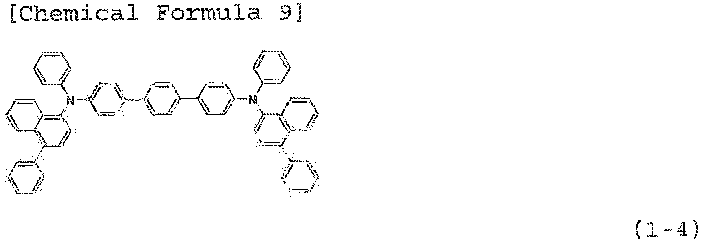

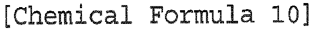

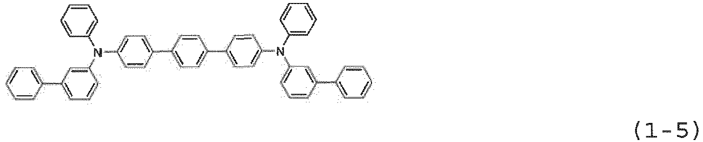

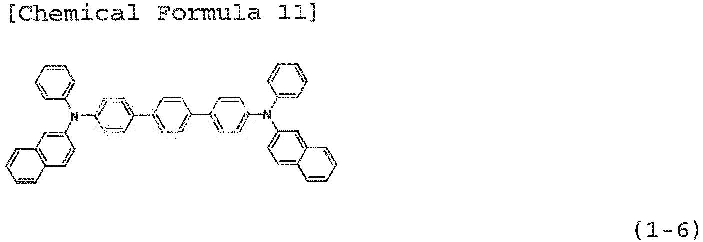

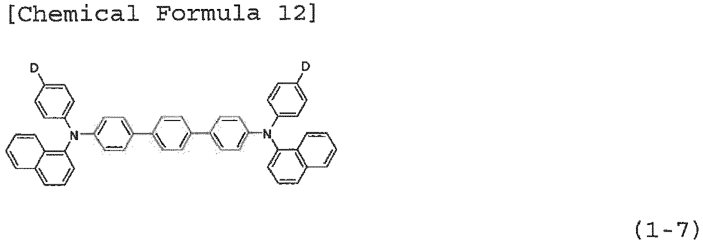

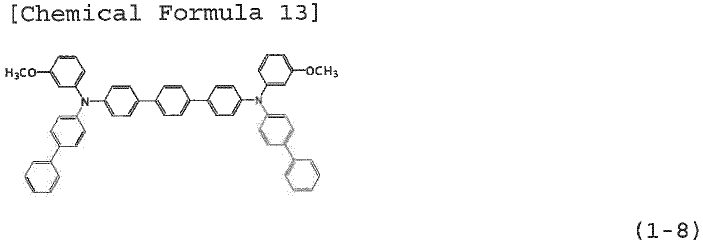

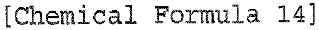

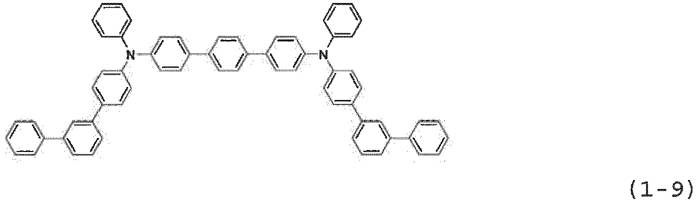

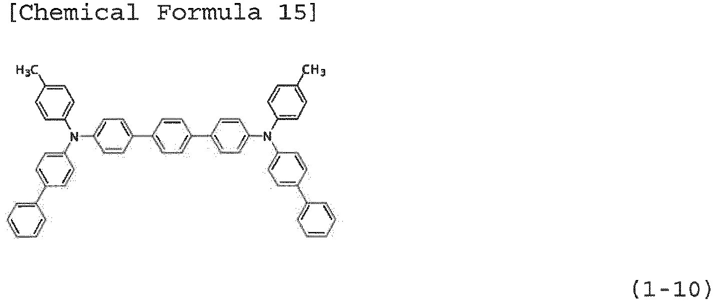

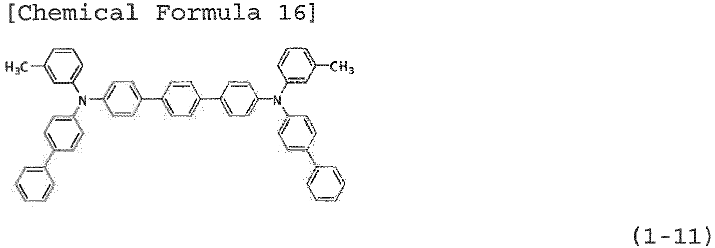

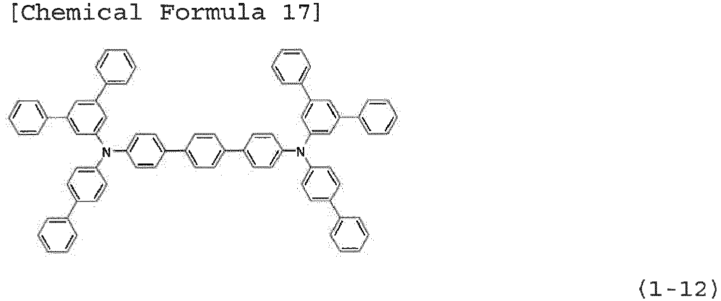

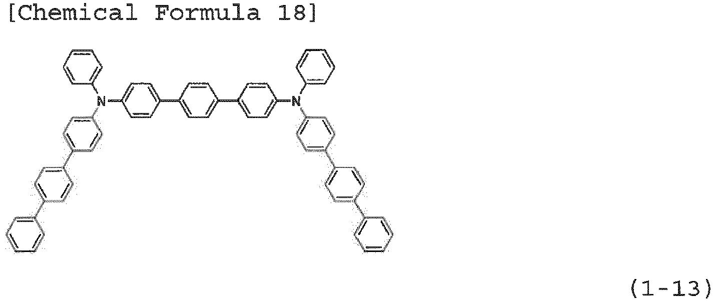

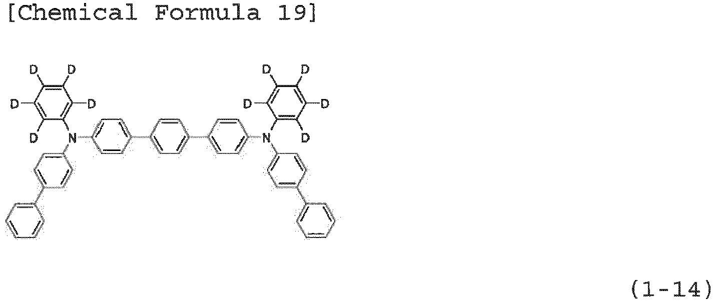

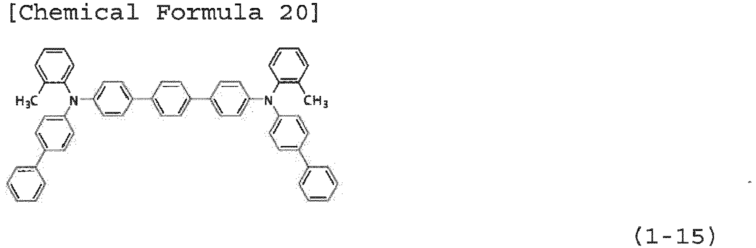

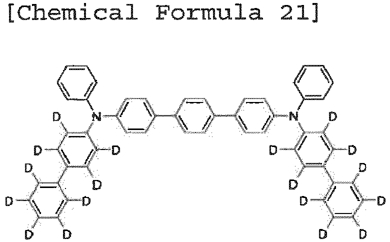

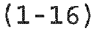

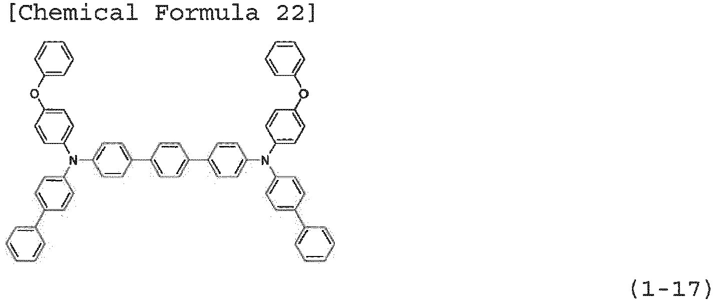

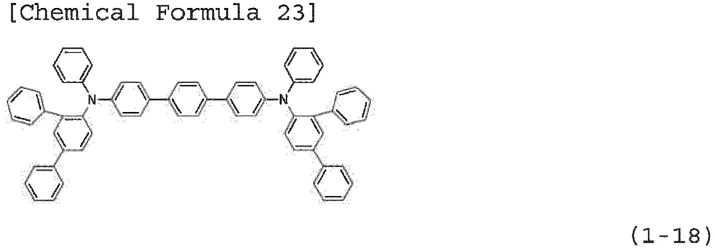

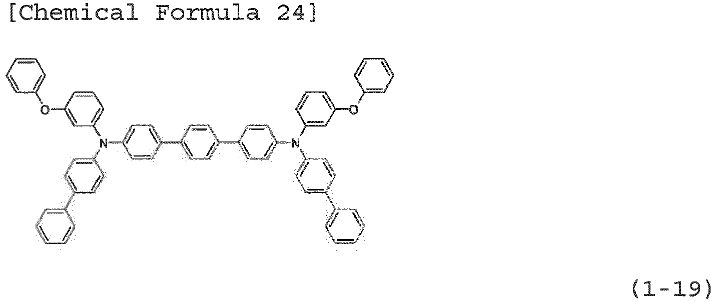

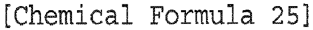

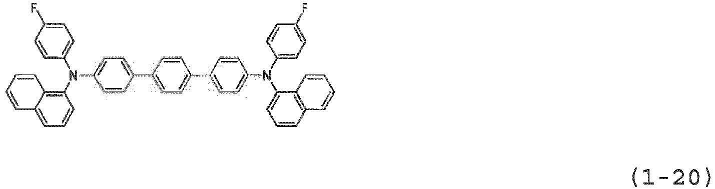

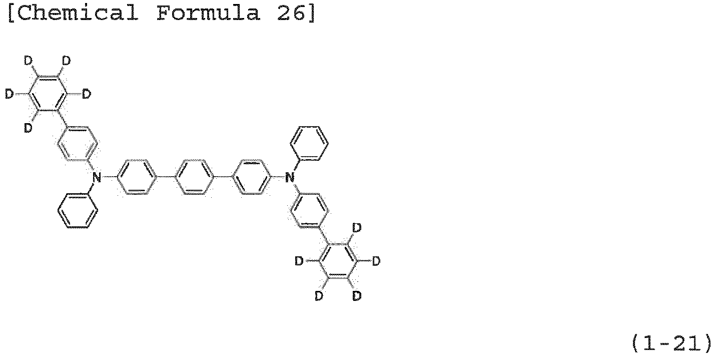

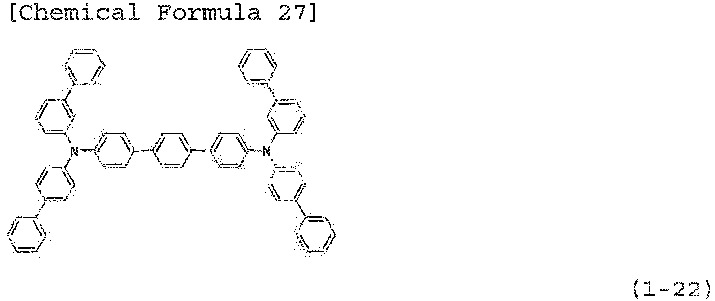

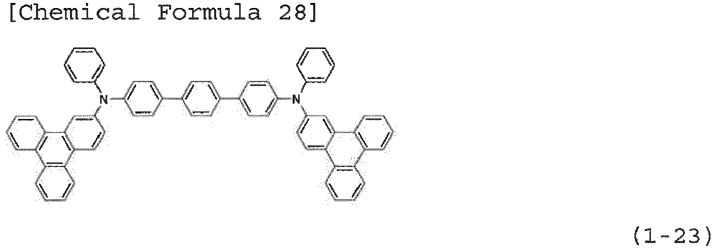

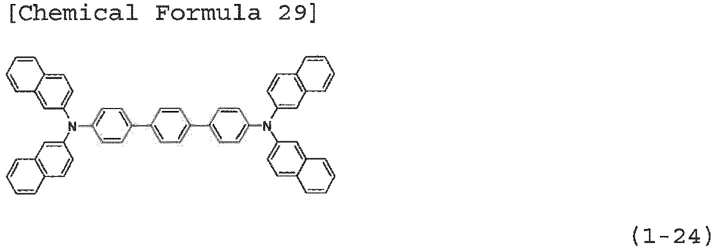

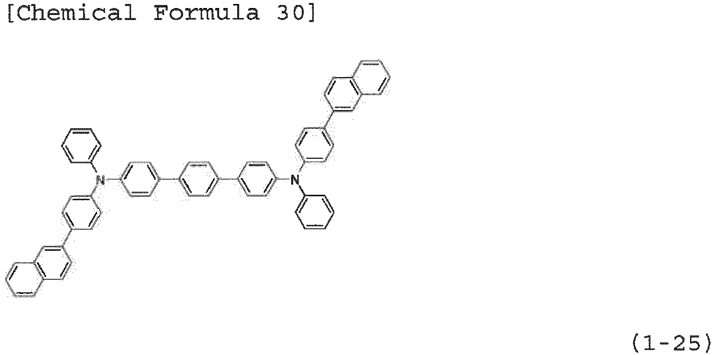

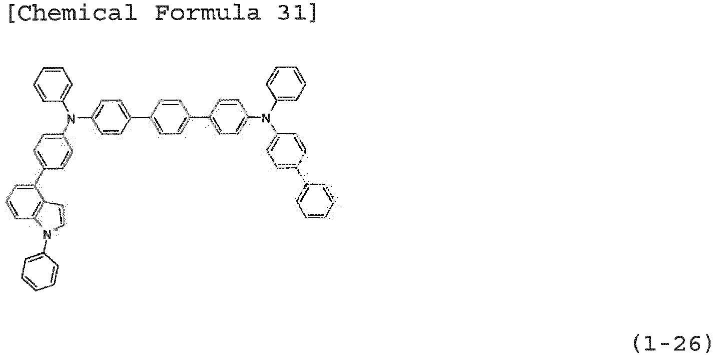

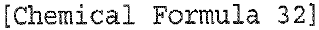

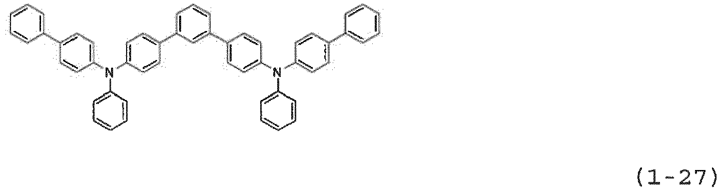

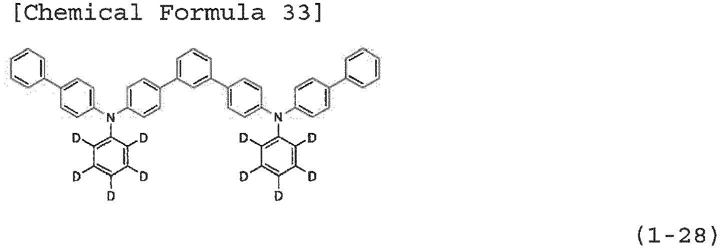

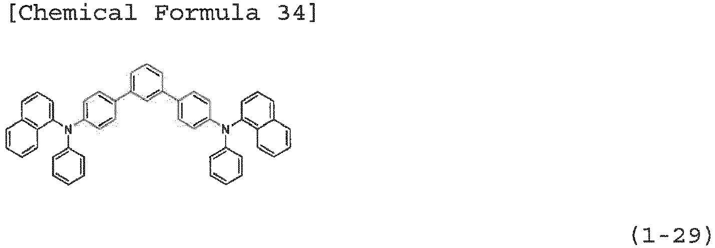

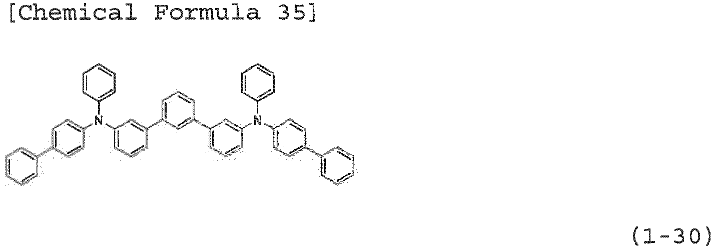

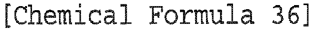

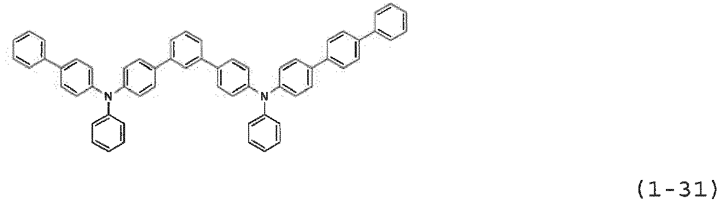

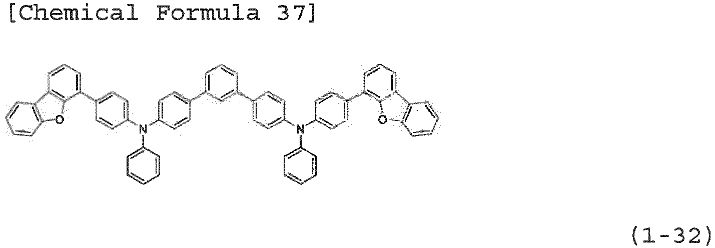

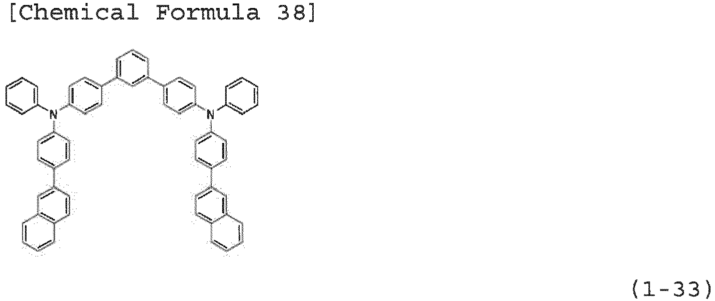

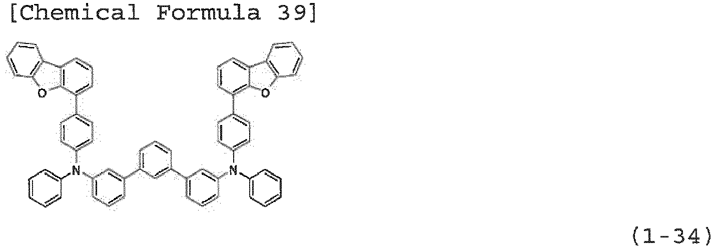

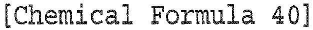

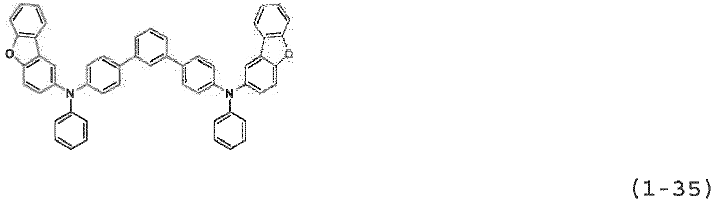

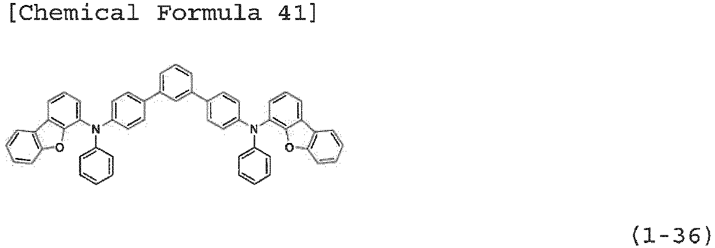

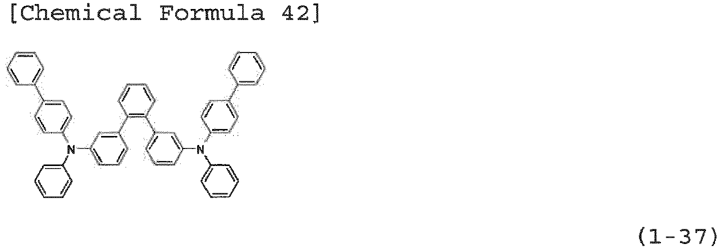

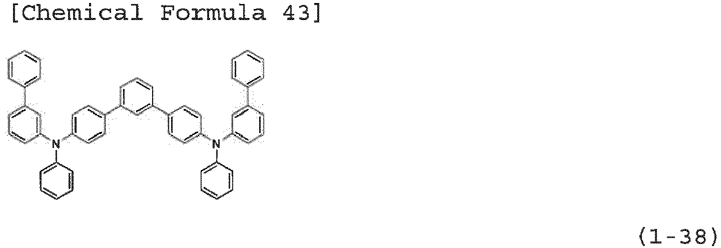

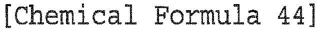

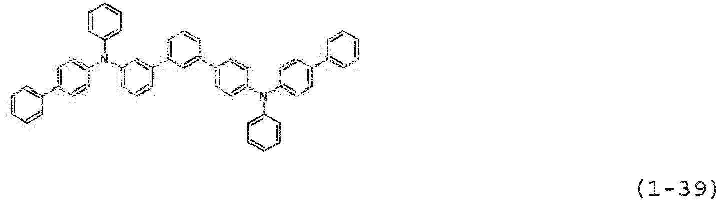

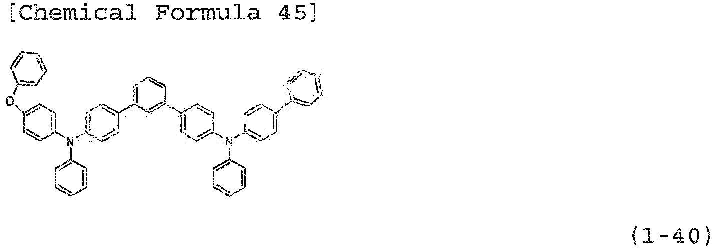

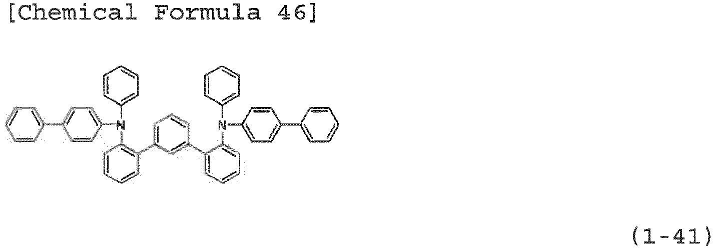

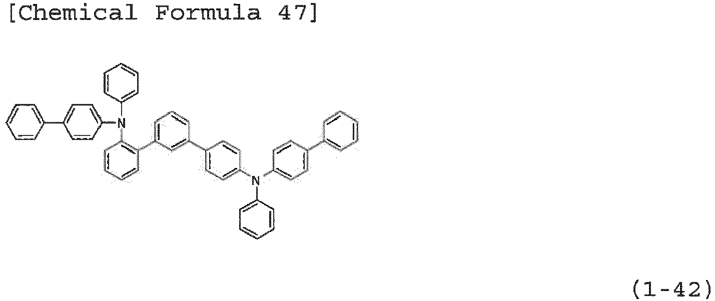

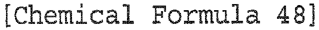

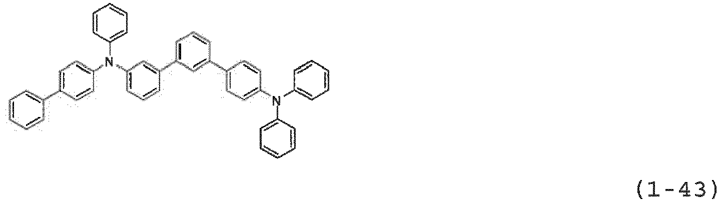

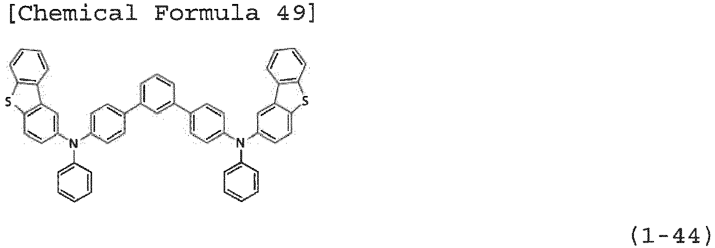

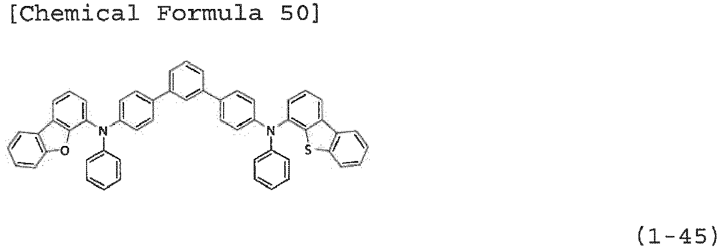

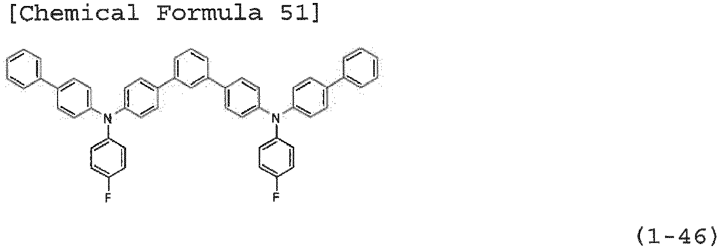

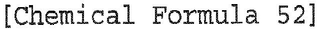

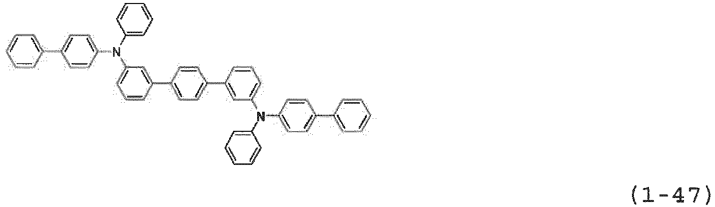

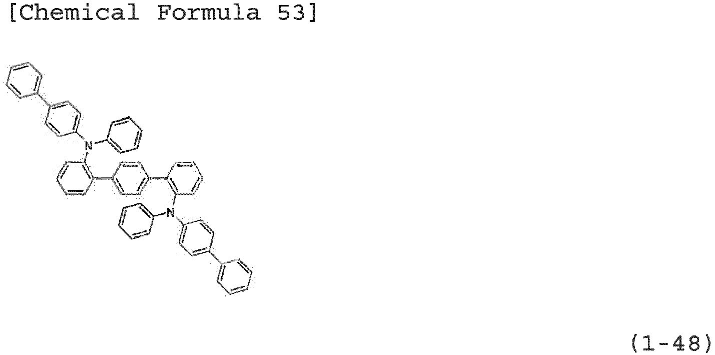

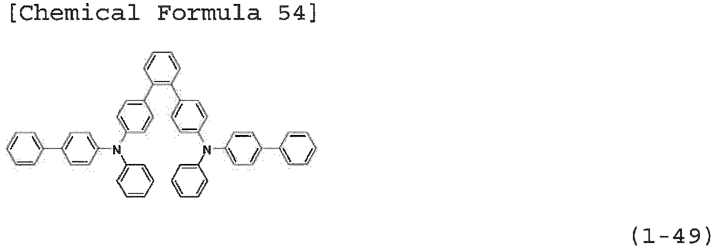

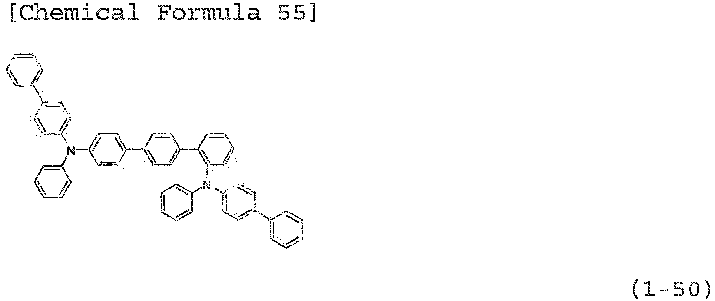

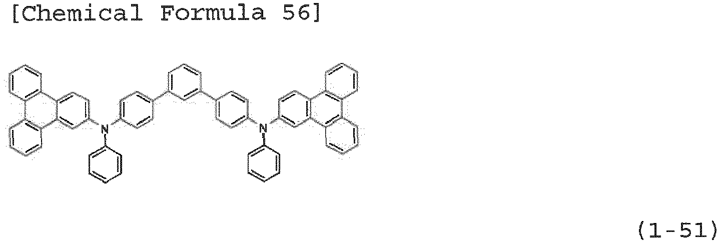

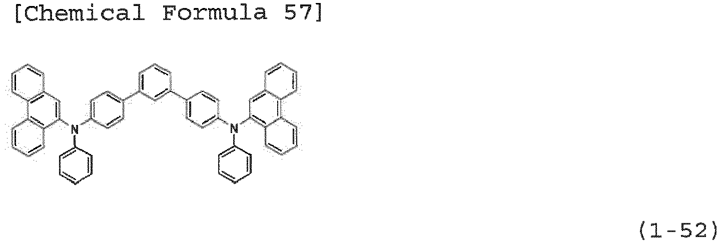

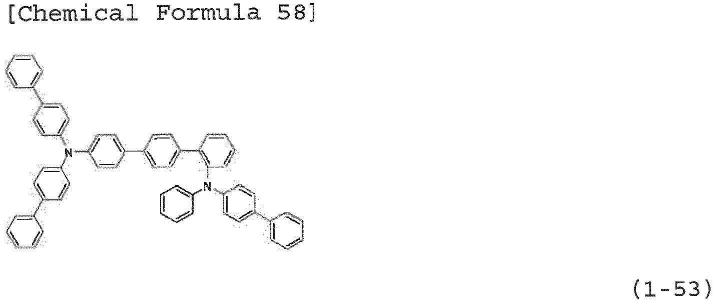

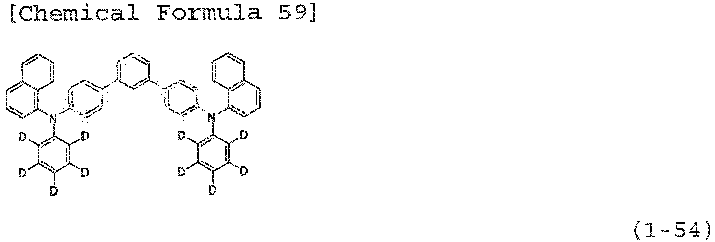

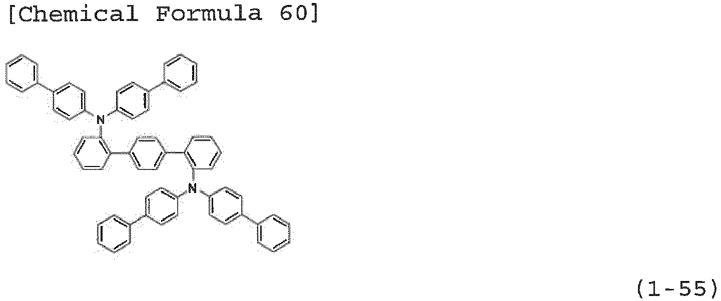

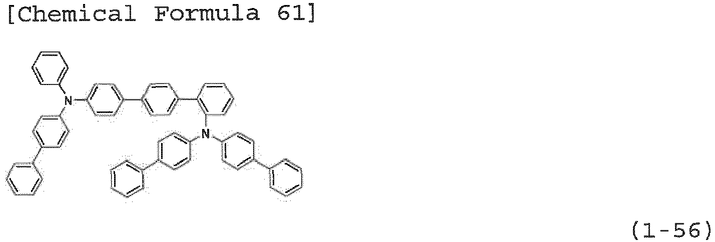

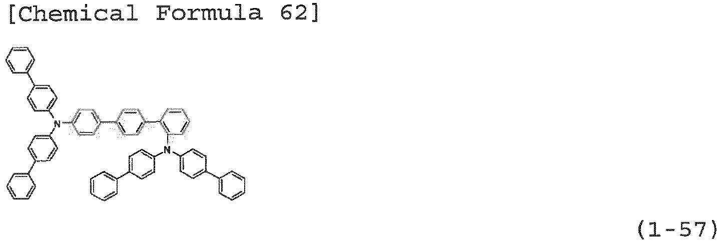

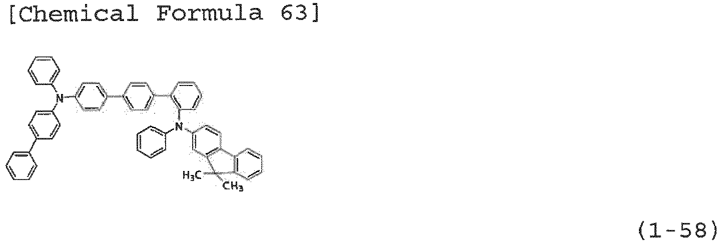

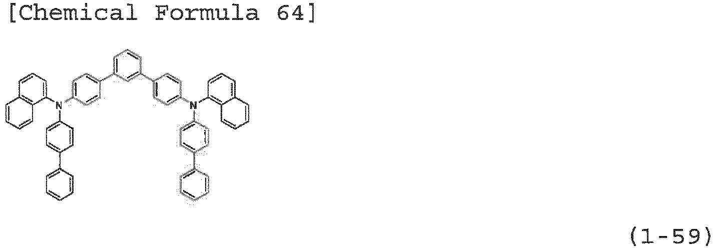

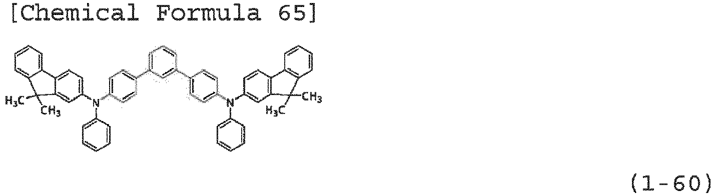

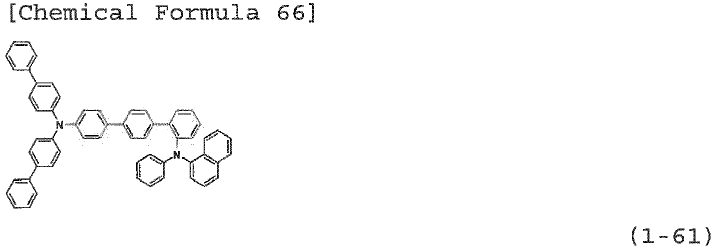

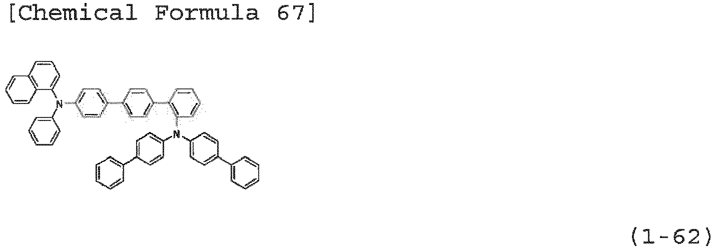

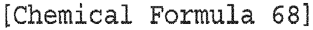

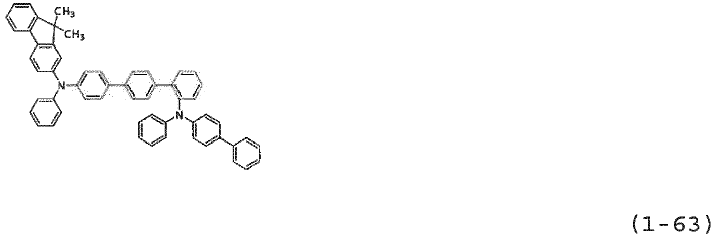

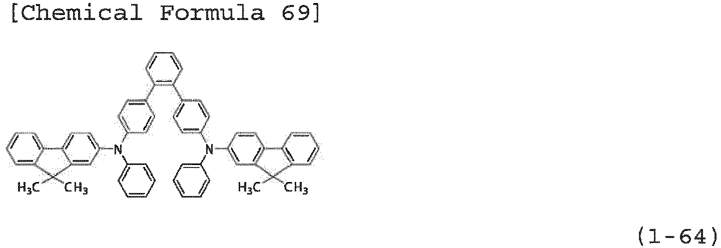

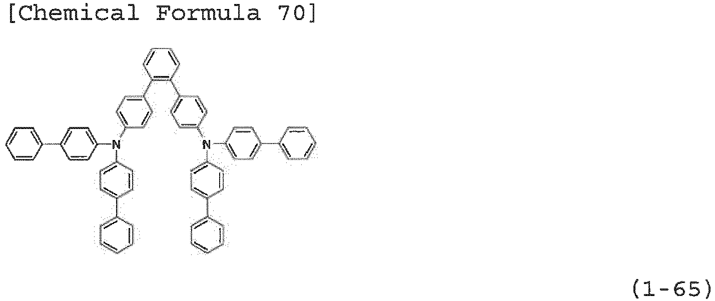

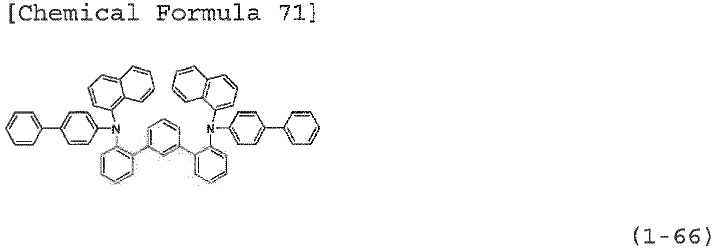

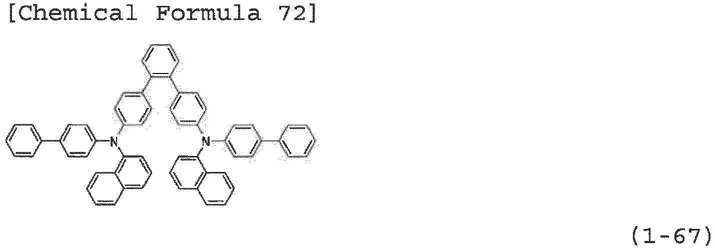

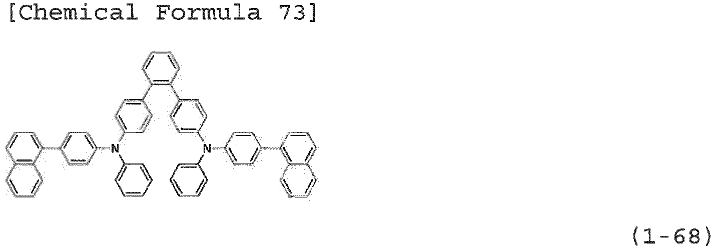

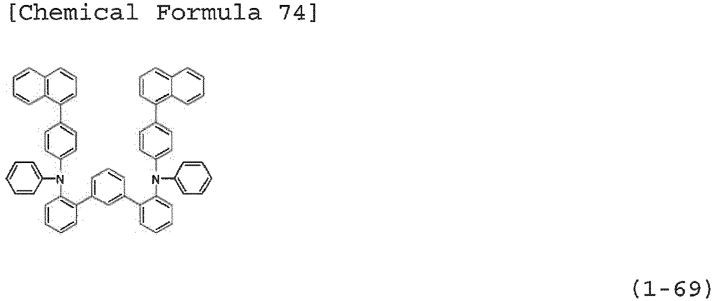

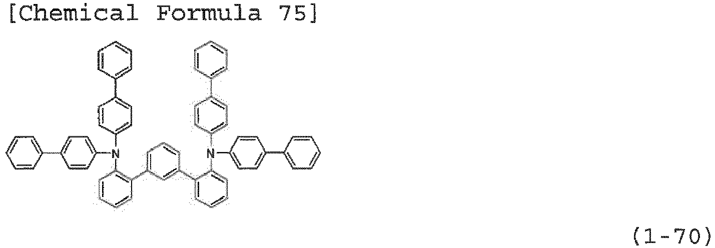

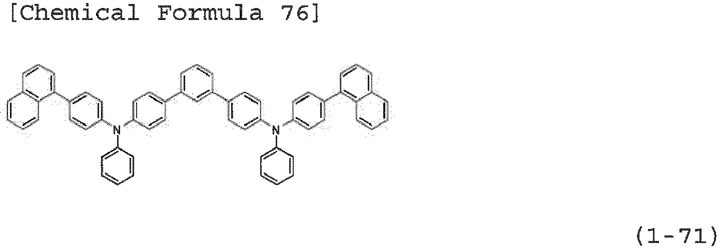

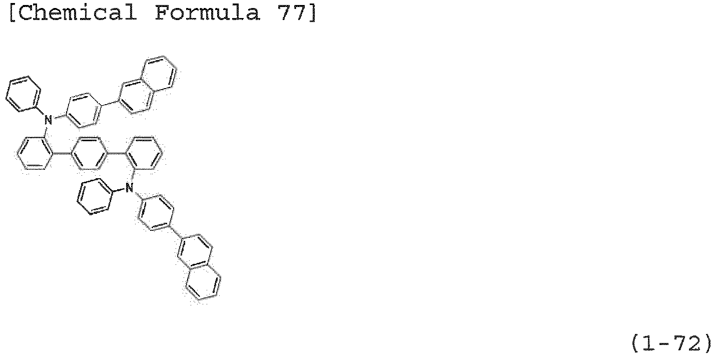

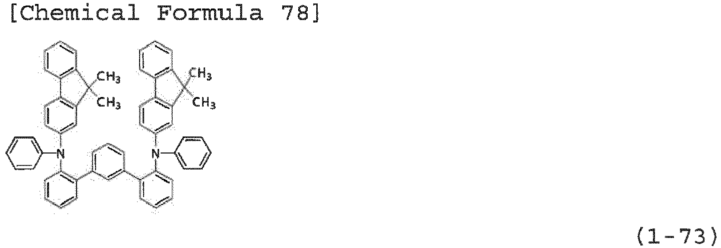

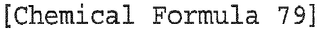

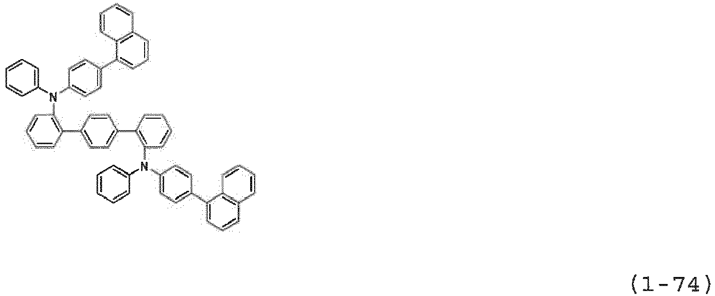

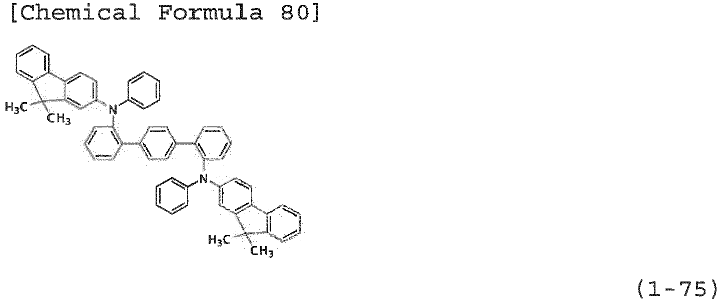

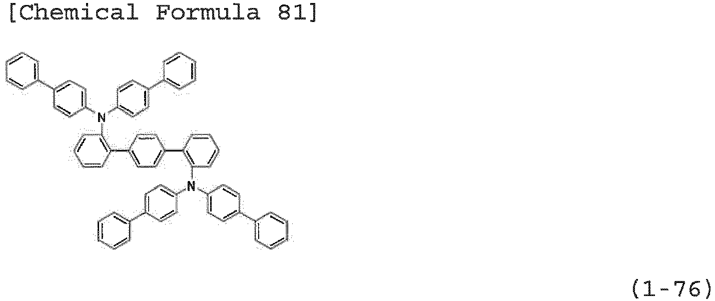

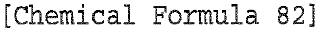

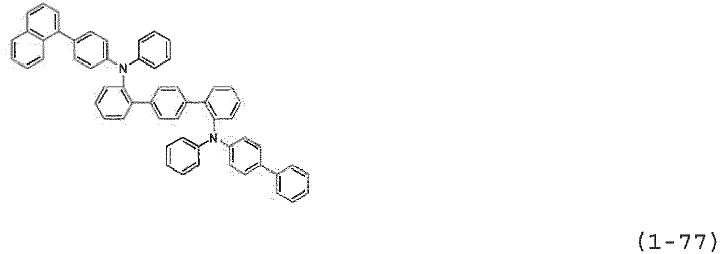

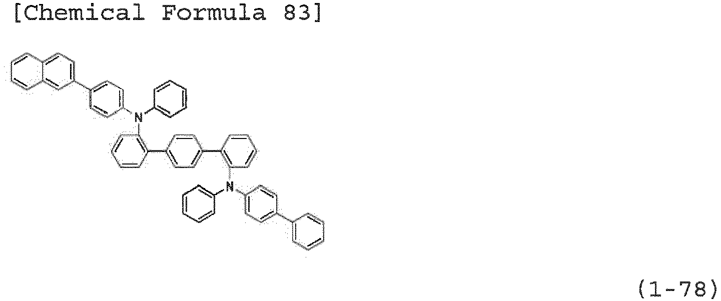

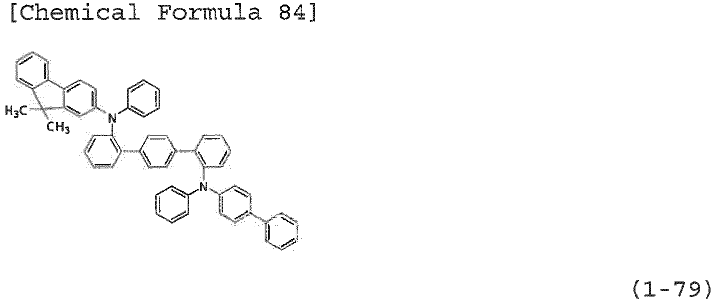

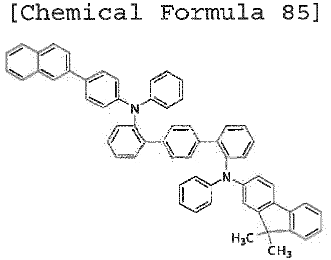

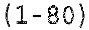

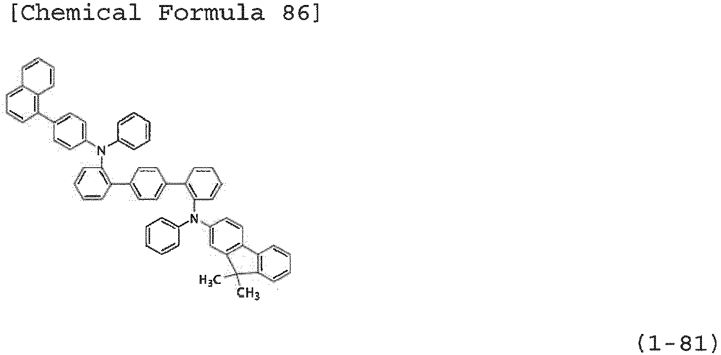

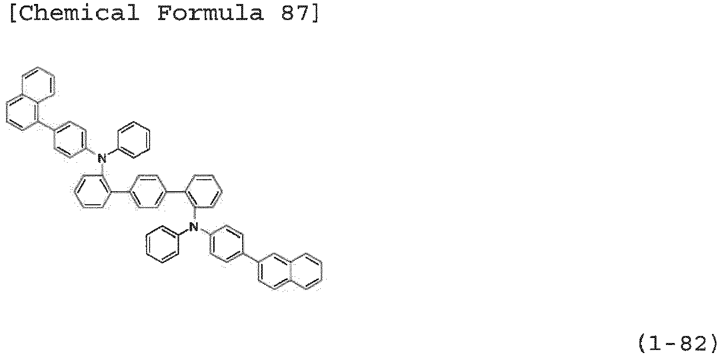

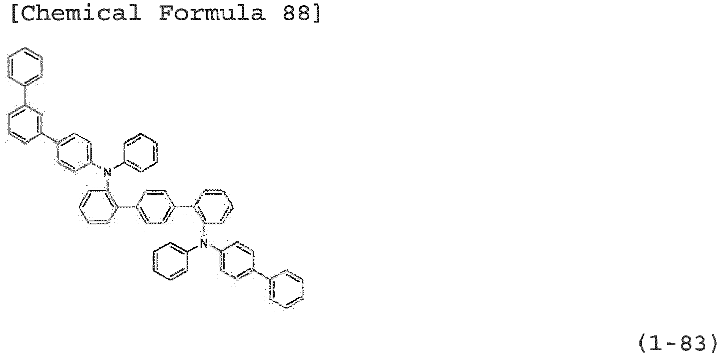

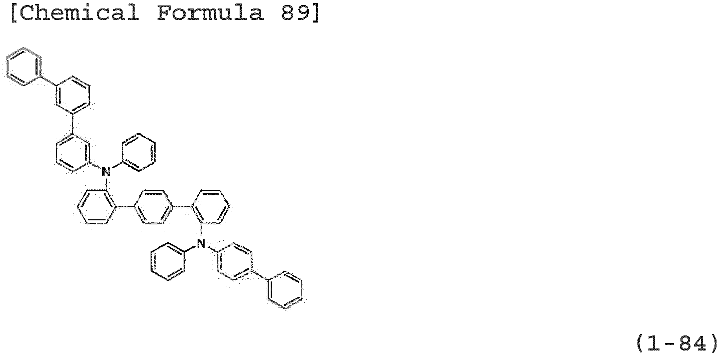

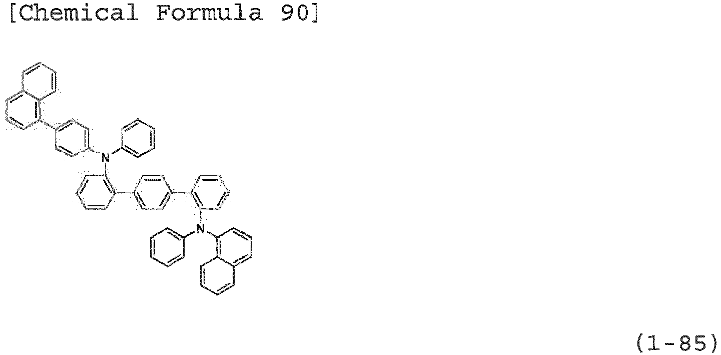

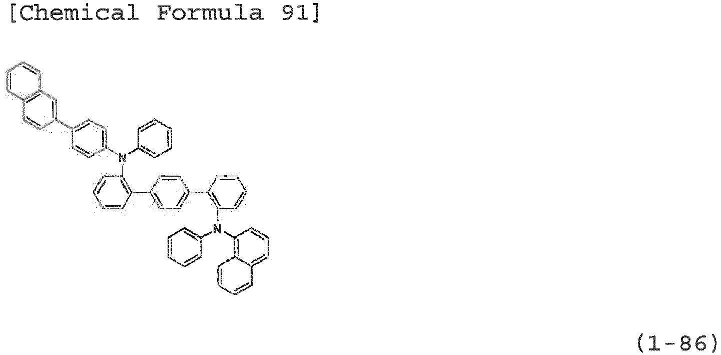

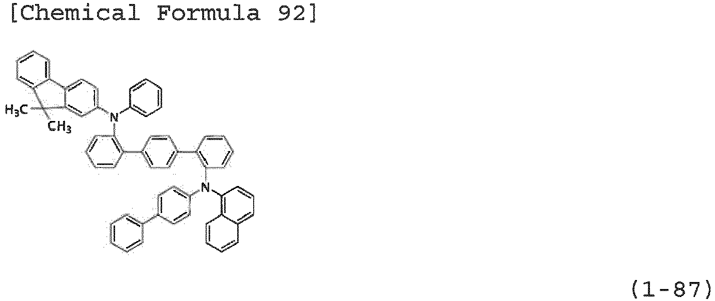

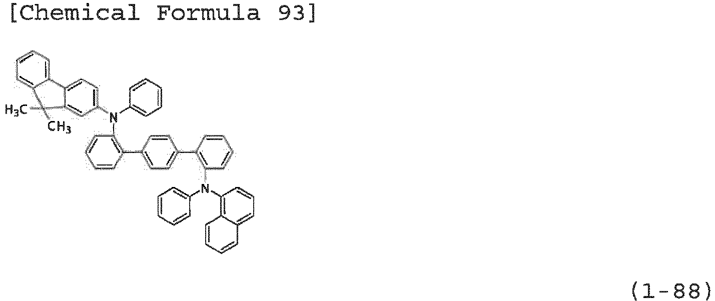

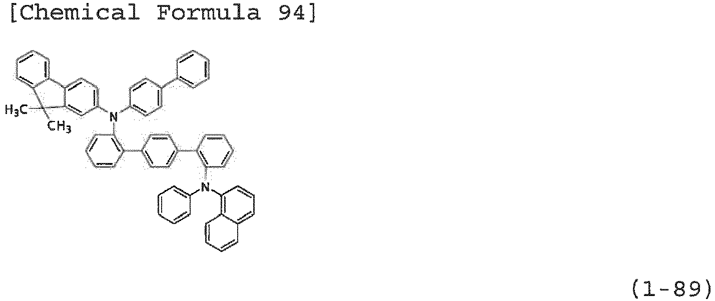

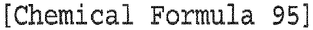

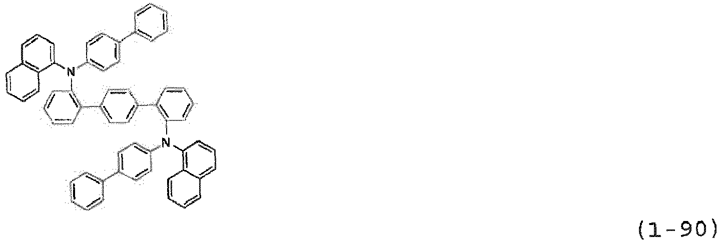

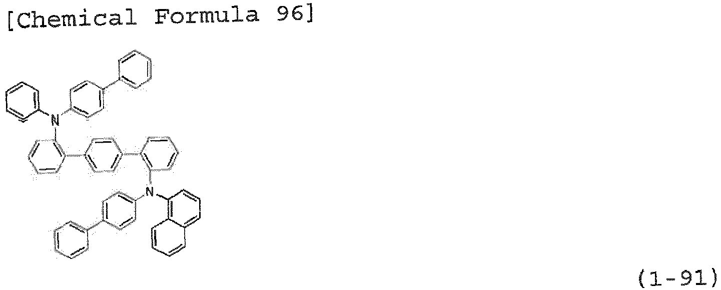

The arylamine compounds described above can be synthesized by a known method (refer to Patent Document 6, for example).

The following presents specific examples of preferred compounds among the compounds of the general formula (2c) used in the organic EL device of the present invention and having an anthracene ring structure. The present invention, however, is not restricted to these compounds.

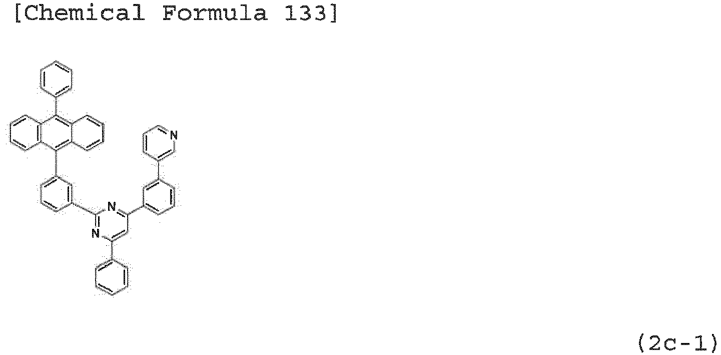

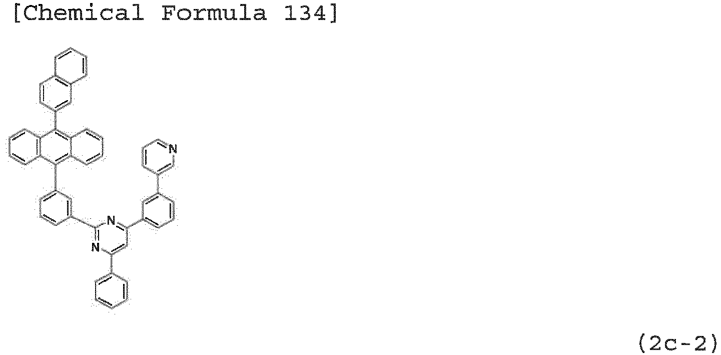

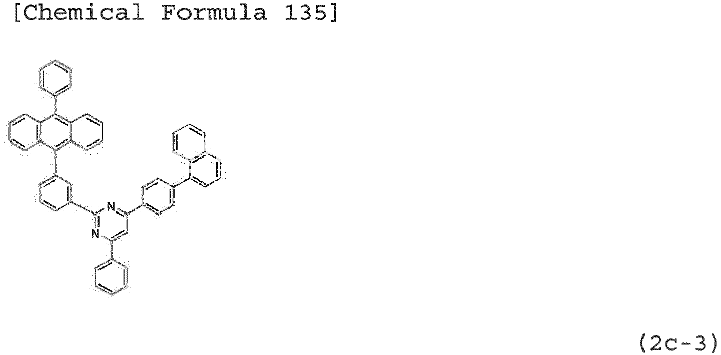

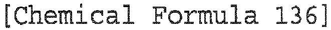

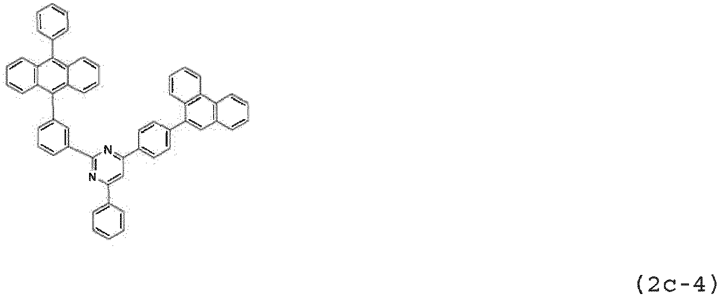

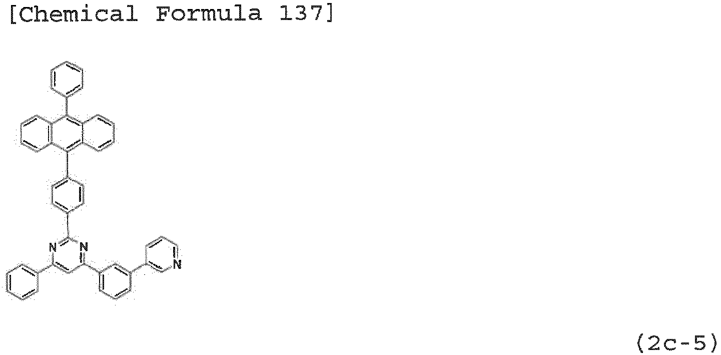

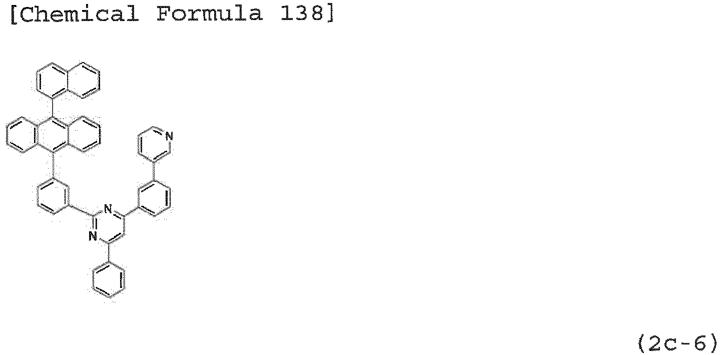

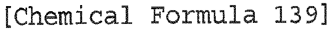

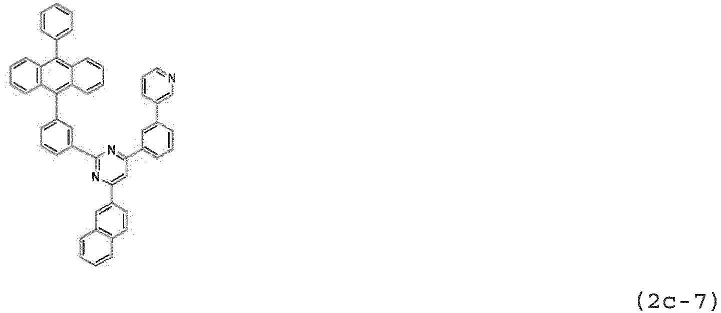

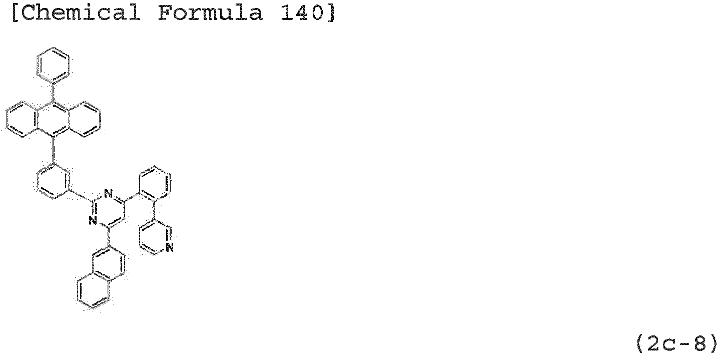

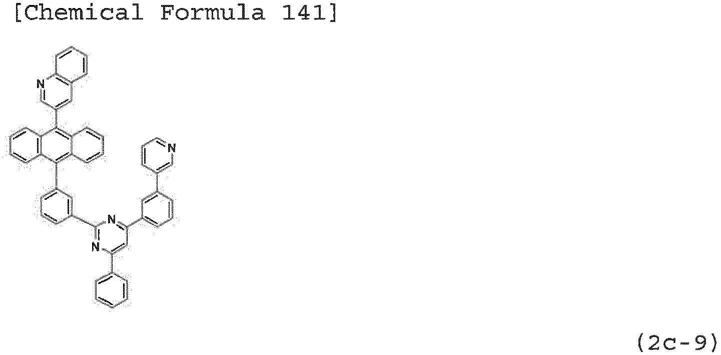

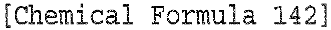

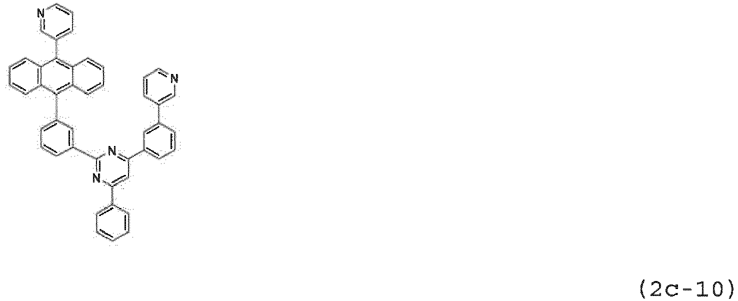

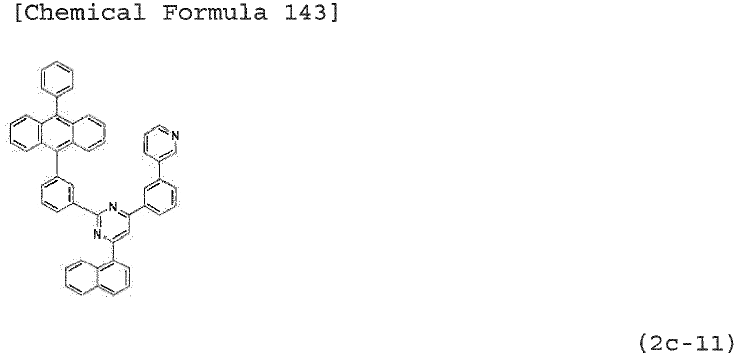

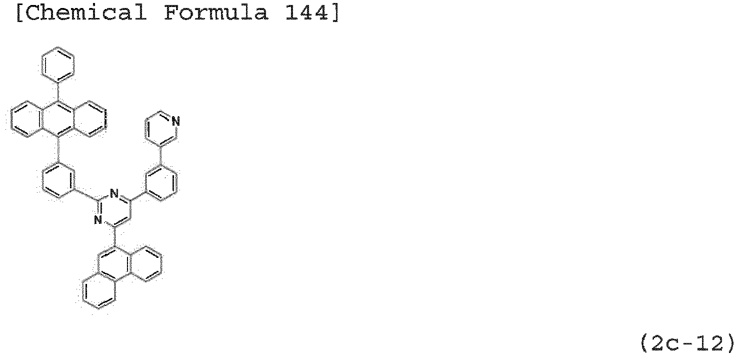

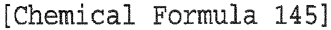

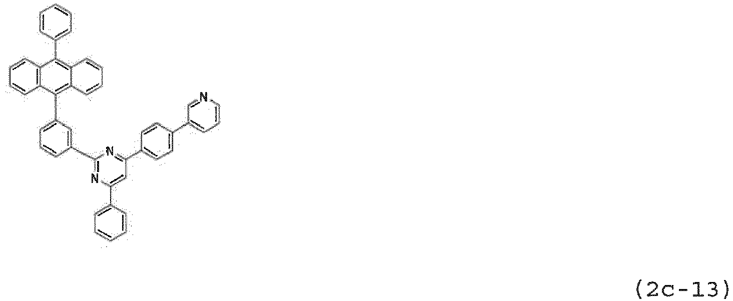

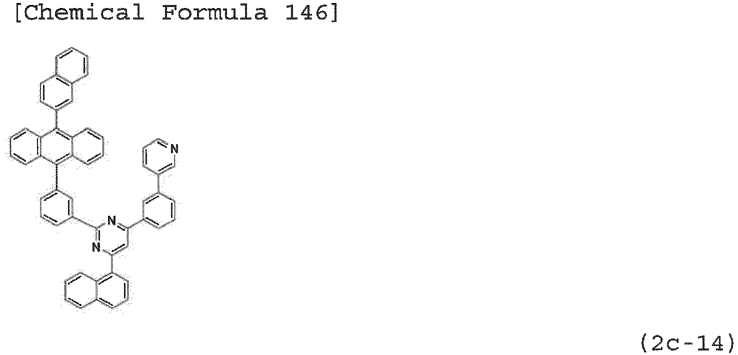

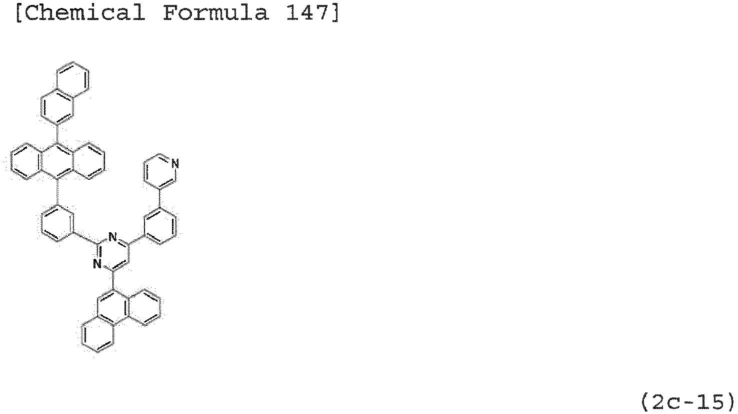

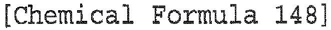

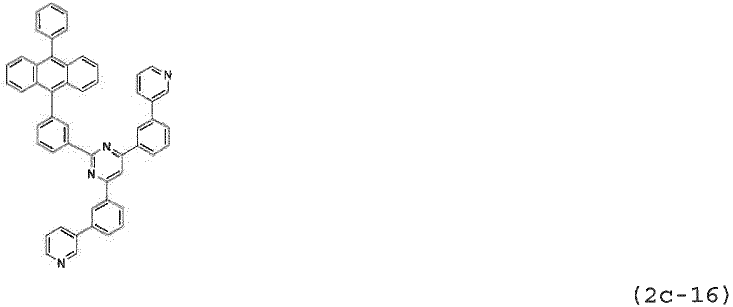

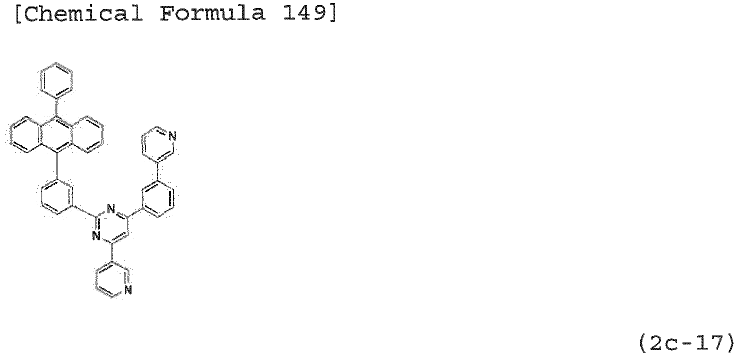

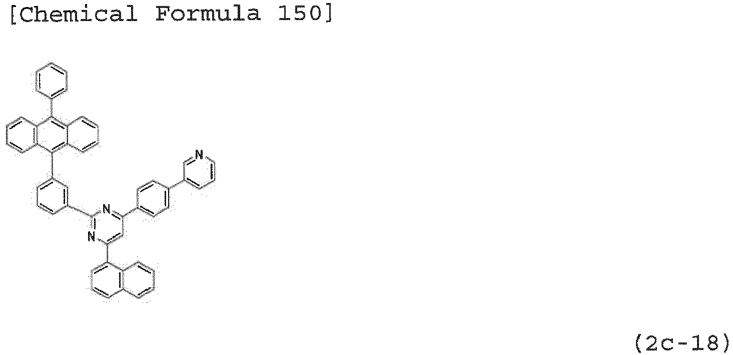

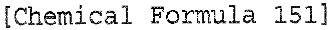

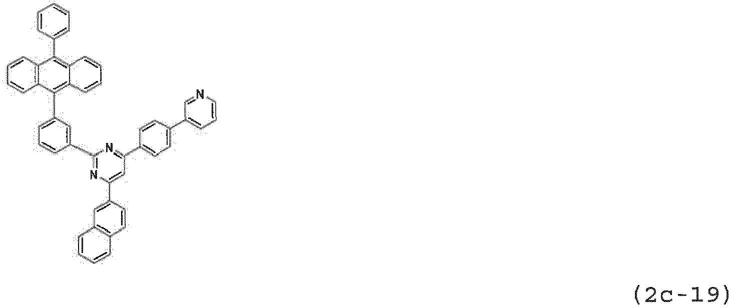

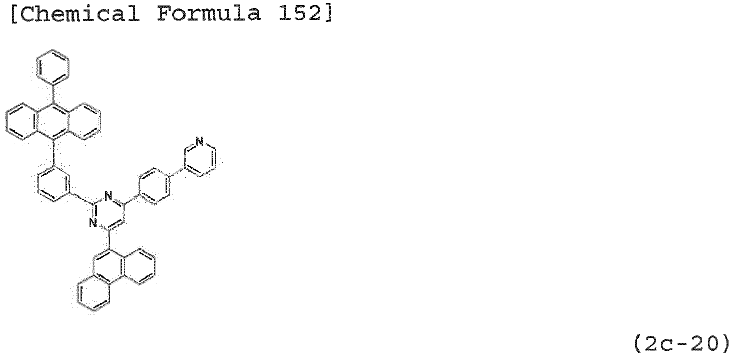

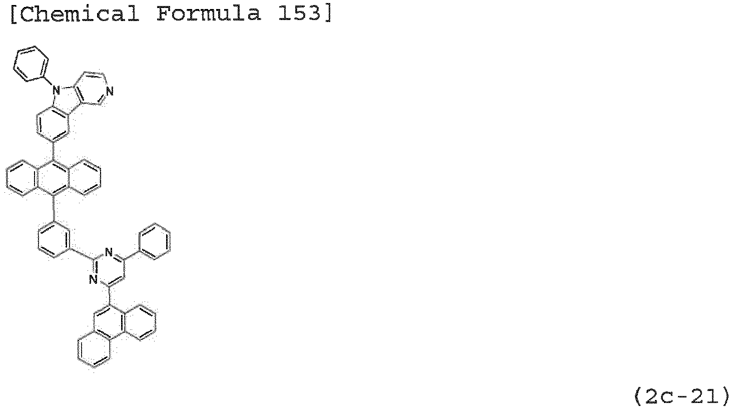

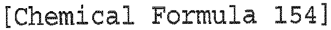

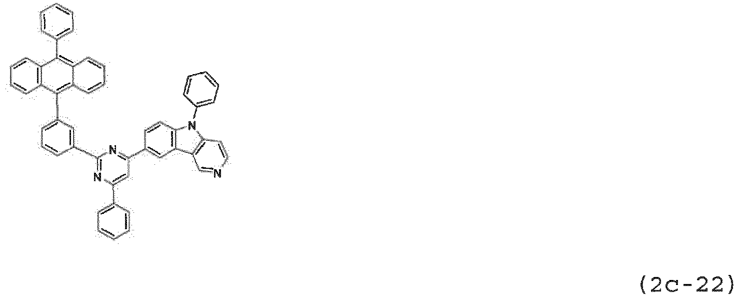

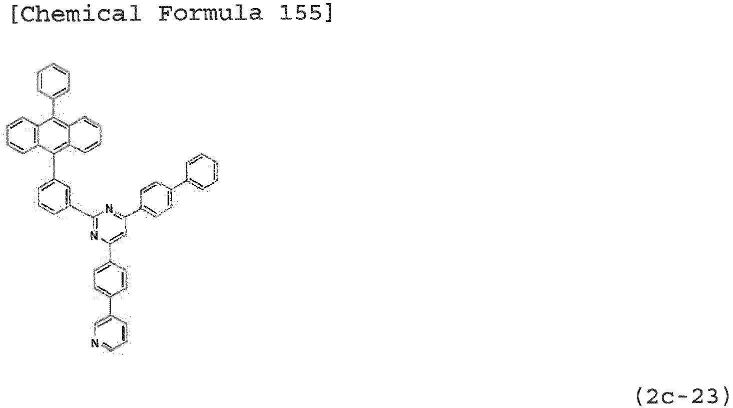

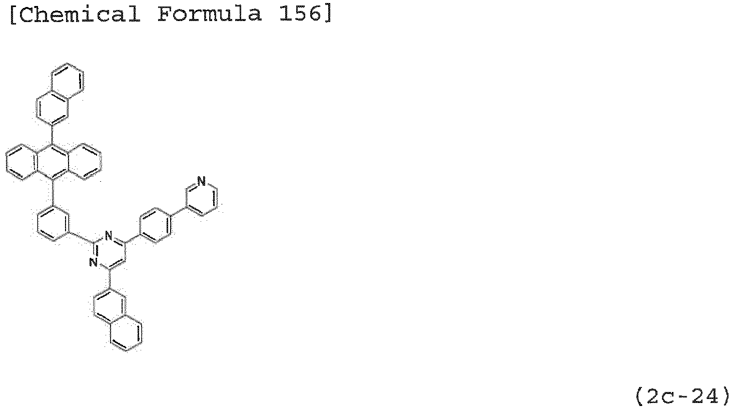

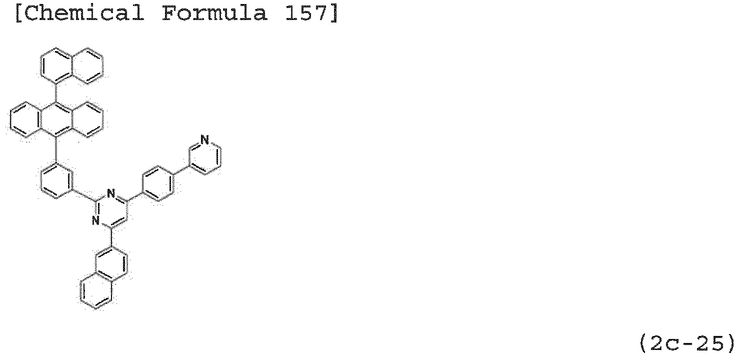

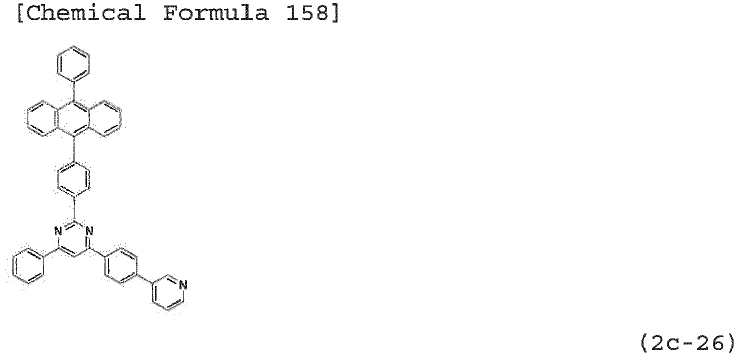

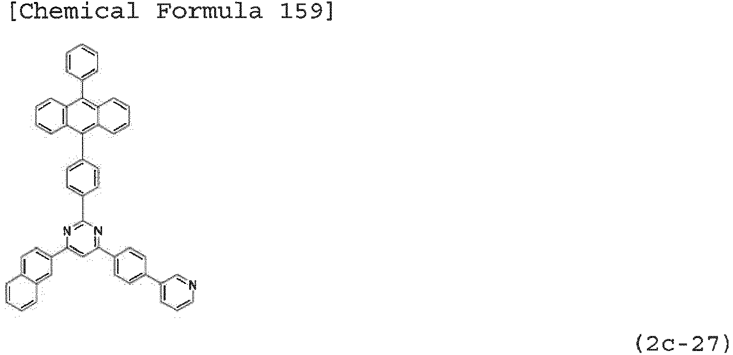

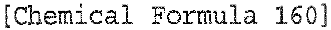

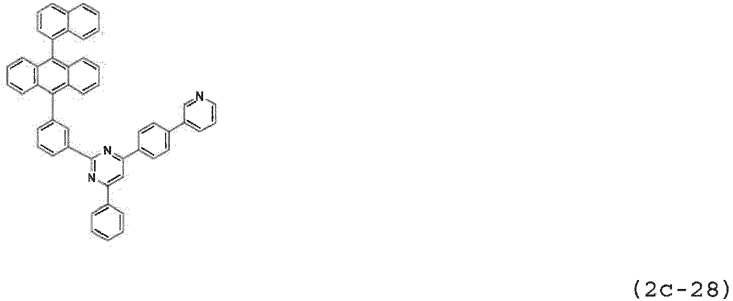

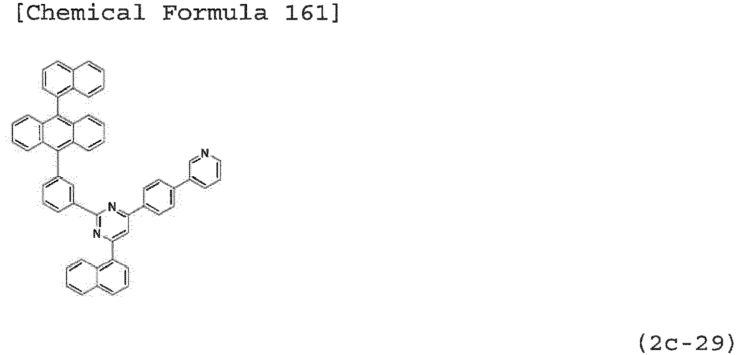

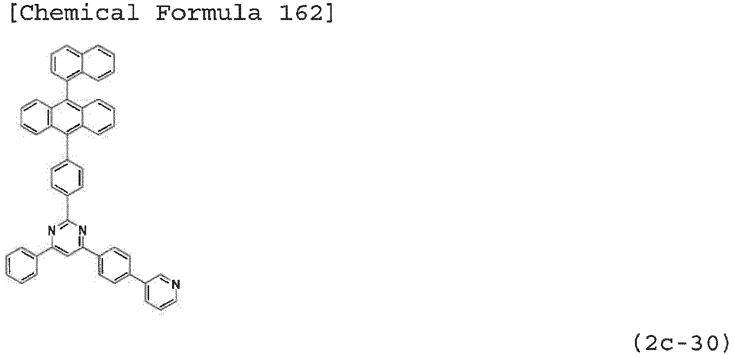

The compounds described above having an anthracene ring structure can be synthesized by a known method (refer to Patent Documents 7 to 9, for example).

The arylamine compounds of the general formula (1) and the compounds of the general formula (2c) having an anthracene ring structure were purified by methods such as column chromatography, adsorption using, for example, a silica gel, activated carbon, or activated clay, recrystallization or crystallization using a solvent, and a sublimation purification method. The compounds were identified by an NMR analysis. A melting point, a glass transition point (Tg), and a work function were measured as material property values. The melting point can be used as an index of vapor deposition, the glass transition point (Tg) as an index of stability in a thin-film state, and the work function as an index of hole transportability and hole blocking performance.

Other compounds used for the organic EL device of the present invention were purified by methods such as column chromatography, adsorption using, for example, a silica gel, activated carbon, or activated clay, and recrystallization or crystallization using a solvent, and finally purified by sublimation.

The melting point and the glass transition point (Tg) were measured by a high-sensitive differential scanning calorimeter (DSC3100SA produced by Bruker AXS) using powder.

For the measurement of the work function, a 100 nm-thick thin film was fabricated on an ITO substrate, and an ionization potential measuring device (PYS-202 produced by Sumitomo Heavy Industries, Ltd.) was used.

The organic EL device of the present invention may have a structure including an anode, a hole injection layer, a hole transport layer, a light emitting layer, an electron transport layer, an electron injection layer, and a cathode successively formed on a substrate, optionally with an electron blocking layer between the hole transport layer and the light emitting layer, and a hole blocking layer between the light emitting layer and the electron transport layer. Some of the organic layers in the multilayer structure may be omitted, or may serve more than one function. For example, a single organic layer may serve as the hole injection layer and the hole transport layer, or as the electron injection layer and the electron transport layer. Further, the organic layers having a same function may have a laminate structure of two or more layers, for example, the light emitting layers may have a laminate structure of two or more layers, or the electron transport layers may have a laminate structure of two or more layers. According to the present invention, the hole transport layer is a single layer.

Electrode materials with high work functions such as ITO and gold are used as the anode of the organic EL device of the present invention. The hole injection layer of the organic EL device of the present invention may be made of, for example, material such as starburst-type triphenylamine derivatives and various triphenylamine tetramers; porphyrin compounds as represented by copper phthalocyanine; accepting heterocyclic compounds such as hexacyano azatriphenylene; and coating-type polymer materials, in addition to the arylamine compounds of the general formula (1). These materials may be formed into a thin film by a vapor deposition method or other known methods such as a spin coating method and an inkjet method.

The arylamine compounds of the general formula (1) are used as the hole transport layer of the organic EL device of the present invention, and are used as a single layer. These materials may be formed into a thin-film by a vapor deposition method or other known methods such as a spin coating method and an inkjet method.

Further, material used for the hole injection layer or the hole transport layer may be obtained by p-doping trisbromophenylamine hexachloroantimony, radialene derivative (refer to WO2014/009310, for example), or the like into the material commonly used for these layers, or may be, for example, polymer compounds each having a structure of a benzidine derivative such as TPD as a part of the compound structure.

In the case where the hole transport layer of the organic EL device has a laminate structure of two or more layers, which is, however, not within the scope of the present invention, examples of material used for the hole transport layer of second and succeeding layers can be arylamine compounds having a structure in which two triphenylamine structures are joined within a molecule via a single bond or a divalent group that does not contain a heteroatom such as benzidine derivatives such as N,N'-diphenyl-N,N'-di(m-tolyl)benzidine (TPD), N,N'-diphenyl-N,N'-di(α-naphthyl)benzidine (NPD), and N,N,N',N'-tetrabiphenylylbenzidine; and 1,1-bis[4-(di-4-tolylamino)phenyl]cyclohexane (TAPC), arylamine compounds having a structure in which four triphenylamine structures are joined within a molecule via a single bond or a divalent group that does not contain a heteroatom, and various triphenylamine trimers, in addition to the arylamine compounds of the general formula (1). These may be individually deposited for film forming, may be used as a single layer deposited mixed with other materials, or may be formed as a laminate of individually deposited layers, a laminate of mixedly deposited layers, or a laminate of the individually deposited layer and the mixedly deposited layer. Examples of material used for the hole injection/transport layer can be coating-type polymer materials such as poly(3,4-ethylenedioxythiophene) (PEDOT)/poly(styrene sulfonate) (PSS). These materials may be formed into a thin-film by a vapor deposition method or other known methods such as a spin coating method and an inkjet method.

Examples of material used for the electron blocking layer of the organic EL device of the present invention can be compounds having an electron blocking effect, including, for example, arylamine compounds having a structure in which four triphenylamine structures are joined within a molecule via a single bond or a divalent group that does not contain a heteroatom, arylamine compounds having a structure in which two triphenylamine structures are joined within a molecule via a single bond or a divalent group that does not contain a heteroatom, carbazole derivatives such as 4,4',4''-tri(N-carbazolyl)triphenylamine (TCTA), 9,9-bis[4-(carbazol-9-yl)phenyl]fluorene, 1,3-bis(carbazol-9-yl)benzene (mCP), and 2,2-bis(4-carbazol-9-ylphenyl)adamantane (Ad-Cz); and compounds having a triphenylsilyl group and a triarylamine structure, as represented by 9-[4-(carbazol-9-yl)phenyl]-9-[4-(triphenylsilyl)phenyl]-9H-fluorene, in addition to the arylamine compounds of the general formula (1). These may be individually deposited for film forming, may be used as a single layer deposited mixed with other materials, or may be formed as a laminate of individually deposited layers, a laminate of mixedly deposited layers, or a laminate of the individually deposited layer and the mixedly deposited layer. These materials may be formed into a thin-film by using a vapor deposition method or other known methods such as a spin coating method and an inkjet method.

Examples of material used for the light emitting layer of the organic EL device of the present invention can be various metal complexes, anthracene derivatives, bis(styryl)benzene derivatives, pyrene derivatives, oxazole derivatives, and polyparaphenylene vinylene derivatives, in addition to quinolinol derivative metal complexes such as Alq₃. Further, the light emitting layer may be made of a host material and a dopant material. Examples of the host material can be preferably anthracene derivatives. Other examples of the host material can be heterocyclic compounds having indole ring as a part of a condensed ring, heterocyclic compounds having carbazole ring as a part of a condensed ring, carbazole derivatives, thiazole derivatives, benzimidazole derivatives, and polydialkyl fluorene derivatives, in addition to the above light-emitting materials. Examples of the dopant material can be preferably pyrene derivatives, amine derivatives having fluorene ring as a part of a condensed ring. Other examples of the dopant material can be quinacridone, coumarin, rubrene, perylene, derivatives thereof, benzopyran derivatives, indenophenanthrene derivatives, rhodamine derivatives, and aminostyryl derivatives. These may be individually deposited for film forming, may be used as a single layer deposited mixed with other materials, or may be formed as a laminate of individually deposited layers, a laminate of mixedly deposited layers, or a laminate of the individually deposited layer and the mixedly deposited layer.

Further, the light-emitting material may be a phosphorescent material. Phosphorescent materials as metal complexes of metals such as iridium and platinum may be used. Examples of the phosphorescent materials include green phosphorescent materials such as Ir(ppy)₃, blue phosphorescent materials such as FIrpic and FIr6, and red phosphorescent materials such as Btp₂Ir(acac). Here, carbazole derivatives such as 4,4'-di(N-carbazolyl)biphenyl (CBP), TCTA, and mCP may be used as the hole injecting and transporting host material. Compounds such as p-bis(triphenylsilyl)benzene (UGH2) and 2,2',2"-(1,3,5-phenylene)-tris(1-phenyl-1H-benzimidazole) (TPBI) may be used as the electron transporting host material. In this way, a high-performance organic EL device can be produced.

In order to avoid concentration quenching, the doping of the host material with the phosphorescent light-emitting material should preferably be made by coevaporation in a range of 1 to 30 weight percent with respect to the whole light emitting layer.

Further, examples of the light-emitting material may be delayed fluorescent-emitting material such as a CDCB derivative of PIC-TRZ, CC2TA, PXZ-TRZ, 4CzIPN or the like (refer to Non-Patent Document 3, for example).

These materials may be formed into a thin-film by using a vapor deposition method or other known methods such as a spin coating method and an inkjet method.

The hole blocking layer of the organic EL device of the present invention may be formed by using hole blocking compounds such as various rare earth complexes, triazole derivatives, triazine derivatives, and oxadiazole derivatives, in addition to phenanthroline derivatives such as bathocuproin (BCP), and the metal complexes of quinolinol derivatives such as aluminum(III) bis(2-methyl-8-quinolinate)-4-phenylphenolate (BAlq). These materials may also serve as the material of the electron transport layer. These may be individually deposited for film forming, may be used as a single layer deposited mixed with other materials, or may be formed as a laminate of individually deposited layers, a laminate of mixedly deposited layers, or a laminate of the individually deposited layer and the mixedly deposited layer. These materials may be formed into a thin-film by using a vapor deposition method or other known methods such as a spin coating method and an inkjet method.

Material used for the electron transport layer of the organic EL device of the present invention is the compounds of the general formula (2c) having an anthracene ring structure. These may be individually deposited for film forming, may be used as a single layer deposited mixed with other materials, or may be formed as a laminate of individually deposited layers, a laminate of mixedly deposited layers, or a laminate of the individually deposited layer and the mixedly deposited layer. These materials may be formed into a thin-film by a vapor deposition method or other known methods such as a spin coating method and an inkjet method.

In the case where the electron transport layer of the organic EL device of the present invention has a laminate structure of two or more layers, examples of material used for the electron transport layer of second and succeeding layers can be the compounds of the general formula (2c) having an anthracene ring structure. Other examples of material can be metal complexes of quinolinol derivatives such as Alq₃ and BAlq, various metal complexes, triazole derivatives, triazine derivatives, oxadiazole derivatives, pyridine derivatives, pyrimidine derivatives, benzimidazole derivatives, thiadiazole derivatives, anthracene derivatives, carbodiimide derivatives, quinoxaline derivatives, pyridoindole derivatives, phenanthroline derivatives, and silole derivatives. These may be individually deposited for film forming, may be used as a single layer deposited mixed with other materials, or may be formed as a laminate of individually deposited layers, a laminate of mixedly deposited layers, or a laminate of the individually deposited layer and the mixedly deposited layer. These materials may be formed into a thin-film by using a vapor deposition method or other known methods such as a spin coating method and an inkjet method.

Examples of material used for the electron injection layer of the organic EL device of the present invention can be alkali metal salts such as lithium fluoride and cesium fluoride; alkaline earth metal salts such as magnesium fluoride; and metal oxides such as aluminum oxide. However, the electron injection layer may be omitted in the preferred selection of the electron transport layer and the cathode.

The cathode of the organic EL device of the present invention may be made of an electrode material with a low work function such as aluminum, or an alloy of an electrode material with an even lower work function such as a magnesium-silver alloy, a magnesium-indium alloy, or an aluminum-magnesium alloy.

The following describes an embodiment of the present invention in more detail based on Examples. The present invention, however, is not restricted to the following Examples.

### Example 1

### <Synthesis of 4,4"-bis((biphenyl-4-yl)-phenylamino}-1,1':4',1"-terphenyl (Compound 1-1)>

(Biphenyl-4-yl)-phenylamine (39.5 g), 4,4"-diiodo-1,1':4',1"-terphenyl (32.4 g), a copper powder (0.42 g), potassium carbonate (27.8 g), 3,5-di-tert-butylsalicylic acid (1.69 g), sodium bisulfite (2.09 g), dodecylbenzene (32 ml), and toluene (50 ml) were added into a reaction vessel and heated up to 210°C while removing the toluene by distillation. After the obtained product was stirred for 30 hours, the product was cooled, and toluene (50 ml) and methanol (100 ml) were added. A precipitated solid was collected by filtration and washed with a methanol/water (5/1, v/v) mixed solution (500 ml). The solid was heated after adding 1,2-dichlorobenzene (350 ml), and insoluble matter was removed by filtration. After the filtrate was left to cool, methanol (400 ml) was added, and a precipitated crude product was collected by filtration. The crude product was washed under reflux with methanol (500 ml) to obtain a gray powder of 4,4"-bis(biphenyl-4-yl)-phenylaminol-1,1':4',1"-terphenyl (Compound 1-1; 45.8 g; yield 91%).

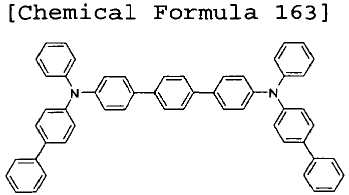

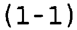

The structure of the obtained gray powder was identified by NMR.
¹H-NMR (CDCl₃) detected 40 hydrogen signals, as follows.
δ (ppm) = 7.68-7.63 (4H), 7.62-7.48 (12H), 7.45 (4H), 7.38-7.10 (20H).

### Example 2

### <Synthesis of 4,4"-bis{(biphenyl-4-yl)-4-tolylamino}-1,1':4',1"-terphenyl (Compound 1-10)>

(Biphenyl-4-yl)-4-tolylamine (16.7 g), 4,4"-diiodo-1,1':4',1"-terphenyl (12.9 g), a copper powder (0.17 g), potassium carbonate (11.2 g), 3,5-di-tert-butylsalicylic acid (0.71 g), sodium bisulfite (0.89 g), dodecylbenzene (20 ml), and toluene (20 ml) were added into a reaction vessel and heated up to 210°C while removing the toluene by distillation. The obtained product was stirred for 28 hours, and after the product was cooled, toluene (150 ml) was added, and insoluble matter was removed by filtration. Methanol (100 ml) was added, and a precipitated crude product was collected by filtration. Recrystallization of the crude product using a toluene/methanol mixed solvent was repeated three times to obtain a yellowish white powder of 4,4"-bis((biphenyl-4-yl)-4-tolylaminol-1,1':4',1"-terphenyl (Compound 1-10; 12.3 g; yield 61%).

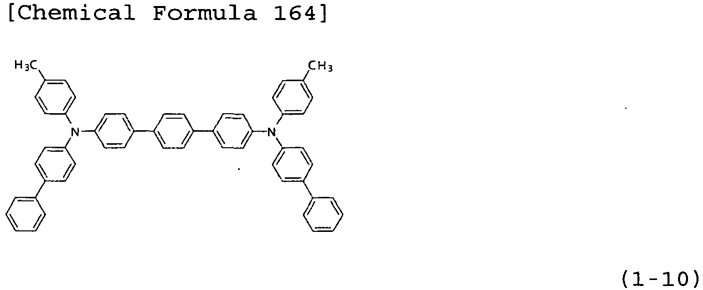

The structure of the obtained yellowish white powder was identified by NMR.
¹H-NMR (CDCl₃) detected 44 hydrogen signals, as follows.
δ (ppm) = 7.68-7.62 (4H), 7.61-7.41 (16H), 7.38-7.08 (18H), 2.38 (6H).

### Example 3

### <Synthesis of 4,4"-bis{(biphenyl-4-yl)-(phenyl-d₅)amino}-1,1':4',1"-terphenyl (Compound 1-14)>

(Biphenyl-4-yl)-(phenyl-d₅) amine (25.3 g), 4,4"-diiodo-1,1':4',1"-terphenyl (20.3 g), a copper powder (0.30 g), potassium carbonate (17.5 g), 3,5-di-tert-butylsalicylic acid (1.05 g), sodium bisulfite (1.31 g), dodecylbenzene (20 ml), and toluene (30 ml) were added into a reaction vessel and heated up to 210°C while removing the toluene by distillation. After the obtained product was stirred for 23 hours, the product was cooled, and toluene (30 ml) and methanol (60 ml) were added. A precipitated solid was collected by filtration and washed with a methanol/water (1/5, v/v) mixed solution (180 ml) followed by washing with methanol (90 ml). An obtained gray powder was heated after adding 1,2-dichlorobenzene (210 ml), and insoluble matter was removed by filtration. After the filtrate was left to cool, methanol (210 ml) was added, and a precipitated crude product was collected by filtration. The crude product was washed under reflux with methanol (210 ml) to obtain a gray powder of 4,4"-bis{(biphenyl-4-yl)-(phenyl-d₅)amino}-1,1':4',1"-terphenyl (Compound 1-14; 29.3 g; yield 96%).

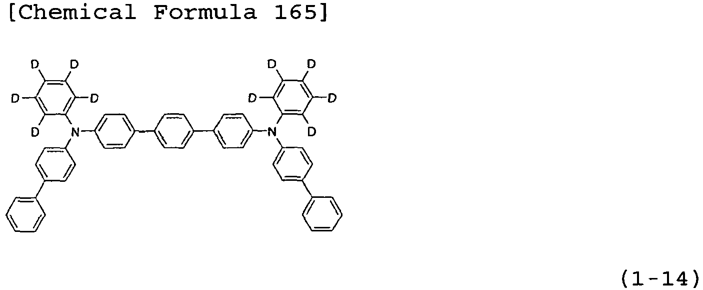

The structure of the obtained gray powder was identified by NMR.
¹H-NMR (THF-d₈) detected 30 hydrogen signals, as follows.
δ (ppm) =7.69 (4H), 7.65-7.52 (12H), 7.39 (4H), 7.28 (2H), 7.20-7.14 (8H).

### Example 4

### <Synthesis of 4,4"-bis{(naphthalen-1-yl)-phenylamino}-1,1':4',1"-terphenyl (Compound 1-2)>

(Naphthalen-1-yl)-phenylamine (40.0 g), 4,4"-diiodo-1,1':4',1"-terphenyl (43.7 g), a copper powder (0.53 g), potassium carbonate (34.4 g), 3,5-di-tert-butylsalicylic acid (2.08 g), sodium bisulfite (2.60 g), dodecylbenzene (40 ml), and xylene (40 ml) were added into a reaction vessel and heated up to 210°C while removing the xylene by distillation. After the obtained product was stirred for 35 hours, the product was cooled. Toluene (100 ml) was added, and a precipitated solid was collected by filtration. 1,2-dichlorobenzene (210 ml) was added to the obtained solid, and the solid was dissolved under heat, and after silica gel (30 g) was added, insoluble matter was removed by filtration. After the filtrate was left to cool, a precipitated crude product was collected by filtration. The crude product was washed under reflux with methanol to obtain a pale yellow powder of 4,4"-bis{(naphthalen-1-yl)-phenylamino}-1,1':4',1"-terphenyl (Compound 1-2; 21.9 g; yield 40%).

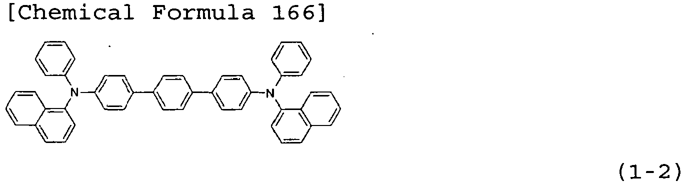

The structure of the obtained pale yellow powder was identified by NMR.
¹H-NMR (THF-d₈) detected 36 hydrogen signals, as follows.
δ (ppm) = 7.98-7.88 (4H), 7.80 (2H), 7.60 (4H), 7.52-7.40 (8H), 7.36 (4H), 7.18 (4H), 7.08-7.01 (8H), 6.93(2H).

### Example 5

### <Synthesis of 4,4"-bis{(naphthalen-2-yl)-phenylamino}-1,1':4',1"-terphenyl (Compound 1-6)>

(Naphthalen-2-yl)-phenylamine (50.0 g), 4,4"-diiodo-1,1':4',1"-terphenyl (50.0 g), tert-butoxy sodium (23.9 g), and xylene (500 ml) were added into a reaction vessel and aerated with nitrogen gas for 1 hour under ultrasonic irradiation. Palladium acetate (0.47 g) and a toluene solution (2.96 ml) containing 50% (w/v) tri-tert-butylphosphine were added, and the mixture was heated up to 120°C and stirred for 15 hours. After the mixture was left to cool, the mixture was concentrated under reduced pressure, and methanol (300 ml) was added. A precipitated solid was collected by filtration and dissolved under heat after adding 1,2-dichlorobenzene (300 ml). After silica gel (140 g) was added, insoluble matter was removed by filtration. The filtrate was concentrated under reduced pressure, and after the product was purified by recrystallization with 1,2-dichlorobenzene (250 ml), the purified product was washed under reflux with methanol to obtain a white powder of 4,4"-bis{(naphthalen-2-yl)-phenylamino}-1,1':4',1"-terphenyl (Compound 1-6; 51.0 g; yield 74%).

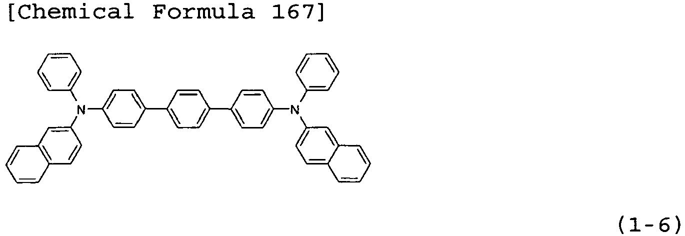

The structure of the obtained white powder was identified by NMR.
¹H-NMR (THF-d₈) detected 36 hydrogen signals, as follows.
δ (ppm) = 7.77 (4H), 7.70 (4H), 7.64-7.58 (6H), 7.48 (2H), 7.40-7.21 (10H), 7.21-7.12 (8H), 7.04 (2H).

### Example 6

### <Synthesis of 4,4"-bis [{ (biphenyl- 2', 3', 4', 5', 6 '-d₅)-4-yl}-phenylamino]-1,1':4',1"-terphenyl (Compound 1-21)>

{(Biphenyl-2',3',4',5',6'-d₅)-4-yl}-phenylamine (24.8 g), 4,4"-diiodo-1,1':4',1"-terphenyl (19.9 g), a copper powder (0.26 g), potassium carbonate (17.2 g), 3,5-di-tert-butylsalicylic acid (2.06 g), sodium bisulfite (1.30 g), and dodecylbenzene (20 ml) were added into a reaction vessel and heated up to 215°C. After the obtained product was stirred for 21 hours, the product was cooled, and toluene (30 ml) and methanol (60 ml) were added. A precipitated solid was collected by filtration and washed with a methanol/water (1/5, v/v) mixed solution. After adding 1,2-dichlorobenzene (300 ml) to the obtained solid, the solid was heated, and insoluble matter was removed by filtration. After the filtrate was left to cool, methanol (300 ml) was added, and a precipitate was collected by filtration to obtain a yellow powder of 4,4"-bis[{(biphenyl-2', 3', 4', 5', 6'-d₅)-4-yl}-phenylamino]-1,1':4',1"-terphenyl (Compound 1-21; 25.5 g; yield 85%).

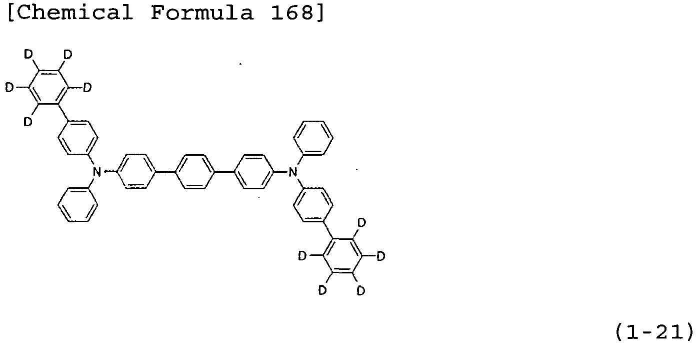

The structure of the obtained yellow powder was identified by NMR.
¹H-NMR (THF-d₈) detected 30 hydrogen signals, as follows.
δ (ppm) = 7.69 (4H), 7.65-7.52 (8H), 7.28 (4H), 7.20-7.12 (10H), 7.03 (4H).

### Example 7

### <Synthesis of 4,4"-bis{(biphenyl-3-yl)-(biphenyl-4-yl)aminol-1,1':4',1"-terphenyl (Compound 1-22)>

(Biphenyl-3-yl)-(biphenyl-4-yl)amine (16.1 g), 4,4"-diiodo-1,1':4',1"-terphenyl (11.0 g), a copper powder (0.29 g), potassium carbonate (9.46 g), 3,5-di-tert-butylsalicylic acid (1.14 g), sodium bisulfite (0.71 g), and dodecylbenzene (22 ml) were added into a reaction vessel and heated up to 220°C. After the obtained product was stirred for 34 hours, the product was cooled, and toluene and heptane were added. A precipitated solid was collected by filtration and dissolved under heat after adding 1,2-dichlorobenzene (200 ml). After silica gel (50 g) was added, insoluble matter was removed by filtration. After the filtrate was concentrated under reduced pressure, toluene and acetone were added. A precipitated solid was collected by filtration, and the precipitated solid was crystallized with 1,2-dichloromethane followed by crystallization with acetone, and further crystallized with 1,2-dichloromethane followed by crystallization with methanol to obtain a pale yellow powder of 4,4"-bis{(biphenyl-3-yl)-(biphenyl-4-yl)amino)-1,1':4',1"-terphenyl (Compound 1-22; 25.5 g; yield 77%).

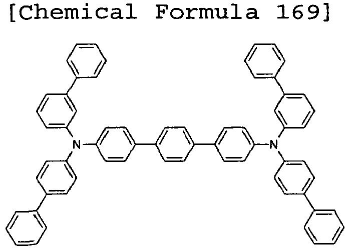

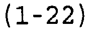

The structure of the obtained pale yellow powder was identified by NMR.
¹H-NMR (THF-d₈) detected 48 hydrogen signals, as follows.
δ (ppm) = 7.71 (4H), 7.67-7.50 (16H), 7.47 (4H), 7.43-7.20 (20H), 7.12 (4H).

### Example 8

### <Synthesis of 4,4"-bis(phenanthren-9-yl)-phenylamino}-1,1':4',1"-terphenyl (Compound 1-3)>

(Phenanthren-9-yl)-phenylamine (16.9 g), 4,4"-diiodo-1,1':4',1"-terphenyl (12.6 g), a copper powder (0.16 g), potassium carbonate (10.9 g), 3,5-di-tert-butylsalicylic acid (0.65 g), sodium bisulfite (0.83 g), and dodecylbenzene (13 ml) were added into a reaction vessel and heated up to 210°C. After the obtained product was stirred for 23 hours, the product was cooled, and toluene (26 ml) and methanol (26 ml) were added. A precipitated solid was collected by filtration and washed with a methanol/water (1/5, v/v) mixed solution (120 ml). The precipitated solid was crystallized with 1,2-dichlorobenzene followed by crystallization with methanol to obtain a white powder of 4,4"-bis{(phenanthren-9-yl)-phenylamino}-1,1':4',1"-terphenyl (Compound 1-3; 9.38 g; yield 47%) .

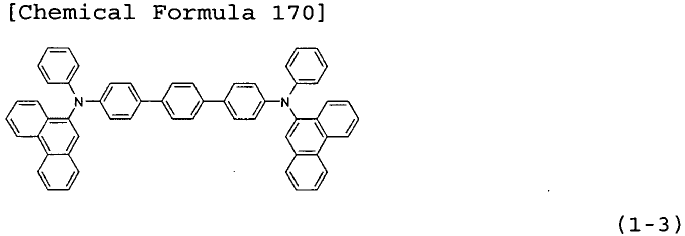

The structure of the obtained yellow powder was identified by NMR.
¹H-NMR (THF-d₈) detected 40 hydrogen signals, as follows.
δ (ppm) = 8.88-8.73 (4H), 8.09 (2H), 7.71 (2H), 7.68-7.41 (18H), 7.21-7.10 (12H), 6.92 (2H).

### Example 9

### <Synthesis of 4,4"-bis{(biphenyl-3-yl)-phenylamino}-1,1':4',1"-terphenyl (Compound 1-5)>

(Biphenyl-3-yl)-phenylamine (12.7 g), 4,4"-diiodo-1,1':4',1"-terphenyl (11.3 g), a copper powder (0.30 g), potassium carbonate (9.72 g), 3,5-di-tert-butylsalicylic acid (1.17 g), sodium bisulfite (0.73 g), and dodecylbenzene (23 ml) were added into a reaction vessel and heated up to 220°C. After the obtained product was stirred for 21 hours, the product was cooled, and after 1,2-dichlorobenzene (250 ml) and silica (30 g) were added, insoluble matter was removed by filtration. After the filtrate was concentrated under reduced pressure, heptane was added. A precipitated solid was collected by filtration, and the precipitated solid was crystallized with a 1,2-dichlorobenzene/heptane mixed solvent and further crystallized with a 1,2-dichlorobenzene/methanol mixed solvent to obtain a pale brown powder of 4,4"-bis{(biphenyl-3-yl)-phenylamino}-1,1':4',1"-terphenyl (Compound 1-5; 10.8 g; yield 64%).

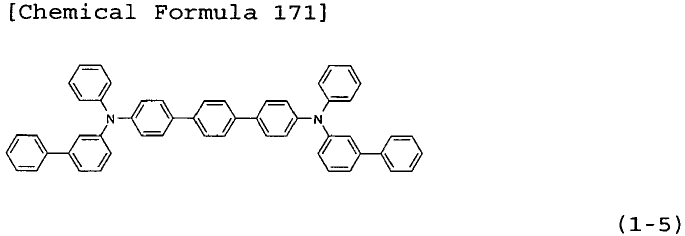

The structure of the obtained pale brown powder was identified by NMR.
¹H-NMR (THF-d₈) detected 40 hydrogen signals, as follows.
δ (ppm) = 7.69 (4H), 7.60 (4H), 7.52 (4H), 7.42-7.21 (16H), 7.20-7.13 (8H), 7.10-7.00 (4H).

### Example 10

### <Synthesis of 4,4"-bis{(triphenylen-2-yl)-phenylamino}-1,1':4',1"-terphenyl (Compound 1-23)>

(Triphenylen-2-yl)-phenylamine (11.9 g), 4,4"-diiodo-1,1':4',1"-terphenyl (8.55 g), tert-butoxy sodium (4.09 g), and xylene (86 ml) were added into a reaction vessel and aerated with nitrogen gas for 40 minutes under ultrasonic irradiation. Palladium acetate (0.08 g) and a toluene solution (0.55 ml) containing 50% (w/v) tri-tert-butylphosphine were added, and the mixture was heated up to 100°C. After the mixture was stirred for 7 hours, the mixture was cooled. Methanol (80 ml) was added, and a precipitated solid was collected by filtration. 1,2-dichlorobenzene (300 ml) was added to the obtained solid, and the solid was heated, and after silica gel (45 g) was added, insoluble matter was removed by filtration. The filtrate was concentrated under reduced pressure, and after purified by recrystallization with 1,2-dichlorobenzene, the purified product was washed under reflux with methanol to obtain a pale yellowish green powder of 4,4"'-bis{(triphenylen-2-yl)-phenylamino}-1,1':4',1"-terphenyl (Compound 1-23; 11.4 g; yield 74%).

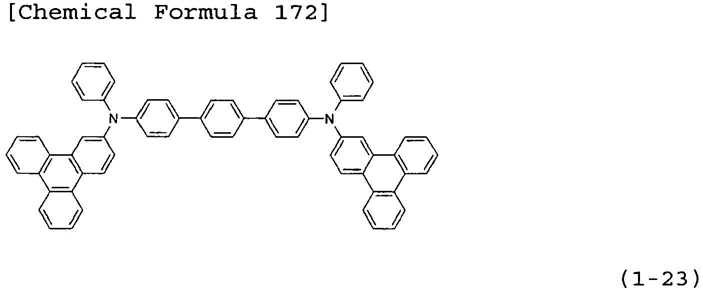

The structure of the obtained pale yellowish green powder was identified by NMR.
¹H-NMR (THF-d₈) detected 44 hydrogen signals, as follows.
δ (ppm) = 8.72-8.62 (8H), 8.45 (2H), 8.36 (2H), 7.75 (4H), 7.70-7.21 (26H), 7.09 (2H).

### Example 11

### <Synthesis of 4,4"-bis{di(naphthalen-2-yl)amino}-1,1':4',1"-terphenyl (Compound 1-24)>

Di(naphthalen-2-yl)amine (12.2 g), 4,4"-diiodo-1,1':4',1"-terphenyl (9.49 g), a copper powder (0.14 g), potassium carbonate (8.2 g), 3,5-di-tert-butylsalicylic acid (0.51 g), sodium bisulfite (0.69 g), dodecylbenzene (15 ml), and toluene (20 ml) were added into a reaction vessel and heated up to 210°C while removing the toluene by distillation. After the obtained product was stirred for 28 hours, the product was cooled, and 1,2-dichlorobenzene (20 ml) and methanol (20 ml) were added. A precipitated solid was collected by filtration and washed with a methanol/water (1/4, v/v) mixed solution (200 ml). Then, the solid was dissolved under heat after adding 1,2-dichlorobenzene (100 ml), and after silica gel was added, insoluble matter was removed by filtration. After the filtrate was left to cool, methanol (250 ml) was added, and a precipitated solid was collected by filtration. The precipitated solid was crystallized with a 1,2-dichlorobenzene/methanol mixed solvent followed by washing under reflux with methanol to obtain a yellowish white powder of 4,4"-bis{di(naphthalen-2-yl)amino}-1,1':4',1"-terphenyl (Compound 1-24; 10.5 g; yield 70%).

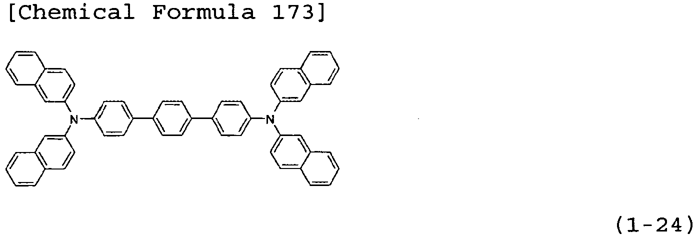

The structure of the obtained yellowish white powder was identified by NMR.
¹H-NMR (THF-d₈) detected 40 hydrogen signals, as follows.
δ (ppm) = 7.82-7.75 (6H), 7.72 (4H), 7.68-7.60 (8H), 7.56 (4H), 7.40-7.30 (14H), 7.24 (4H).

### Example 12

### <Synthesis of 4,4"-bis[{4-(naphthalen-2-yl)phenyl)}-phenylamino]-1,1':4',1"-terphenyl (Compound 1-25)>

{4-(Naphthalen-2-yl)phenyl}-phenylamine (16.6 g), 4,4"-diiodo-1,1':4',1"-terphenyl (11.8 g), a copper powder (0.18 g), potassium carbonate (10.5 g), 3,5-di-tert-butylsalicylic acid (0.61 g), sodium bisulfite (0.83 g), dodecylbenzene (15 ml), and toluene (20 ml) were added into a reaction vessel and heated up to 210°C while removing the toluene by distillation. After the obtained product was stirred for 19 hours, the product was cooled, and toluene (20 ml) and methanol (20 ml) were added. A precipitated solid was collected by filtration, washed with a methanol/water (1/4, v/v) mixed solution (180 ml), and further washed with methanol (100 ml). An obtained brownish yellow powder was heated after adding 1,2-dichlorobenzene (175 ml), and insoluble matter was removed by filtration. After the filtrate was left to cool, methanol (200 ml) was added, and a precipitated solid was collected by filtration. The precipitated solid was crystallized with a 1,2-dichlorobenzene/methanol mixed solvent followed by washing under reflux with methanol to obtain a brownish white powder of 4,4"-bis[(4-(naphthalen-2-yl)phenyl}-phenylamino]-1,1':4',1"-terphenyl (Compound 1-25; 11.9 g; yield 53%).

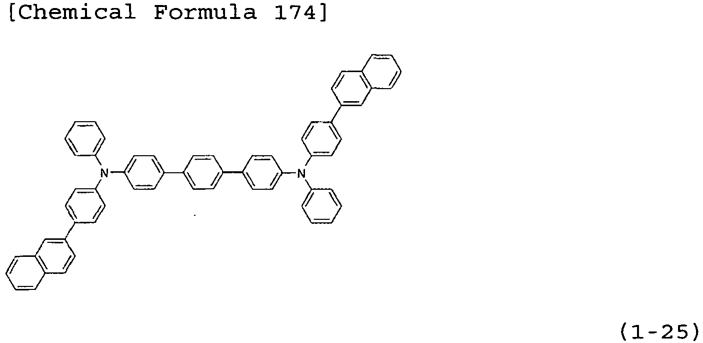

The structure of the obtained brownish white powder was identified by NMR.
¹H-NMR (THF-d₈) detected 44 hydrogen signals, as follows.
δ (ppm) = 8.10 (2H), 7.93-7.78 (8H), 7.76-7.70 (8H), 7.62 (4H), 7.44 (4H), 7.30 (4H), 7.25-7.16 (12H), 7.05 (2H) .

### Example 13

### <Synthesis of 4-1{biphenyl-4-yl)-phenylamino}-4"-[{4-(1-phenyl-indol-4-yl)phenyl}-phenylaminol-1,1':4¹,1"-terphenyl (Compound 1-26)>

(4'-Bromo-1,1'-biphenyl-4-yl)-{4-(1-phenyl-indol-4-yl)phenyl}-phenylamine (7.25 g), {4-(4,4,5,5-tetramethyl-1,3,2-dioxabororan-2-yl)phenyl}-(1,1'-biphenyl-4-yl)-phenylamine (5.76 g), a 2 M potassium carbonate aqueous solution (12.3 ml), toluene (80 ml), and ethanol (20 ml) were added into a reaction vessel and aerated with nitrogen gas for 40 minutes under ultrasonic irradiation. After adding tetrakistriphenylphosphinepalladium (0.43 g), the mixture was heated and refluxed for 7 hours while being stirred. After the mixture was left to cool, water (50 ml) and toluene (100 ml) were added, and insoluble matter was removed by filtration. An organic layer was collected by liquid separation, then dried over anhydrous magnesium sulfate and concentrated under reduced pressure to obtain a crude product. After the crude product was purified by column chromatography (support: silica gel, eluent: toluene/heptane), the purified product was crystallized with THF followed by crystallization with methanol to obtain a pale yellow powder of 4-{(biphenyl-4-yl)-phenylaminol-4"-[{4-(1-phenyl-indol-4-yl)phenyl)}-phenylamino]-1,1':4',1"-terphenyl (Compound 1-26; 6.80 g; yield 67%).

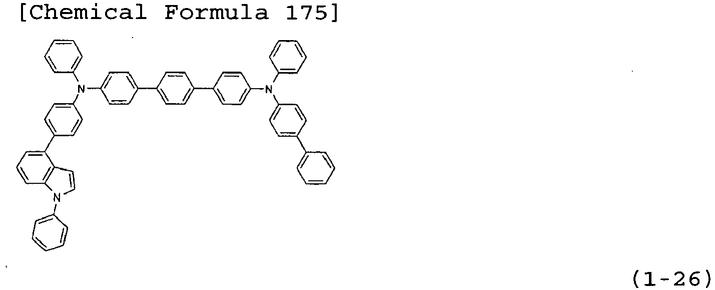

The structure of the obtained pale yellow powder was identified by NMR.
¹H-NMR (THF-d₈) detected 45 hydrogen signals, as follows.
δ (ppm) = 7.70 (4H), 7.68-7.50 (16H), 7.42-7.11 (23H), 7.05 (1H), 6.88 (1H).

### Example 14

### <Synthesis of 4,4"-bis{(biphenyl-4-yl)-phenylamino}-1,1':3',1"-terphenyl (Compound 1-27)>

3-Bromoiodobenzene (8.83 g), (biphenyl-4-yl)-{4-(4,4,5,5-tetramethyl-[1,3,2]dioxaborolan-2-yl)phenyl}-phenylamine (30.5 g), potassium carbonate (13.0 g), water (30 ml), toluene (300 ml), and ethanol (75 ml) were added into a nitrogen-substituted reaction vessel and aerated with nitrogen gas under ultrasonic irradiation for 30 minutes. The mixture was heated after adding tetrakis(triphenylphosphine)palladium (1.1 g), and stirred at 80°C for 16 hours. The mixture was cooled to a room temperature, and methanol (300 ml) was added. A precipitated solid was collected by filtration, and the solid was dissolved under heat after adding 1,2-dichlorobenzene (270 ml). Silica gel (16 g) was added, and the mixture was stirred for 30 minutes. After insoluble matter was removed by filtration, a crude product precipitated by adding methanol (300 ml) was collected by filtration. The crude product was washed under reflux with methanol (200 ml) to obtain a white powder of 4,4"-bis{(biphenyl-4-yl)-phenylamino}-1,1':3',1"-terphenyl (Compound 1-27; 14.3 g; yield 71%).

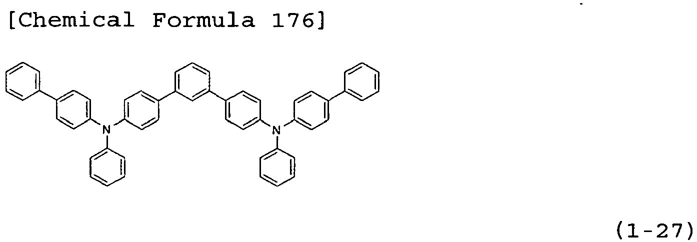

The structure of the obtained white powder was identified by NMR.
¹H-NMR (CDCl₃) detected 40 hydrogen signals, as follows.
δ (ppm) = 7.87 (1H), 7.64-7.50 (12H), 7.48-7.32 (6H), 7.31-6.98 (21H).

### Example 15

### <Synthesis of 4,4"-bis{(biphenyl-4-yl)-(phenyl-d₅)amino}-1,1':3',1"-terphenyl (Compound 1-28)>

1,3-dibromobenzene (6.51 g), (biphenyl-4-yl)-{4-(4,4,5,5-tetramethyl-[1,3,2]dioxaborolan-2-yl)phenyl}-(phenyl-d₅)amine (26.9 g), potassium carbonate (11.4 g), water (50 ml), toluene (200 ml), and ethanol (50 ml) were added into a nitrogen-substituted reaction vessel and aerated with nitrogen gas under ultrasonic irradiation for 30 minutes. The mixture was heated after adding tetrakis(triphenylphosphine)palladium (0.95 g), and stirred at 70°C for 12 hours. The mixture was cooled to a room temperature, and methanol (200 ml) was added. A precipitated solid was collected by filtration, and the solid was dissolved under heat after adding 1,2-dichlorobenzene (400 ml). Silica gel (20 g) was added, and the mixture was stirred for 30 minutes. After insoluble matter was removed by filtration, a precipitate formed by adding methanol (500 ml) was collected by filtration. The precipitate was dissolved by adding 1,2-dichlorobenzene (100 ml), and a crude product precipitated by adding toluene (100 ml) and methanol (100 ml) was collected by filtration. The crude product was washed under reflux with methanol (250 ml) to obtain a white powder of 4,4"-bis{(biphenyl-4-yl)-(phenyl-d₅)amino}-1,1':3',1"-terphenyl (Compound 1-28; 18.3 g; yield 91%).

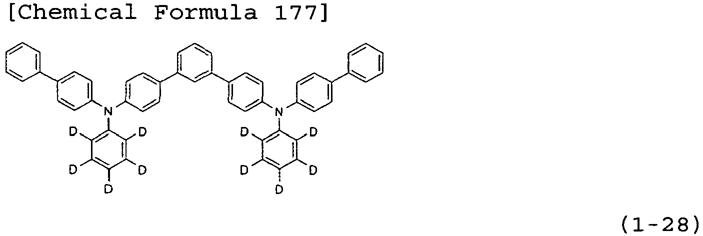

The structure of the obtained white powder was identified by NMR.
¹H-NMR (CDCl₃) detected 30 hydrogen signals, as follows.
δ (ppm) = 7.87 (1H), 7.64-7.32 (18H), 7.31-6.98 (11H).

### Example 16

### <Synthesis of 4,4"-bis{(naphthalen-1-yl)-phenylamino}-1,1':3',1"-terphenyl (Compound 1-29)>

The reaction was carried out under the same conditions as those of Example 15, except that (biphenyl-4-yl)-{4-(4,4,5,5-tetramethyl-[1,3,2]dioxaborolan-2-yl)phenyl}-(phenyl-d₅)amine was replaced with (naphthalen-1-yl)-{4-(4,4,5,5-tetramethyl-[1,3,2] dioxaborolan-2-yl)phenyl}-phenylamine. As a result, a white powder of 4,4"-bis{(naphthalen-1-yl)-phenylamino}-1,1':3',1"-terphenyl (Compound 1-29; 8.8 g; yield 59%) was obtained.

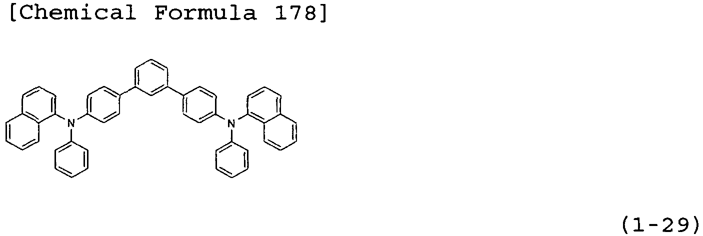

The structure of the obtained white powder was identified by NMR.
¹H-NMR (CDCl₃) detected 36 hydrogen signals, as follows.
δ (ppm) = 7.99 (2H), 7.92 (2H), 7.81 (2H), 7.72 (1H), 7.57-6.92 (29H).

### Example 17

### <Synthesis of 4,4"-bis[{4-(dibenzofuran-4-yl)phenyl}-phenylamino}-1,1':3',1"-terphenyl (Compound 1-32)>

The reaction was carried out under the same conditions as those of Example 15, except that (biphenyl-4-yl)-{4-(4,4,5,5-tetramethyl-[1,3,2]dioxaborolan-2-yl)phenyl}-(phenyl-d₅)amine was replaced with {4-(dibenzofuran-4-yl)phenyl}-{4-(4,4,5,5-tetramethyl-[1,3,2]dioxaborolan-2-yl)phenyl}-phenylamine. As a result, a white powder of 4,4"-bis[{4-(dibenzofuran-4-yl)phenyl}-phenylamino]-1,1':3',1"-terphenyl (Compound 1-32; 6.8 g; yield 86%) was obtained.

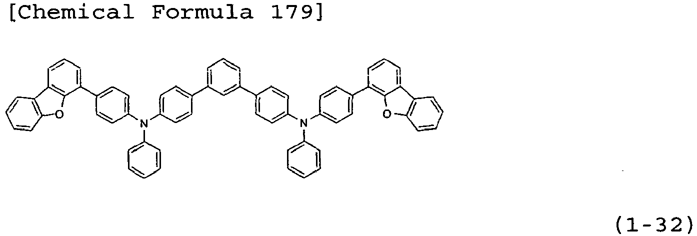

The structure of the obtained white powder was identified by NMR.
¹H-NMR (CDCl₃) detected 44 hydrogen signals, as follows.
δ (ppm) = 8.01 (2H), 7.97-7.82 (8H), 7.67-7.24 (34H).

### Example 18

### <Synthesis of 2,4"-bis{(biphenyl-4-yl)-phenylamino}-1,1':4',1"-terphenyl (Compound 1-50)>

4-Bromo-4'-{(biphenyl-4-yl)-phenylamino}-biphenyl (16.8 g), (biphenyl-4-yl)-{2-(4,4,5,5-tetramethyl-[1,3,2]dioxaborolan-2-yl)phenyl}-phenylamine (19.0 g), potassium carbonate (7.4 g), water (26 ml), toluene (200 ml), and ethanol (50 ml) were added into a nitrogen-substituted reaction vessel and aerated with nitrogen gas under ultrasonic irradiation for 30 minutes. After adding tetrakis(triphenylphosphine)palladium (0.87 g), the mixture was heated and refluxed for 20 hours while being stirred. After the mixture was cooled to a room temperature, an organic layer was collected by liquid separation, then dried over anhydrous magnesium sulfate and concentrated to obtain a crude product. After the crude product was purified by column chromatography (support: silica gel, eluent: heptane/toluene), the purified product was crystallized with an ethyl acetate/methanol mixed solvent to obtain a white powder of 2,4"-bis{(biphenyl-4-yl)-phenylamino}-1,1':4',1"-terphenyl (Compound 1-50; 20.8 g; yield 82%).

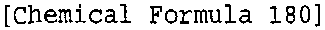

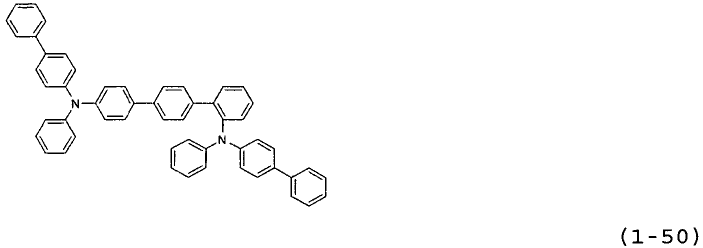

The structure of the obtained white powder was identified by NMR.
¹H-NMR (CDCl₃) detected 40 hydrogen signals, as follows.
δ (ppm) = 7.61 (2H), 7.56-6.83 (38H).

### Example 19

### <Synthesis of 4,4"-bis{(triphenylen-2-yl)-phenylamino}-1,1':3',1"-terphenyl (Compound 1-51)>

4,4"-Dibromo-1,1':3',1"-terphenyl (8.2 g), (triphenylen-2-yl)-phenylamine (15.4 g), tert-butoxy sodium (5.1 g), and toluene (180 ml) were added into a nitrogen-substituted reaction vessel and aerated with nitrogen gas under ultrasonic irradiation for 30 minutes. Palladium acetate (0.11 g) and a toluene solution (0.31 ml) containing 50% (w/v) tri-tert-butylphosphine were added, and the mixture was heated and refluxed for 5 hours while being stirred.

The mixture was cooled to a room temperature and subjected to an extraction procedure using 1,2-dichlorobenzene and then to purification by adsorption with a silica gel, followed by crystallization with a 1,2-dichlorobenzene/methanol mixed solvent to obtain a yellowish white powder of 4,4"-bis{(triphenylen-2-yl)-phenylamino}-1,1':3',1"-terphenyl (Compound 1-51; 11.67 g; yield 64%).

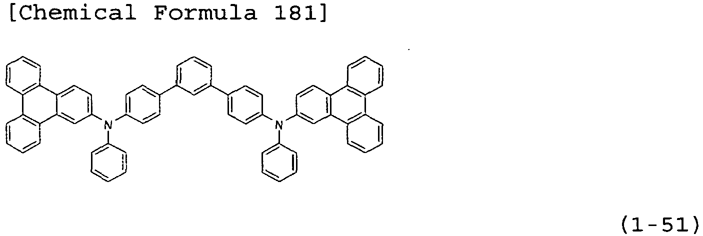

The structure of the obtained yellowish white powder was identified by NMR.
¹H-NMR (CDCl₃) detected 44 hydrogen signals, as follows.
δ (ppm) = 8.67 (4H), 8.57 (4H), 8.41 (2H), 8.36 (2H), 7.88 (1H), 7.70-7.10 (31H).

### Example 20

### <Synthesis of 4,4"-bis{(phenanthren-9-yl)-phenylamino}-1,1':3',1"-terphenyl (Compound 1-52)>

The reaction was carried out under the same conditions as those of Example 19, except that (triphenylen-2-yl)-phenylamine was replaced with (phenanthren-9-yl)-phenylamine. As a result, a yellowish white powder of 4,4"-bis{(phenanthren-9-yl)-phenylamino}-1,1':3',1"-terphenyl (Compound 1-52; 8.0 g; yield 50%) was obtained.

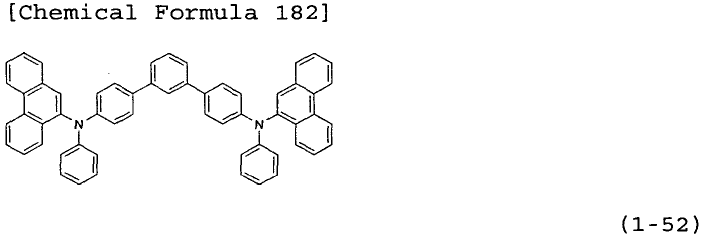

The structure of the obtained yellowish white powder was identified by NMR.
¹H-NMR (CDCl₃) detected 40 hydrogen signals, as follows.
δ (ppm) = 8.81-8.71 (4H), 8.10 (2H), 7.83-7.39 (20H), 7.29-6.97 (14H).

### Example 21

### <Synthesis of 4-{bis(biphenyl-4-yl)amino}-2"-{(biphenyl-4-yl)-phenylamino}-1,1':4',1"-terphenyl (Compound 1-53)>

2-{(biphenyl-4-yl)-phenylamino}-4"-bromo-1,1':4',1"-terphenyl (12.1 g), bis(biphenyl-4-yl)amine (8.0 g), tris(dibenzylideneacetone)palladium (0.6 g), tri-tert-butylphosphine (0.22 g), and tert-butoxy sodium (6.3 g) were added into a nitrogen-substituted reaction vessel, heated and refluxed for 3 hours while being stirred. After the mixture was cooled to a room temperature, methanol (600 ml) was added, and a precipitated crude product was collected by filtration. The crude product was dissolved in toluene, and after insoluble matter was removed by filtration, purification by crystallization with methanol was carried out. Then, recrystallization with a THF/methanol mixed solvent was carried out to obtain a white powder of 4-{bis(biphenyl-4-yl)amino}-2"-{(biphenyl-4-yl)-phenylamino}-1,1':4',1"-terphenyl (Compound 1-53; 15 g; yield 87%).

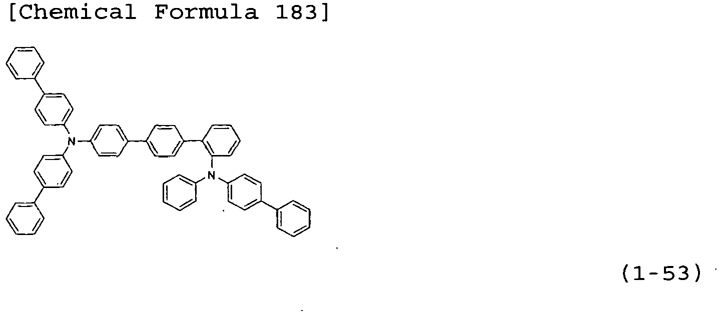

The structure of the obtained white powder was identified by NMR.
¹H-NMR (CDCl₃) detected 44 hydrogen signals, as follows.
δ (ppm) = 7.62 (4H), 7.58-6.91 (38H), 6.87 (2H).

### Example 22

### <Synthesis of 4,4"-bis{(naphthalen-1-yl)-(phenyl-d₅)amino}-1,1':3',1"-terphenyl (Compound 1-54)>

The reaction was carried out under the same conditions as those of Example 15, except that (biphenyl-4-yl)-{4 -(4,4,5,5-tetramethyl-[1,3,2]dioxaborolan-2-yl)phenyl}-(phenyl-d₅)amine was replaced with (naphthalen-1-yl)-{4-(4,4,5,5-tetramethyl-[1,3,2]dioxaborolan-2-yl)phenyl}-(phenyl-d₅)amine. As a result, a white powder of 4,4"-bis{(naphthalen-1-yl)-(phenyl-d₅)amino}-1,1':3',1"-terphenyl (Compound 1-54; 5.2 g; yield 30%) was obtained.

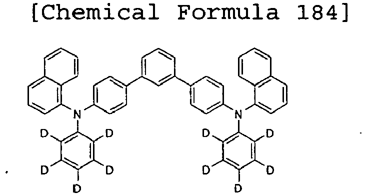

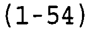

The structure of the obtained white powder was identified by NMR.
¹H-NMR (CDCl₃) detected 26 hydrogen signals, as follows.
δ (ppm) = 7.99 (2H), 7.92 (2H), 7.81 (2H), 7.72 (1H), 7.55-7.36 (15H), 7.13-7.07 (4H).

### Example 23

### <Synthesis of 2-{bis(biphenyl-4-yl)amino}-4"-{(biphenyl-4-yl)-phenylamino}-1,1':4',1"-terphenyl (Compound 1-56)>

The reaction was carried out under the same conditions as those of Example 18, except that (biphenyl-4-yl)-{2-(4,4,5,5-tetramethyl-[1,3,2]dioxaborolan-2-yl)phenyl}-phenylamine was replaced with bis(biphenyl-4-yl)-{2-(4,4,5,5-tetramethyl-[1,3,2]dioxaborolan-2-yl)phenyl}amine. As a result, a white powder of 2-{bis(biphenyl-4-yl)amino}-4"-{(biphenyl-4-yl)-phenylamino}-1,1':4',1"-terphenyl (Compound 1-56; 15.7 g; yield 94%) was obtained.

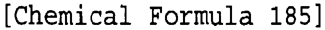

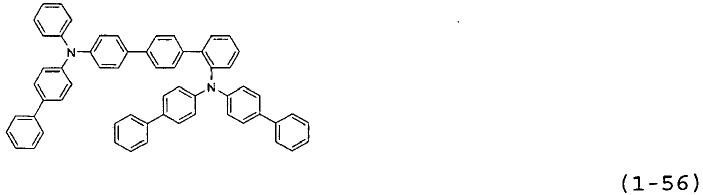

The structure of the obtained white powder was identified by NMR.
¹H-NMR (CDCl₃) detected 44 hydrogen signals, as follows.
δ (ppm) = 7.60 (2H), 7.56-6.97 (32H).

### Example 24

### <Synthesis of 2,4"-bis{bis(biphenyl-4-yl)amino}-1,1':4',1"-terphenyl (Compound 1-57)>

The reaction was carried out under the same conditions as those of Example 18, except that 4-bromo-4'-{(biphenyl-4-yl)-phenylamino}-biphenyl was replaced with 4-bromo-4'-{bis(biphenyl-4-yl)amino}-biphenyl, and (biphenyl-4-yl)-{2-(4,4,5,5-tetramethyl-[1,3,2]dioxaborolan-2-yl)phenyl}-phenylamine was replaced with 2-{bis(biphenyl-4-yl)amino}phenylboronic acid. As a result, a white powder of 2,4"-bis{bis(biphenyl-4-yl)amino}-1,1':4',1"-terphenyl (Compound 1-57; 12 g; yield 76%) was obtained.

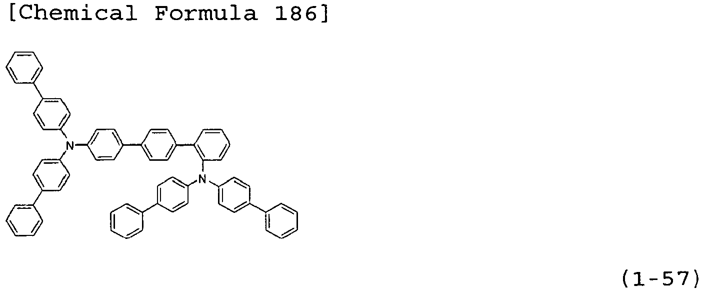

The structure of the obtained white powder was identified by NMR.
¹H-NMR (CDCl₃) detected 48 hydrogen signals, as follows.
δ (ppm) = 7.65-6.98 (48H).

### Example 25

### <Synthesis of 4,4"-bis{(biphenyl-4-yl)-(naphthalen-1-yl)amino}-1,1':3',1"-terphenyl (Compound 1-59)>

The reaction was carried out under the same conditions as those of Example 19, except that (triphenylen-2-yl)-phenylamine was replaced with (biphenyl-4-yl)-(naphthalen-1-yl)amine. As a result, a white powder of 4,4"-bis{(biphenyl-4-yl)-(naphthalen-1-yl)amino}-1,1':3',1"-terphenyl (Compound 1-59; 6.4 g; yield 36%) was obtained.

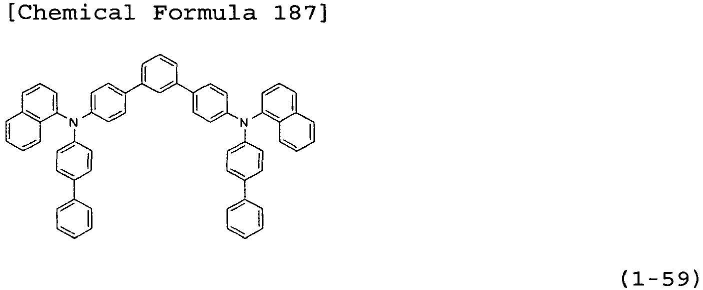

The structure of the obtained white powder was identified by NMR.
¹H-NMR (CDCl₃) detected 44 hydrogen signals, as follows.
δ (ppm) = 8.02 (2H), 7.94 (2H), 7.84 (2H), 7.76 (1H), 7.62-7.38 (27H), 7.33 (2H), 7.19-7.13 (8H).

### Example 26

### <Synthesis of 4,4"-bis{(9,9-dimethyl-9H-fluoren-2-yl)-phenylamino}-1,1':3',1"-terphenyl (Compound 1-60)>

The reaction was carried out under the same conditions as those of Example 19, except that (triphenylen-2-yl)-phenylamine was replaced with (9,9-dimethyl-9H-fluoren-2-yl)-phenylamine. As a result, a white powder of 4,4"-bis{(9,9-dimethyl-9H-fluoren-2-yl)-phenylamino}-1,1':3',1"-terphenyl (Compound 1-60; 14.6 g; yield 80%) was obtained.

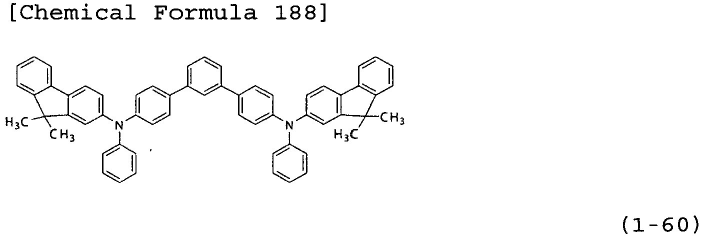

The structure of the obtained white powder was identified by NMR.
¹H-NMR (CDCl₃) detected 48 hydrogen signals, as follows.
δ (ppm) = 7.84 (1H), 7.70-7.03 (35H), 1.48 (12H).

### Example 27

### <Synthesis of 2-{bis(biphenyl-4-yl)amino}-4"-{(naphthalen-1-yl)-phenylamino}-1,1':4',1"-terphenyl (Compound 1-62)>

The reaction was carried out under the same conditions as those of Example 18, except that 4-bromo-4'-{(biphenyl-4-yl)-phenylamino}-biphenyl was replaced with 4-bromo-4'-{(naphthalen-1-yl)-phenylamino}-biphenyl, and (biphenyl-4-y1)-{2-(4,4,5,5-tetramethyl-[1,3,2]dioxaborolan-2-yl)phenyl}-phenylamine was replaced with 2-{bis(biphenyl-4-yl)amino}phenylboronic acid. As a result, a white powder of 2-{bis(biphenyl-4-yl)amino}-4"-{(naphthalen-1-yl)-phenylamino}-1,1':4',1"-terphenyl (Compound 1-62; 12.8 g; yield 75%) was obtained.

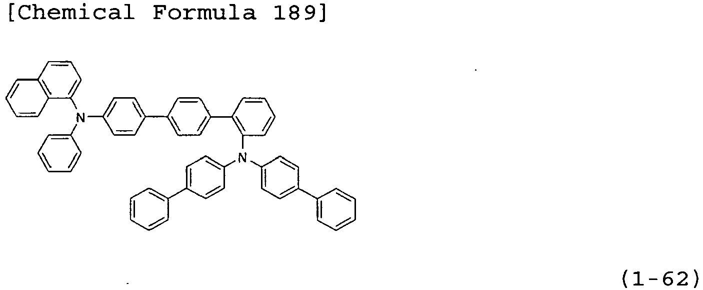

The structure of the obtained white powder was identified by NMR.
¹H-NMR (CDCl₃) detected 42 hydrogen signals, as follows.
δ (ppm) = 7.99 (2H), 7.93 (2H), 7.81 (2H), 7.57-6.96 (36H) .

### Example 28

### <Synthesis of 2-{(biphenyl-4-yl)-phenylamino}-4"-{(9,9-dimethyl-9H-fluoren-2-yl)-phenylamino}-1,1':4',1"-terphenyl (Compound 1-63)>

The reaction was carried out under the same conditions as those of Example 18, except that 4-bromo-4'-{(biphenyl-4-yl)-phenylamino}-biphenyl was replaced with 4-bromo-4'-{(9,9-dimethyl-9H-fluoren-2-yl)-phenylamino}-biphenyl. As a result, a white powder of 2-{(biphenyl-4-yl)-phenylamino}-4"-{(9,9-dimethyl-9H-fluoren-2-yl)-phenylamino}-1,1':4',1"-terphenyl (Compound 1-63; 11.7 g; yield 73%) was obtained.

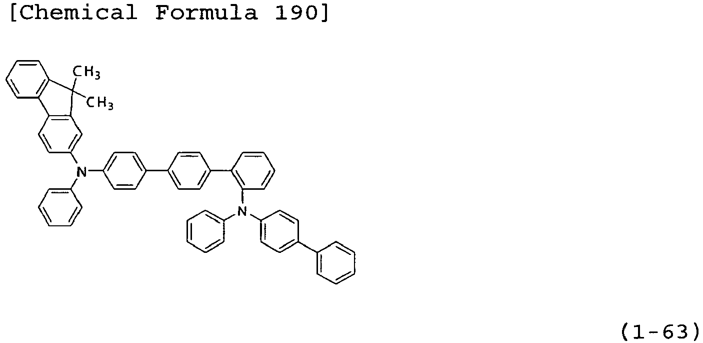

The structure of the obtained white powder was identified by NMR.
¹H-NMR (CDCl₃) detected 44 hydrogen signals, as follows.
δ (ppm) = 7.68 (1H), 7.64-6.84 (37H), 1.48 (6H).

### Example 29

### <Synthesis of 4,4"-bis{(biphenyl-4-yl)-(naphthalen-1-yl)aminol-1,1':2',1"-terphenyl (Compound 1-67)>

The reaction was carried out under the same conditions as those of Example 19, except that 4,4"-dibromo-1,1':3',1"-terphenyl was replaced with 4,4"-dibromo-1,1':2',1"-terphenyl, and (triphenylen-2-yl)-phenylamine was replaced with (biphenyl-4-yl)-(naphthalen-1-yl)amine. As a result, a white powder of 4,4"-bis{(biphenyl-4-yl)-(naphthalen-1-yl)amino}-1,1':2',1"-terphenyl (Compound 1-67; 5.0 g yield 30%) was obtained.

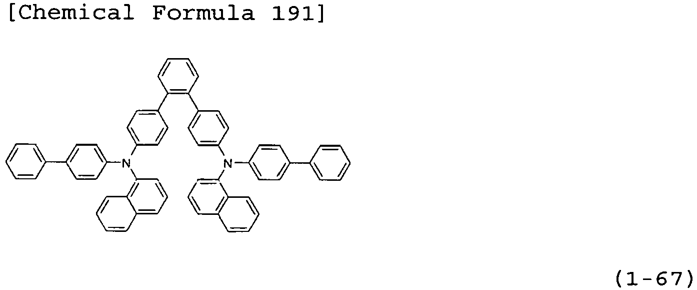

The structure of the obtained white powder was identified by NMR.
¹H-NMR (CDCl₃) detected 44 hydrogen signals, as follows.
δ (ppm) = 7.93-7.84 (4H), 7.79 (2H), 7.60-7.26 (24H), 7.25-6.92 (14H).

### Example 30

### <Synthesis of 4,4"-bis[{4-(naphthalen-1-yl)phenyl}-phenylamino]-1,1':2',1"-terphenyl (Compound 1-68)>

The reaction was carried out under the same conditions as those of Example 19, except that 4,4"-dibromo-1,1':3',1"-terphenyl was replaced with 4,4"-dibromo-1,1':2',1"-terphenyl, and (triphenylen-2-yl)-phenylamine was replaced with {4-(naphthalen-1-yl)phenyl}-phenylamine. As a result, a white powder of 4,4"-bis[{4-(naphthalen-1-yl)phenyl}-phenylamino]-1,1':2',1"-terphenyl (Compound 1-68; 7.3 g; yield 43%) was obtained.

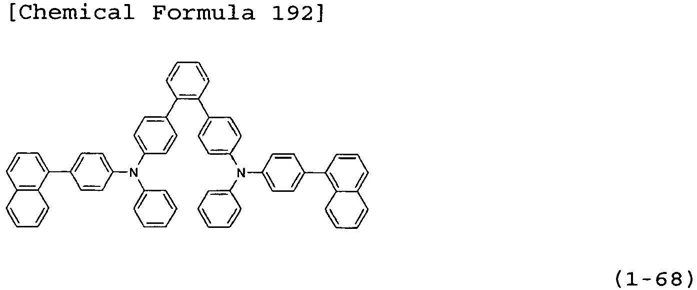

The structure of the obtained white powder was identified by NMR.
¹H-NMR (CDCl₃) detected 44 hydrogen signals, as follows.
δ (ppm) = 8.01 (2H), 7.91 (2H), 7.84 (2H), 7.53-6.98 (38H) .

### Example 31

### <Synthesis of 2,2"-bis[{4-(naphthalen-1-yl)phenyl}-phenylamino]-1,1':3',1"-terphenyl (Compound 1-69)>

The reaction was carried out under the same conditions as those of Example 14, except that 3-bromoiodobenzene was replaced with 1,3-diiodobenzene, and (biphenyl-4-yl)-{4-(4,4,5,5-tetramethyl-[1,3,2]dioxaborolan-2-yl)phenyl}-phenylamine was replaced with 2-[{4-(naphthalen-1-yl)phenyl}-phenylamino]-phenylboronic acid. As a result, a white powder of 2,2"-bis[{4-(naphthalen-1-yl)phenyl}-phenylamino]-1,1':3',1"-terphenyl (Compound 1-69; 7.3 g; yield 43%) was obtained.

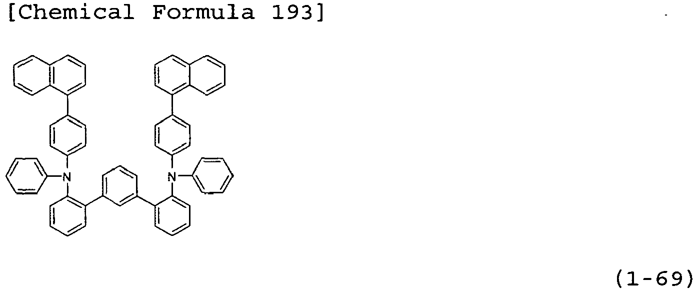

The structure of the obtained white powder was identified by NMR.
¹H-NMR (CDCl₃) detected 44 hydrogen signals, as follows.
δ (ppm) = 7.94-6.85 (44H).

### Example 32

### <Synthesis of 4,4"-bis[{4-(naphthalen-1-yl)phenyl}-phenylamino]-1,1':3',1"-terphenyl (Compound 1-71)>

The reaction was carried out under the same conditions as those of Example 19, except that (triphenylen-2-yl)-phenylamine was replaced with {4-(naphthalen-1-yl)phenyl}-phenylamine. As a result, a white powder of 4,4"-bis[{4-(naphthalen-1-yl)phenyl}-phenylamino]-1,1':3',1"-terphenyl (Compound 1-71; 16.7 g; yield 79%) was obtained.

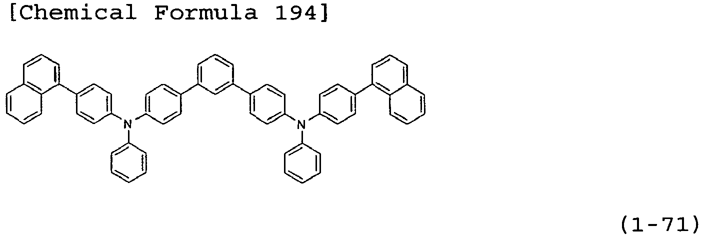

The structure of the obtained white powder was identified by NMR.
¹H-NMR (CDCl₃) detected 44 hydrogen signals, as follows.
δ (ppm) = 8.08 (2H), 7.94 (2H), 7.90-7.80 (3H), 7.65-7.00 (37H).

### Example 33

### <Synthesis of 2,2" -bis{(9,9-dimethyl-9H-fluoren-2-yl)-phenylamino}-1,1':4',1"-terphenyl (Compound 1-75)>

The reaction was carried out under the same conditions as those of Example 15, except that 1,3-dibromobenzene was replaced with 1,4-dibromobenzene, and (biphenyl-4-yl)-{4-(4,4,5,5-tetramethyl-[1,3,2]dioxaborolan-2-yl)phenyl}-(phenyl-d₅)amine was replaced with 2-{(9,9-dimethyl-9H-fluoren-2-yl)-phenylamino}-phenylboronic acid. As a result, a white powder of 2,2"-bis{(9,9-dimethyl-9H-fluoren-2-yl)-phenylamino}-1,1':4',1"-terphenyl (Compound 1-75; 13.7 g; yield 76%) was obtained.

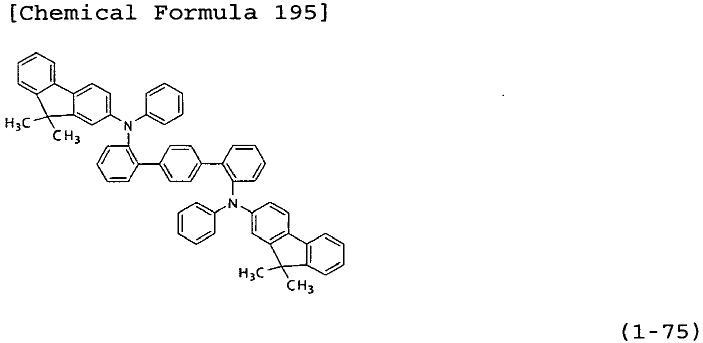

The structure of the obtained white powder was identified by NMR.
¹H-NMR (THF-d₈) detected 48 hydrogen signals, as follows.
δ (ppm) = 7.53 (2H), 7.35-6.81 (30H), 6.76 (2H), 6.67 (2H), 1.29 (12H).

### Example 34

### <Synthesis of 2,2"-bis{bis(biphenyl-4-yl)amino}-1,1':4',1"-terphenyl (Compound 1-76)>

The reaction was carried out under the same conditions as those of Example 15, except that 1,3-dibromobenzene was replaced with 1,4-dibromobenzene, and (biphenyl-4-yl)-{4-(4,4,5,5-tetramethyl-[1,3,2]dioxaborolan-2-yl)phenyl}-(phenyl-d₅)amine was replaced with 2-{bis(biphenyl-4-yl)amino}-phenylboronic acid. As a result, a white powder of 2,2"-bis{bis(biphenyl-4-yl)amino}-1,1':4',1"-terphenyl (Compound 1-76; 15.7 g; yield 78%) was obtained.

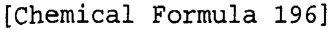

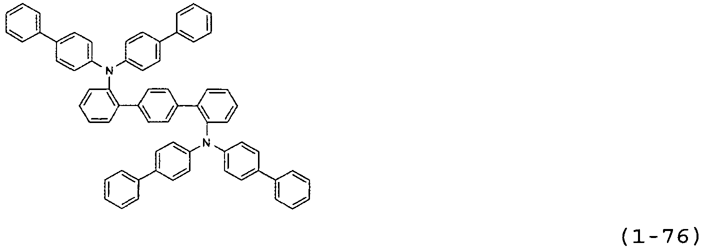

The structure of the obtained white powder was identified by NMR.
¹H-NMR (THF-d₈) detected 48 hydrogen signals, as follows.
δ (ppm) = 7.51-7.45 (8H), 7.33-7.18 (28H), 7.00 (4H), 6.90-6.82 (8H).

### Example 35

### <Synthesis of 2-{(9,9-dimethyl-9H-fluoren-2-yl)-phenylamino}-2"-[{4-(naphthalen-1-yl)phenyl}-phenylamino]-1,1':4',1"-terphenyl (Compound 1-81)>

The reaction was carried out under the same conditions as those of Example 18, except that 4-bromo-4'-{(biphenyl-4-yl)-phenylamino}-biphenyl was replaced with 4-bromo-2'-{4-(naphthalen-1-yl)phenyl}-phenylamino}-biphenyl, and (biphenyl-4-yl)-{2-(4,4,5,5-tetramethyl-[1,3,2]dioxaborolan-2-yl)phenyl}-phenylamine was replaced with 2-{(9,9-dimethyl-9H-fluoren-2-yl)-phenylamino}-phenylboronic acid. As a result, a white powder of 2-{(9,9-dimethyl-9H-fluoren-2-yl)-phenylamino}-2"-[{4-(naphthalen-1-yl)phenyl}-phenylamino]-1,1':4',1"-terphenyl (Compound 1-81; 7.3 g; yield 48%) was obtained.

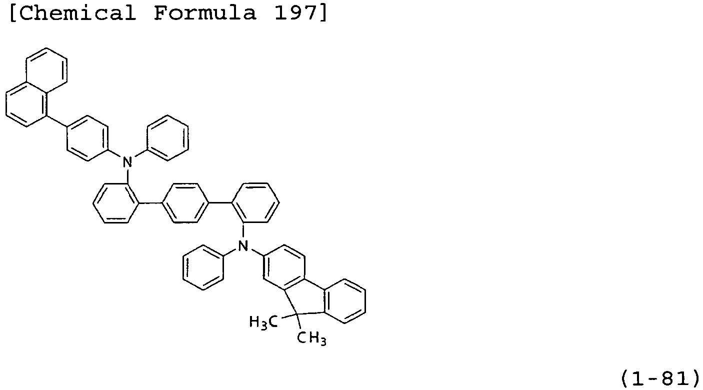

The structure of the obtained white powder was identified by NMR.
¹H-NMR (THF-d₈) detected 46 hydrogen signals, as follows.
δ (ppm) = 7.89-7.76 (3H), 7.55-6.69 (37H), 1.29 (6H).

### Example 36

The melting points and the glass transition points of the arylamine compounds of the general formula (1) were measured using a high-sensitive differential scanning calorimeter (DSC3100SA produced by Bruker AXS).

| | Melting point | Glass transition point |
|---|---|---|
| Compound of Example 1 | 263°C | 111°C |
| Compound of Example 2 | 210°C | 113°C |
| Compound of Example 3 | 265°C | 111°C |
| Compound of Example 4 | 279°C | 107°C |
| Compound of Example 5 | 266°C | 104°C |
| Compound of Example 6 | 263°C | 111°C |
| Compound of Example 7 | 262°C | 117°C |
| Compound of Example 8 | 303°C | 149°C |
| Compound of Example 10 | 365°C | 163°C |
| Compound of Example 11 | 289°C | 138°C |
| Compound of Example 13 | No melting point observed | 125°C |
| Compound of Example 14 | 252°C | 108°C |
| Compound of Example 15 | 252°C | 108°C |
| Compound of Example 16 | No melting point observed | 106°C |
| Compound of Example 17 | No melting point observed | 135°C |
| Compound of Example 18 | No melting point observed | 107°C |
| Compound of Example 19 | 323°C | 159°C |
| Compound of Example 20 | 290°C | 146°C |
| Compound of Example 21 | No melting point observed | 119°C |
| Compound of Example 22 | No melting point observed | 106°C |
| Compound of Example 23 | No melting point observed | 118°C |
| Compound of Example 24 | No melting point observed | 133°C |
| Compound of Example 25 | No melting point observed | 136°C |
| Compound of Example 26 | 286°C | 124°C |
| Compound of Example 27 | No melting point observed | 117°C |
| Compound of Example 28 | 218°C | 114°C |
| Compound of Example 29 | No melting point observed | 127°C |
| Compound of Example 31 | No melting point observed | 110°C |
| Compound of Example 32 | No melting point observed | 122°C |
| Compound of Example 33 | 269°C | 117°C |
| Compound of Example 34 | 277°C | 122°C |
| Compound of Example 35 | No melting point observed | 117°C |

The arylamine compounds of the general formula (1) have glass transition points of 100°C or higher, demonstrating that the compounds have a stable thin-film state.

### Example 37

A 100 nm-thick vapor-deposited film was fabricated on an ITO substrate using the arylamine compounds of the general formula (1), and a work function was measured using an ionization potential measuring device (PYS-202 produced by Sumitomo Heavy Industries, Ltd.).

| | Work function |
|---|---|
| Compound of Example 1 | 5.65 eV |
| Compound of Example 3 | 5.65 eV |
| Compound of Example 4 | 5.67 eV |
| Compound of Example 5 | 5.66 eV |
| Compound of Example 6 | 5.69 eV |
| Compound of Example 7 | 5.63 eV |
| Compound of Example 8 | 5.70 eV |
| Compound of Example 9 | 5.72 eV |
| Compound of Example 10 | 5.62 eV |
| Compound of Example 11 | 5.61 eV |
| Compound of Example 12 | 5.62 eV |
| Compound of Example 13 | 5.67 eV |
| Compound of Example 14 | 5.75 eV |
| Compound of Example 15 | 5.75 eV |
| Compound of Example 16 | 5.79 eV |
| Compound of Example 17 | 5.68 eV |
| Compound of Example 18 | 5.76 eV |
| Compound of Example 19 | 5.70 eV |
| Compound of Example 20 | 5.79 eV |
| Compound of Example 21 | 5.71 eV |
| Compound of Example 22 | 5.79 eV |
| Compound of Example 23 | 5.72 eV |
| Compound of Example 24 | 5.70 eV |
| Compound of Example 25 | 5.71 eV |
| Compound of Example 26 | 5.65 eV |
| Compound of Example 27 | 5.70 eV |
| Compound of Example 28 | 5.67 eV |
| Compound of Example 29 | 5.69 eV |
| Compound of Example 32 | 5.76 eV |

As the results show, the arylamine compounds of the general formula (1) have desirable energy levels compared to the work function 5.4 eV of common hole transport materials such as NPD and TPD, and thus possess desirable hole transportability.

### Example 38

### <Synthesis of 4-phenyl-2-{3-(10-phenylanthracen-9-yl)phenyl}-6-{3-(pyridin-3-yl)phenyl}pyrimidine (Compound 2c-1) >

2-Chloro-4-phenyl-6-{3-(pyridin-3-yl)phenyl}pyrimidine (7.0 g), {3-(10-phenylanthracen-9-yl)phenyl}boronic acid (9.9 g),tetrakis(triphenylphosphine)palladium (0.025 g), a 2 M potassium carbonate aqueous solution (18 ml), toluene (64 ml), and ethanol (16 ml) were added into a nitrogen-substituted reaction vessel, heated and refluxed for 12 hours while being stirred. The mixture was cooled to a room temperature, and the mixture was stirred after adding toluene (100 ml) and water (100 ml). Then, an organic layer was collected by liquid separation. The organic layer was dried over anhydrous magnesium sulfate and then concentrated under reduced pressure to obtain a crude product. The crude product was purified by column chromatography (support: NH silica gel, eluent: toluene/cyclohexane) to obtain a pale yellow powder of 4-phenyl-2-{3-(10-phenylanthracen-9-yl)phenyl}-6-{3-(pyridin-3-yl)phenyl}pyrimidine (Compound 2c-1; 5.2 g; yield 40%).

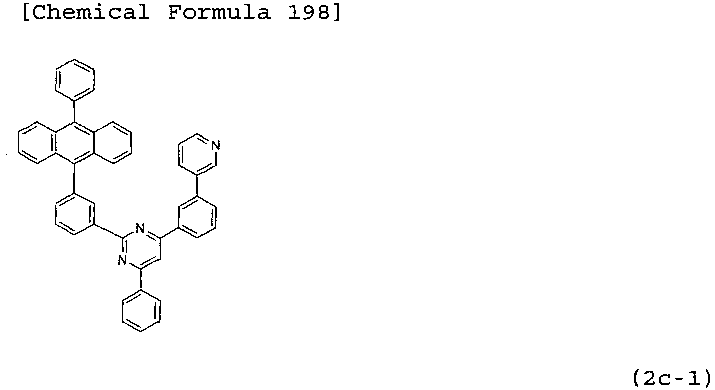

The structure of the obtained pale yellow powder was identified by NMR.
¹H-NMR (CDCl₃) detected 31 hydrogen signals, as follows.
δ (ppm) = 8.95 (1H), 8.86 (1H), 8.65 (1H), 8.46 (1H), 8.29 (3H), 8.10 (1H), 7.97 (1H), 7.70-7.88 (6H), 7.48-7.70 (10H), 7.30-7.45 (6H).

### Example 39

### <Synthesis of 4-phenyl-2-[3-{10-(naphthalen-1-yl)anthracen-9-yl}phenyl]-6-{3-(pyridin-3-yl)phenyl}pyrimidine (Compound 2c-6)>

2-Chloro-4-phenyl-6-{3-(pyridin-3-yl)phenyl}pyrimidine (7.0 g), [3-{10-(naphthalen-1-yl)anthracen-9-yl}phenyl]boronic acid (11.2 g), tetrakis(triphenylphosphine)palladium (0.025 g), a 2 M potassium carbonate aqueous solution (18 ml), toluene (64 ml), and ethanol (16 ml) were added into a nitrogen-substituted reaction vessel, heated and refluxed for 12 hours while being stirred. The mixture was cooled to a room temperature, and the mixture was stirred after adding toluene (100 ml) and water (100 ml). Then, an organic layer was collected by liquid separation. The organic layer was dried over anhydrous magnesium sulfate and then concentrated under reduced pressure to obtain a crude product. The crude product was purified by column chromatography (support: NH silica gel, eluent: toluene/cyclohexane) to obtain a pale yellow powder of 4-phenyl-2-[3-{10-(naphthalen-1-yl)anthracen-9-yl}phenyl]-6-{3-(pyridin-3-yl)phenyl}pyrimidine (Compound 2c-6; 7.5 g; yield 54%).

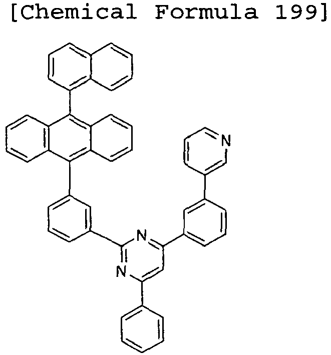

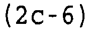

The structure of the obtained pale yellow powder was identified by NMR.
¹H-NMR (CDCl₃) detected 33 hydrogen signals, as follows.
δ (ppm) = 8.86-9.00 (3H), 8.65 (1H), 8.48 (1H), 8.31 (3H), 7.93-8.14 (5H), 7.80-7.92 (3H), 7.45-7.79 (13H), 7.30-7.45 (4H).

### Example 40

### <Synthesis of 4-phenyl-2-{3-(10-phenylanthracen-9-yl)phenyl}-6-{4-(pyridin-3-yl)phenyl}pyrimidine (Compound 2c-13)>

2-Chloro-4-phenyl-6-{4-(pyridin-3-yl)phenyl}pyrimidine (7.0 g), {3-(10-phenylanthracen-9-yl)phenyl}boronic acid (9.9 g), tetrakis(triphenylphosphine)palladium (0.025 g), a 2 M potassium carbonate aqueous solution (18 ml), toluene (64 ml), and ethanol (16 ml) were added into a nitrogen-substituted reaction vessel, heated and refluxed for 12 hours while being stirred. The mixture was cooled to a room temperature, and the mixture was stirred after adding toluene (100 ml) and water (100 ml). Then, an organic layer was collected by liquid separation. The organic layer was dried over anhydrous magnesium sulfate and then concentrated under reduced pressure to obtain a crude product. The crude product was purified by column chromatography (support: NH silica gel, eluent: toluene/cyclohexane) to obtain a pale yellow powder of 4-phenyl-2-{3-(10-phenylanthracen-9-yl)phenyl}-6-{4-(pyridin-3-yl)phenyl}pyrimidine (Compound 2c-13; 5.5 g; yield 42%).

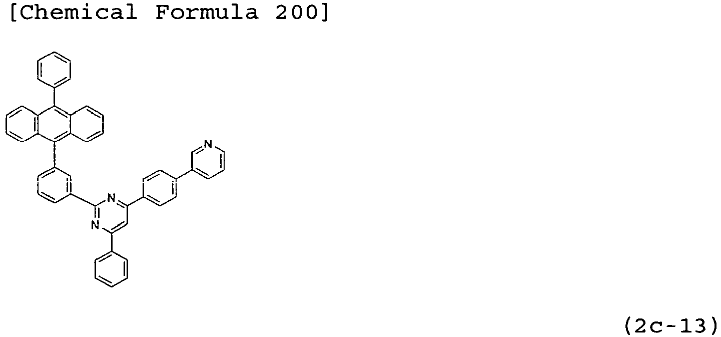

The structure of the obtained pale yellow powder was identified by NMR.
¹H-NMR (CDCl₃) detected 31 hydrogen signals, as follows.
δ (ppm) = 8.84-9.00 (3H), 8.63 (1H), 8.40 (2H), 8.29 (2H), 8.10 (1H), 7.94 (1H), 7.70-7.88 (7H), 7.49-7.70 (9H), 7.31-7.45 (5H).

### Example 41

### <Synthesis of 4-(naphthalen-2-yl)-2-{3-(10-phenylanthracen-9-yl)phenyl}-6-{4-(pyridin-3-yl)phenyl}pyrimidine (Compound 2c-19)>

2-Chloro-4-(naphthalen-2-yl)-6-{4-(pyridin-3-yl)phenyl}pyrimidine (8.0 g), {3-(10-phenylanthracen-9-yl)phenyl}boronic acid (9.9 g), tetrakis(triphenylphosphine)palladium (0.025 g), a 2 M potassium carbonate aqueous solution (18 ml), toluene (64 ml), and ethanol (16 ml) were added into a nitrogen-substituted reaction vessel, heated and refluxed for 12 hours while being stirred. The mixture was cooled to a room temperature, and the mixture was stirred after adding toluene (100 ml) and water (100 ml). Then, an organic layer was collected by liquid separation. The organic layer was dried over anhydrous magnesium sulfate and then concentrated under reduced pressure to obtain a crude product. The crude product was purified by column chromatography (support: NH silica gel, eluent: toluene/cyclohexane) to obtain a pale yellow powder of 4-(naphthalen-2-yl)-2-{3-(10-phenylanthracen-9-yl)phenyl}-6-{4-(pyridin-3-yl)phenyl}pyrimidine (Compound 2c-19; 7.8 g; yield 56%).

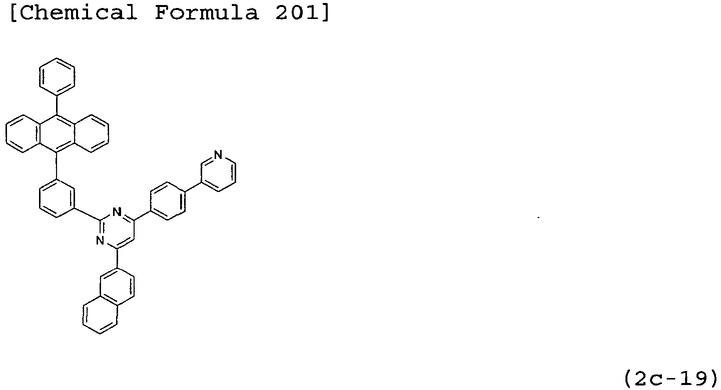

The structure of the obtained pale yellow powder was identified by NMR.
¹H-NMR (CDCl₃) detected 33 hydrogen signals, as follows.
δ (ppm) = 8.89-9.07 (3H), 8.79 (1H), 8.65 (1H), 8.37-8.50 (3H), 8.25 (1H), 7.72-8.09 (10H), 7.49-7.71 (9H), 7.33-7.45 (5H).

### Example 42

### <Synthesis of 4-phenyl-2-[3-{10-(naphthalen-1-yl)anthracen-9-yl}phenyl]-6-{4-(pyridin-3-yl)phenyl}pyrimidine (Compound 2c-28)>

2-Chloro-4-phenyl-6-{4-(pyridin-3-yl)phenyl}pyrimidine (7.0 g), [3-{10-(naphthalen-1-yl)anthracen-9-yl}phenyl]boronic acid (11.2 g), tetrakis(triphenylphosphine)palladium (0.025 g), a 2 M potassium carbonate aqueous solution (18 ml), toluene (64 ml), and ethanol (16 ml) were added into a nitrogen-substituted reaction vessel, heated and refluxed for 12 hours while being stirred. The mixture was cooled to a room temperature, and the mixture was stirred after adding toluene (100 ml) and water (100 ml). Then, an organic layer was collected by liquid separation. The organic layer was dried over anhydrous magnesium sulfate and then concentrated under reduced pressure to obtain a crude product. The crude product was purified by column chromatography (support: NH silica gel, eluent: toluene/cyclohexane) to obtain a pale yellow powder of 4-phenyl-2-[3-{10-(naphthalen-1-yl)anthracen-9-yl}phenyl]-6-{4-(pyridin-3-yl)phenyl}pyrimidine (Compound 2c-28; 8.4 g; yield 60%).

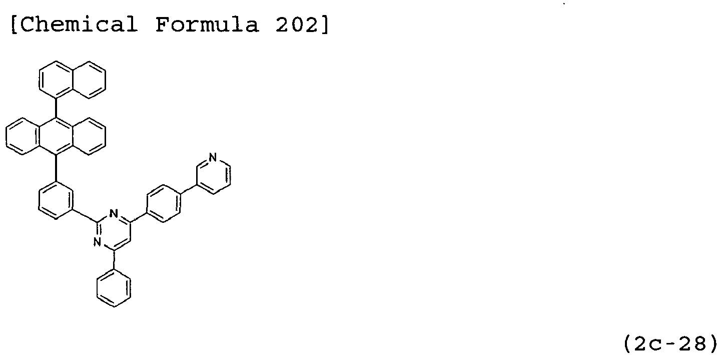

The structure of the obtained pale yellow powder was identified by NMR.
¹H-NMR (CDCl₃) detected 33 hydrogen signals, as follows.
δ (ppm) = 8.86-9.04 (3H), 8.65 (1H), 8.43 (2H), 8.32 (2H), 8.01-8.15 (3H), 7.95 (1H), 7.69-7.92 (7H), 7.31-7.68 (14H).

### Example 43

The melting points and the glass transition points of the compounds of the general formula (2c) having an anthracene ring structure were determined using a high-sensitive differential scanning calorimeter (DSC3100SA produced by Bruker AXS).

| | Melting point | Glass transition point |
|---|---|---|
| Compound of Example 38 | 257°C | 126°C |
| Compound of Example 39 | 282°C | 147°C |
| Compound of Example 40 | 293°C | 144°C |
| Compound of Example 41 | 295°C | 152°C |
| Compound of Example 42 | 312°C | 168°C |

The compounds of the general formula (2c) having an anthracene ring structure have glass transition points of 100°C or higher, demonstrating that the compounds have a stable thin-film state.

### Example 44

A 100 nm-thick vapor-deposited film was fabricated on an ITO substrate using the compounds of the general formula (2c) having an anthracene ring structure, and a work function was measured using an ionization potential measuring device (PYS-202 produced by Sumitomo Heavy Industries, Ltd.).

| | Work function |
|---|---|
| Compound of Example 38 | 5.97 eV |
| Compound of Example 39 | 6.05 eV |
| Compound of Example 40 | 5.97 eV |
| Compound of Example 41 | 6.03 eV |
| Compound of Example 42 | 6.04 eV |

As the results show, the compounds of the general formula (2c) having an anthracene ring structure have greater work functions than the work function 5.4 eV of common hole transport materials such as NPD and TPD, and thus possess a high hole blocking ability.

### Example 45 (Working Example)

The organic EL device, as shown in FIG. 1, was fabricated by vapor-depositing a hole injection layer 3, a hole transport layer 4, a light emitting layer 5, an electron transport layer 6, an electron injection layer 7, and a cathode (aluminum electrode) 8 in this order on a glass substrate 1 on which an ITO electrode was formed as a transparent anode 2 beforehand.

Specifically, the glass substrate 1 having ITO (film thickness of 150 nm) formed thereon was subjected to ultrasonic washing in isopropyl alcohol for 20 minutes and then dried for 10 minutes on a hot plate heated to 200°C. After UV ozone treatment for 15 minutes, the glass substrate with ITO was installed in a vacuum vapor deposition apparatus, and the pressure was reduced to 0.001 Pa or lower. The compound (HIM-1) of the structural formula below was then formed in a film thickness of 5 nm as the hole injection layer 3 so as to cover the transparent anode 2. The hole transport layer 4 was formed on the hole injection layer 3 by forming the compound (Compound 1-1) of Example 1 in a film thickness of 65 nm. Then, the light emitting layer 5 was formed on the hole transport layer 4 in a film thickness of 20 nm by dual vapor deposition of pyrene derivatives (EMD-1) of the structural formula below and anthracene derivatives (EMH-1) of the structural formula below at a vapor deposition rate ratio of EMD-1:EMH-1 = 5:95. The electron transport layer 6 was formed on the light emitting layer 5 in a film thickness of 30 nm by dual vapor deposition of the compound (Compound 2c-13) of Example 40 and the compound (ETM-1) of the structural formula below at a vapor deposition rate ratio of Compound 2c-13:ETM-1 = 50:50. The electron injection layer 7 was formed on the electron transport layer 6 by forming lithium fluoride in a film thickness of 1 nm. Finally, the cathode 8 was formed by vapor-depositing aluminum in a thickness of 100 nm. The characteristics of the thus fabricated organic EL device were measured in the atmosphere at an ordinary temperature. Table 1 summarizes the results of emission characteristics measurements performed by applying a DC voltage (current density of 1 mA/cm²) to the fabricated organic EL device.

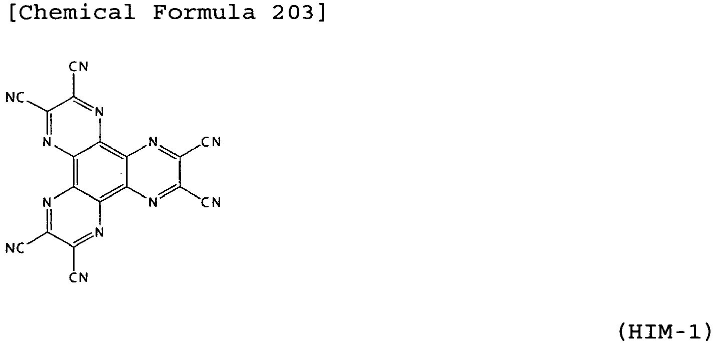

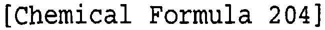

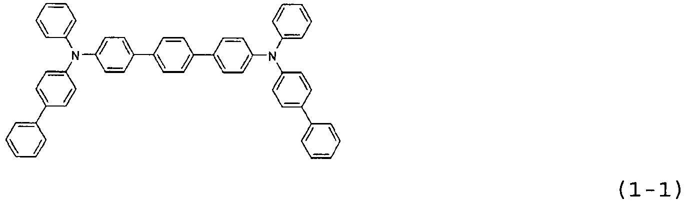

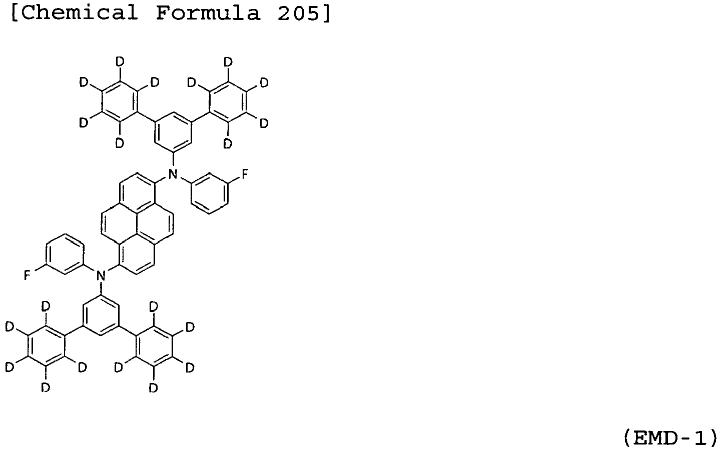

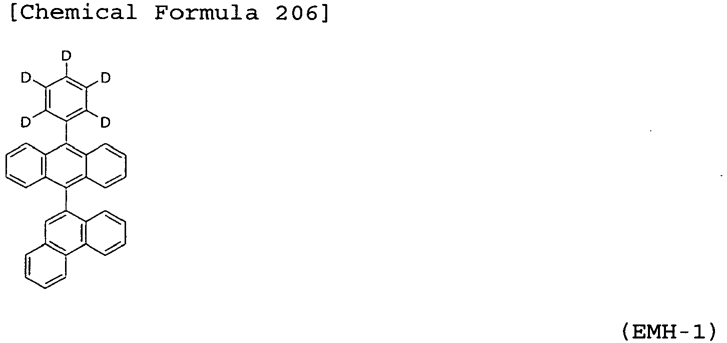

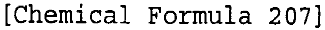

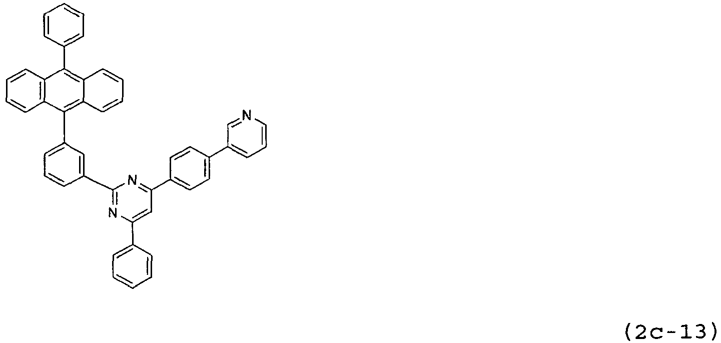

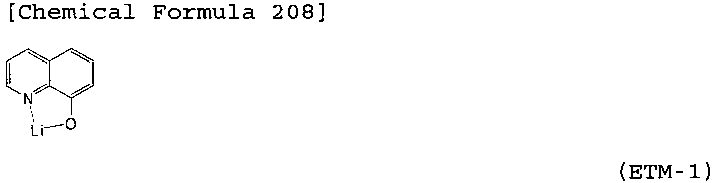

### Comparative Example 1

For comparison, an organic EL device was fabricated under the same conditions used in Example 45, except that the hole transport layer 4 was formed by forming the compound (HTM-1) of the structural formula below in a film thickness of 65 nm, instead of using the compound (Compound 1-1) of Example 1. The characteristics of the organic EL device thus fabricated were measured in the atmosphere at an ordinary temperature. Table 1 summarizes the results of emission characteristics measurements performed by applying a DC voltage (current density of 1 mA/cm²) to the fabricated organic EL device.

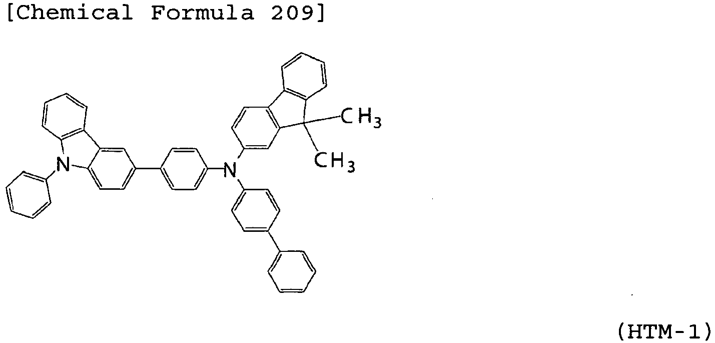

Table 1 summarizes the results of device lifetime measurements performed with organic EL devices fabricated in Example 45 and Comparative Example 1. A device lifetime was measured as the time elapsed until the emission luminance of 2,000 cd/m² (initial luminance) at the start of emission was attenuated to 1,900 cd/m² (corresponding to attenuation to 95% when taking the initial luminance as 100%) when carrying out constant current driving.

**[Table 1]**

| | Hole transport layer | Light emitting layer | Electron transport layer | Voltage [V] (1mA/cm²) | Luminance [cd/m²] (1mA/cm²) | Current efficiency [cd/A] (1 mA/cm²) | Power efficiency [lm/W] (1mA/cm²) | Device lifetime (Attenuation to 95%) |
|---|---|---|---|---|---|---|---|---|
| Ex. 45 | Compound 1-1 | EMD-1/EMH-1 | Compound 2c-13/ETM-1 | 3.11 | 86 | 8.52 | 8.59 | 144 h |
| Com. Ex. 1 | HTM-1 | EMD-1/EMH-1 | Compound 2c-13/ETM-1 | 3.06 | 80 | 7.71 | 7.91 | 61 h |

As shown in Table 1, the current efficiency upon passing a current with a low current density of 1 mA/cm² was 8.52 cd/A for the organic EL device in Example 45, which was higher than 7.71 cd/A for the organic EL device in Comparative Example 1. Further, the power efficiency was 8.59 lm/W for the organic EL device in Example 45, which was higher than 7.91 lm/W for the organic EL device in Comparative Example 1. Table 1 also shows that the device lifetime (attenuation to 95%) was 144 hours for the organic EL device in Example 45, showing achievement of a far longer lifetime than 61 hours for the organic EL device in Comparative Example 1.

### Example 46 (Working Example)

An organic EL device was fabricated under the same conditions used in Example 45, except using the amine derivative (EMD-2) having a fluorene ring as a part of a condensed ring instead of the pyrene derivatives (EMD-1) of the structural formula as material of the light emitting layer 5, and further except forming the light emitting layer 5 in a film thickness of 25 nm by performing dual vapor deposition of the amine derivatives (EMD-2) having a fluorene ring as a part of a condensed ring and the anthracene derivatives (EMH-1) of the structural formula at a vapor deposition rate ratio of EMD-2:EMH-1 = 5:95. The characteristics of the organic EL device thus fabricated were measured in the atmosphere at an ordinary temperature. Table 2 summarizes the results of emission characteristics measurements performed by applying a DC voltage (current density of 10 mA/cm²) to the fabricated organic EL device.

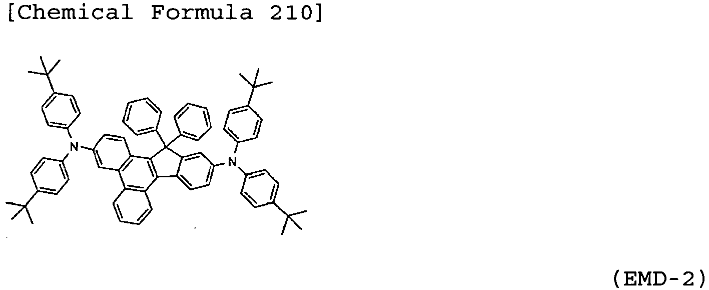

### Comparative Example 2

For comparison, an organic EL device was fabricated under the same conditions used in Example 46, except that the hole transport layer 4 was formed by forming the compound (HTM-1) of the structural formula in a film thickness of 65 nm, instead of using the compound (Compound 1-1) of Example 1. The characteristics of the organic EL device thus fabricated were measured in the atmosphere at an ordinary temperature. Table 2 summarizes the results of emission characteristics measurements performed by applying a DC voltage (current density of 10 mA/cm²) to the fabricated organic EL device.

Table 2 summarizes the results of device lifetime measurements performed with organic EL devices fabricated in Example 46 and Comparative Example 2. A device lifetime was measured as the time elapsed until the emission luminance of 2,000 cd/m² (initial luminance) at the start of emission was attenuated to 1,900 cd/m² (corresponding to attenuation to 95% when taking the initial luminance as 100%) when carrying out constant current driving.

**[Table 2]**

| | Hole transport layer | Light emitting layer | Electron transport layer | Voltage [V] (10mA/cm²) | Luminance [cd/m²] (10mA/cm²) | Current efficiency [cd/A] (10mA/cm²) | Power efficiency [lm/W] (10mA/cm²) | Device lifetime (Attenuation to 95%) |
|---|---|---|---|---|---|---|---|---|
| Ex. 46 | Compound 1-1 | EMD-2/EMH-1 | Compound 2c-13/ETM-1 | 3.83 | 890 | 8.89 | 7.29 | 159 h |
| Com. Ex. 2 | HTM-1 | EMD-2/EMH-1 | Compound 2c-13 ETM-1 | 3.77 | 800 | 7.98 | 6.65 | 60h |

As shown in Table 2, the current efficiency upon passing a current with a current density of 10 mA/cm² was 8.89 cd/A for the organic EL device in Example 46, which was higher than 7.98 cd/A for the organic EL device in Comparative Example 2. Further, the power efficiency was 7.29 lm/W for the organic EL device in Example 46, which was higher than 6.65 lm/W for the organic EL device in Comparative Example 2. Table 2 also shows that the device lifetime (attenuation to 95%) was 159 hours for the organic EL device in Example 46, showing achievement of a far longer lifetime than 60 hours for the organic EL device in Comparative Example 2.

In the organic EL devices of the present invention, the combination of specific arylamine compounds and specific compounds having an anthracene ring structure can improve carrier balance inside the organic EL devices. Further, the organic EL devices of the present invention can achieve high luminous efficiency and a long lifetime, compared to the conventional organic EL devices by combining those compounds in carrier balance matching characteristics of the light-emitting material.

In the organic EL devices of the present invention with the combination of specific arylamine compounds and specific compounds having an anthracene ring structure, luminous efficiency and durability of an organic EL device can be improved to attain potential applications for, for example, home electric appliances and illuminations.

### Description of Reference Numeral

- 1: Glass substrate
- 2: Transparent anode
- 3: Hole injection layer
- 4: Hole transport layer
- 5: Light emitting layer
- 6: Electron transport layer
- 7: Electron injection layer
- 8: Cathode

## Claims

1. An organic electroluminescent device comprising at least an anode, a hole transport layer, a light emitting layer, an electron transport layer and a cathode in this order, wherein the hole transport layer is a single layer, an arylamine compound of the following general formula (1) is used as the hole transport layer, and the electron transport layer comprises a compound of the following general formula (2c) having an anthracene ring structure: 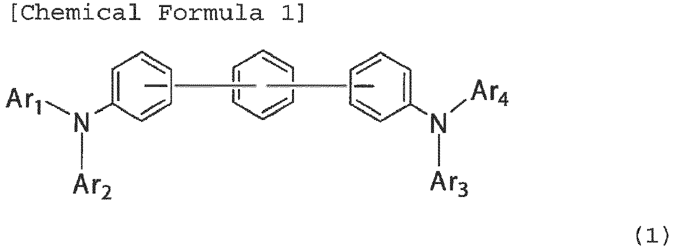 wherein Ar₁ to Ar₄ may be the same or different, and represent a substituted or unsubstituted aromatic hydrocarbon group, a substituted or unsubstituted aromatic heterocyclic group, or a substituted or unsubstituted condensed polycyclic aromatic group,
wherein the compound having an anthracene ring structure is a compound of the following general formula (2c) having an anthracene ring structure, 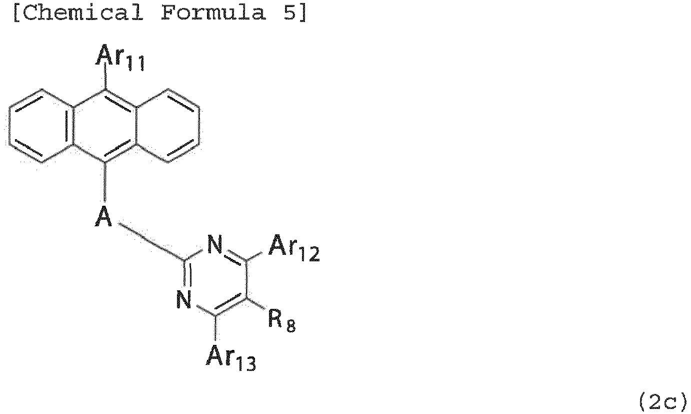 wherein A represents a divalent group of a substituted or unsubstituted aromatic hydrocarbon, a divalent group of a substituted or unsubstituted aromatic heterocyclic ring, a divalent group of substituted or unsubstituted condensed polycyclic aromatics, or a single bond; Ar₁₁, Ar₁₂ and Ar₁₃ may be the same or different, and represent a substituted or unsubstituted aromatic hydrocarbon group, a substituted or unsubstituted aromatic heterocyclic group, or a substituted or unsubstituted condensed polycyclic aromatic group; and R₈ represents a hydrogen atom, a deuterium atom, a fluorine atom, a chlorine atom, cyano, nitro, linear or branched alkyl of 1 to 6 carbon atoms that may have a substituent, cycloalkyl of 5 to 10 carbon atoms that may have a substituent, linear or branched alkenyl of 2 to 6 carbon atoms that may have a substituent, linear or branched alkyloxy of 1 to 6 carbon atoms that may have a substituent, cycloalkyloxy of 5 to 10 carbon atoms that may have a substituent, a substituted or unsubstituted aromatic hydrocarbon group, a substituted or unsubstituted aromatic heterocyclic group, or a substituted or unsubstituted condensed polycyclic aromatic group, or substituted or unsubstituted aryloxy.

2. The organic electroluminescent device according to claim 1, wherein the light emitting layer includes a blue luminous dopant.

3. The organic electroluminescent device according to claim 2, wherein the light emitting layer includes the blue luminous dopant that is a pyrene derivative.

4. The organic electroluminescent device according to claim 2, wherein the light emitting layer includes the blue luminous dopant that is an amine derivative having a fluorene ring as a part of a condensed ring.

5. The organic electroluminescent device according to any one of claims 1 to 4, wherein the light emitting layer includes an anthracene derivative.

6. The organic electroluminescent device according to claim 5, wherein the light emitting layer includes a host material that is an anthracene derivative.

## Patentansprüche

1. Organische Elektrolumineszenzvorrichtung, umfassend zumindest eine Anode, eine Lochtransportschicht, eine lichtemittierende Schicht, eine Elektrontransportschicht und eine Kathode in dieser Reihenfolge, wobei die Lochtransportschicht eine einzelne Schicht ist, eine Arylaminverbindung der nachstehenden allgemeinen Formel (1) als die Lochtransportschicht verwendet wird, und die Elektrontransportschicht eine Verbindung der nachstehenden allgemeinen Formel (2c) mit einer Anthracenringstruktur umfasst: 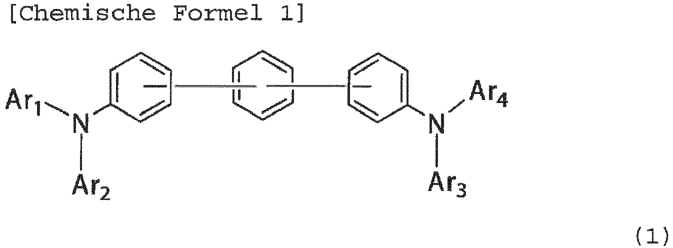 wobei Ar₁ bis Ar₄ gleich oder verschieden sein können und eine substituierte oder unsubstituierte aromatische Kohlenwasserstoffgruppe, eine substituierte oder unsubstituierte aromatische heterocyclische Gruppe oder eine substituierte oder unsubstituierte kondensierte polycyclische aromatische Gruppe darstellen,
wobei die Verbindung mit einer Anthracenringstruktur eine Verbindung der nachstehenden allgemeinen Formel (2c) mit einer Anthracenringstruktur ist, 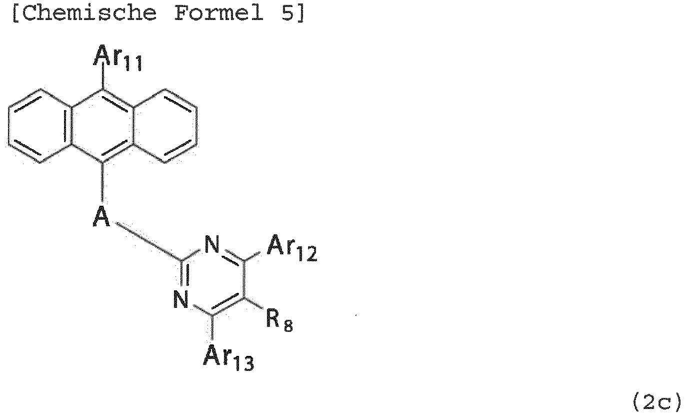 wobei A eine zweiwertige Gruppe eines substituierten oder unsubstituierten aromatischen Kohlenwasserstoffs, eine zweiwertige Gruppe eines substituierten oder unsubstituierten aromatischen heterocyclischen Rings, eine zweiwertige Gruppe substituierter oder unsubstituierter kondensierter polycyclischer Aromaten oder eine Einfachbindung darstellt; Ar₁₁, Ar₁₂ und Ar₁₃ gleich oder verschieden sein können und eine substituierte oder unsubstituierte aromatische Kohlenwasserstoffgruppe, eine substituierte oder unsubstituierte aromatische heterocyclische Gruppe oder eine substituierte oder unsubstituierte kondensierte polycyclische aromatische Gruppe darstellen; und R₈ ein Wasserstoffatom, ein Deuteriumatom, ein Fluoratom, ein Chloratom, Cyano, Nitro, unverzweigtes oder verzweigtes Alkyl mit 1 bis 6 Kohlenstoffatomen, die einen Substituenten aufweisen können, Cycloalkyl mit 5 bis 10 Kohlenstoffatomen, die einen Substituenten aufweisen können, unverzweigtes oder verzweigtes Alkenyl mit 2 bis 6 Kohlenstoffatomen, die einen Substituenten aufweisen können, unverzweigtes oder verzweigtes Alkyloxy mit 1 bis 6 Kohlenstoffatomen, die einen Substituenten aufweisen können, Cycloalkyloxy mit 5 bis 10 Kohlenstoffatomen, die einen Substituenten aufweisen können, eine substituierte oder unsubstituierte aromatische Kohlenwasserstoffgruppe, eine substituierte oder unsubstituierte aromatische heterocyclische Gruppe oder eine substituierte oder unsubstituierte kondensierte polycyclische aromatische Gruppe oder substituiertes oder unsubstituiertes Aryloxy darstellt.

2. Organische Elektrolumineszenzvorrichtung nach Anspruch 1, wobei die lichtemittierende Schicht einen blau leuchtenden Dotierstoff einschließt.

3. Organische Elektrolumineszenzvorrichtung nach Anspruch 2, wobei die lichtemittierende Schicht den blau leuchtenden Dotierstoff, der ein Pyrenderivat ist, einschließt.

4. Organische Elektrolumineszenzvorrichtung nach Anspruch 2, wobei die lichtemittierende Schicht den blau leuchtenden Dotierstoff, der ein Aminderivat mit einem Fluorenring als Teil eines kondensierten Rings ist, einschließt.

5. Organische Elektrolumineszenzvorrichtung nach einem der Ansprüche 1 bis 4, wobei die lichtemittierende Schicht ein Anthracenderivat einschließt.

6. Organische Elektrolumineszenzvorrichtung nach Anspruch 5, wobei die lichtemittierende Schicht ein Wirtsmaterial, das ein Anthracenderivat ist, einschließt.

## Revendications

1. Dispositif électroluminescent organique, comprenant au moins une anode, une couche de transport de trous, une couche émettrice de lumière, une couche de transport d'électrons, et une cathode, en cet ordre, dans lequel la couche de transport de trous est une couche unique, un composé arylamine de la formule générale (1) suivante est utilisé comme couche de transport de trous, et la couche de transport d'électrons comprend un composé de la formule générale (2c) suivante ayant une structure à cycle anthracénique : 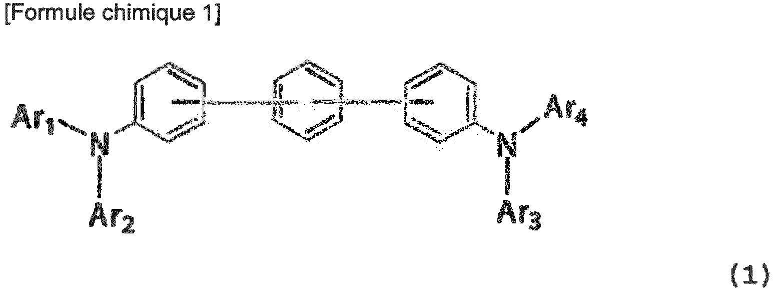 dans laquelle Ar₁ à Ar₄ peuvent être identiques ou différents, et représentent un groupe hydrocarboné aromatique substitué ou non substitué, un groupe hétérocyclique aromatique substitué ou non substitué, ou un groupe aromatique polycyclique condensé substitué ou non substitué,
dans lequel le composé ayant une structure à cycle anthracénique est un composé de la formule générale (2c) suivante ayant une structure à cycle anthracénique, 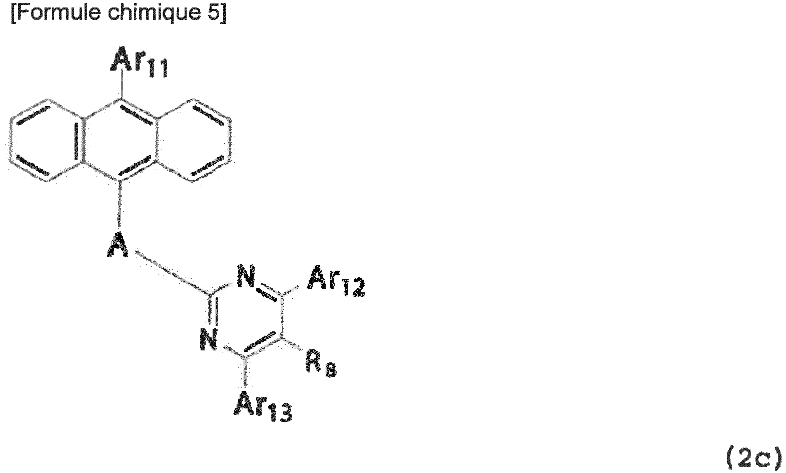 dans laquelle A représente un groupe bivalent d'un hydrocarbure aromatique substitué ou non substitué, un groupe bivalent d'un hétérocycle aromatique substitué ou non substitué, un groupe bivalent d'un aromatique polycyclique condensé substitué ou non substitué, ou une simple liaison ; Ar₁₁, Ar₁₂ et Ar₁₃ peuvent être identiques ou différents, et représentent un groupe hydrocarboné aromatique substitué ou non substitué, un groupe hétérocyclique aromatique substitué ou non substitué, ou un groupe aromatique polycyclique condensé substitué ou non substitué ; et R₈ représente un atome d'hydrogène, un atome de deutérium, un atome de fluor, un atome de chlore, cyano, nitro, alkyle linéaire ou ramifié ayant 1 à 6 atomes de carbone, qui peut porter un substituant, cycloalkyle ayant 5 à 10 atomes de carbone, qui peut porter un substituant, alcényle linéaire ou ramifié ayant 2 à 6 atomes de carbone, qui peut porter un substituant, alkyloxy linéaire ou ramifié ayant 1 à 6 atomes de carbone, qui peut porter un substituant, cycloalkyloxy ayant 5 à 10 atomes de carbone, qui peut porter un substituant, un groupe hydrocarboné aromatique substitué ou non substitué, un groupe hétérocyclique aromatique substitué ou non substitué, un groupe aromatique polycyclique condensé substitué ou non substitué, ou un aryloxy substitué ou non substitué.

2. Dispositif électroluminescent organique selon la revendication 1, dans lequel la couche émettrice de lumière comprend un dopant de lumière bleue.

3. Dispositif électroluminescent organique selon la revendication 2, dans lequel la couche émettrice de lumière comprend le dopant de lumière bleue, qui est un dérivé du pyrène.

4. Dispositif électroluminescent organique selon la revendication 2, dans lequel la couche émettrice de lumière comprend le dopant de lumière bleue, qui est un dérivé amine ayant un cycle fluorène comme partie d'un cycle condensé.

5. Dispositif électroluminescent organique selon l'une quelconque des revendications 1 à 4, dans lequel la couche émettrice de lumière comprend un dérivé de l'anthracène.

6. Dispositif électroluminescent organique selon la revendication 5, dans lequel la couche émettrice de lumière comprend un matériau hôte qui est un dérivé de l'anthracène.
